# EUROPEAN PATENT SPECIFICATION

(11) **EP 1 871 844 B1**
(45) Date of publication and mention of the grant of the patent: **27.08.2014**
(21) Application number: 06739926.1
(22) Date of filing: 29.03.2006
(51) Int. Cl.: A63H 33/28

(54) **BUBBLE COMPOSITION WITH COLOR CHANGING INDICATOR**
SEIFENBLASEN MIT FARBÄNDERUNGSANZEIGE
BULLES DE SAVON A INDICATEUR CHANGEANT DE COULEUR

(30) Priority: 29.03.2005 US 666028 P; 06.07.2005 US 696872 P; 25.08.2005 US 711183 P; 25.08.2005 US 711450 P; 18.10.2005 US 727608 P; 07.11.2005 US 734150 P; 28.03.2006 US 390979
(43) Date of publication of application: 02.01.2008
(73) Proprietor: C2C Technologies LLC, St. Paul, MN 55116 (US)
(72) Inventor: SABNIS, Ram, W., Daly City, CA 94015 (US); KEHOE, Timothy, D., St. Paul, MN 55116 (US); BALCHUNIS, Robert, J., St. Paul, MN 55117 (US)
(74) Representative: Golding, Louise Ann
(86) International application number: PCT/US2006/011456
(87) International publication number: WO 2006/105191

(56) References cited:
- EP-A- 0 549 145
- EP-A- 1 457 529
- GB-A- 2 086 407
- US-A1- 2004 026 664
- PATENT ABSTRACTS OF JAPAN vol. 010, no. 017 (C-324), 23 January 1986 (1986-01-23) & JP 60 170674 A (TOSHIBA KK), 4 September 1985 (1985-09-04)
- PATENT ABSTRACTS OF JAPAN vol. 011, no. 232 (C-437), 29 July 1987 (1987-07-29) & JP 62 045672 A (SUMITOMO ELECTRIC IND LTD), 27 February 1987 (1987-02-27)
- ANONYMOUS: "colored_bubbles" INTERNET ARTICLE, [Online] XP002404490 Retrieved from the Internet: URL:www.geocities.com/bislz/index.html> [retrieved on 2006-10-25]
- ANONYMOUS: "How To Make Colored Soap Bubbles" INTERNET ARTICLE, [Online] XP002404491 Retrieved from the Internet: URL:http://chemistry.abaout.com/od/chemist ryhowtoguide/ht/colorbuble.htm> [retrieved on 2006-10-25]

## Description

### FIELD OF THE INVENTION

The invention relates generally to compositions that can change color from colored to colorless, one color to another color, or colorless to colored under appropriate conditions, and combinations thereof, and methods to prepare the compositions, such as bubbles, and use of these compositions. Particularly, the present invention relates to compositions for blowing bubbles, which can be particularly useful for children. The compositions are non-toxic and, if necessary, are washable.

### BACKGROUND OF THE INVENTION

Paints are generally of two types. The first type is solvent borne (or oil based) paints in which a polymeric binder is dissolved or dispersed in an organic solvent. The second type is water borne paints in which a polymeric binder is a dispersion of insoluble polymer in water. Water borne paints are often referred as 'emulsion paints' and these represent the most common type of wall and ceiling paints.

Paint formulations generally comprise a film forming polymer, one or more type of non-film forming solids, such as titanium dioxide and the like, extenders, thickeners, and other additives such as leveling agents etc. The dispersed insoluble film forming polymers are usually vinyl, acrylic, epoxy, polyurethane, polyester, alkyds, glycidyl esters, glycidyl ethers etc.

The non-film forming solids are generally colorants and/or titanium dioxide. The most commonly used colorants are pigments which can be either inorganic or organic pigments. Commercially available pigments are available the aggregates and agglomerates forms. Use of pigments in the paint composition often requires pigment dispersion mills for grinding the pigments. Pigments are generally ground to their primary particle size for uniformity before using them in the paints. The ground pigment formulations may flocculate back into agglomerates form which lowers the shelf life of the paints. Thus, the overall process increases the time/cost, and reduces the quality.

Dyes are also used as colorants for paint formulations. Commonly used dyes are basic dyes and food dyes. The paint compositions based on either basic dyes or food dyes are not easily washable, especially, from porous and hard surfaces.

Extenders are particulate non-film forming solids which are often added to paints to lower cost, to modify the rheology, or to improve pigment utilization by inhibiting pigment particle agglomeration. They differ from true pigments in having little or no effect on opacity.

Thickeners are generally polymeric materials that, as name suggests, increase the viscosity of the paint. Thickeners vary widely in their chemical form, but can generally be described as water soluble or water swellable polymers having hydrophilic groups.

Color change has been a fascination of individuals for a long time. Traditionally, compounds that have exhibited the ability to change from colored to colorless have been leuco dyes.

Leuco dyes are of limited use to produce materials that begin as colored and end as colorless since three components are generally required to effect the transition. Generally, a color former (the leuco dye), a developer (such as a phenolic compound) and a reversible matrix, such as a long chain alcohol, are combined. An often noted drawback with leuco dye systems is their water insolubility.

Traditional painting materials, provide specific color to the substrate. A drawback with traditional painting materials is that they do not change color when required, tend to be messy and leave unwanted marks. Thus parents often limit the use of painting materials, especially finger painting materials because of the possibility of household objects, such as carpet, furniture, skin and clothing, being stained by the finger painting materials. The unwanted marks are usually extremely difficult or impossible to remove.

Accordingly, many manufactures and inventors have spent great time and resources trying to create products and methods to reduce or eliminate staining. They have also spent a great deal of effort to produce products that simulate marking. However, such products have not offered the creative freedom and flexibility of traditional finger painting materials. Most of the commercial finger painting materials labeled "washable" have been found to be difficult or impossible, for example, to remove from fingers, hands or cloths. The traditional paints or finger paints generally do not change color, nor are they completely washable, often leaving residue or stain.

Bubbles have long fascinated children, adults, and scientists alike. The formation of bubbles for recreation and entertainment is a well-recognized and widely practiced past-time. In its simplest form, bubble blowing involves dipping a shaped article having an opening into a liquid soap solution followed by blowing into the opening to form one or more bubbles. A bubble is generally defined as a small volume of gas contained within a thin liquid spherical envelop. A wand, for example, is generally immersed into a bubble solution and air is blown through spherical opening to generate bubbles. Surface tension causes the bubble solution to for a film across the opening. Upon application of a sufficient force or pressure upon one side of the film, a bubble is formed and expelled from the opening.

A variety of bubble solutions have been marketed over the years, many of them claiming to have special features like longer lasting bubbles, solutions that produce greater numbers of bubbles, or solution that provide bubbles having a colorful in appearance. Some manufacturers adorn their bubble packaging with illustrations of colored bubbles, or add colorants to tint their bubble solution, in an effort to provoke the illusion of a colored bubble. Some manufactures have added modifying agents like glycerin to produce a transparent bubble with a transparent iridescent rainbow effect. One manufacturer added color directly to the bubble and/or the bubble solution in an effort to create designs on a piece of paper with what they labeled a colored bubble. This composition of liquid solution does not produce a visually colored bubble, but rather a bubble that is used as a vehicle to transport the color to the marking surface. The bubble wall is transparent and does not produce a uniformly colored bubble. Rather the color runs to the bottom of the bubble wall. Others manufacturers claim to produce bubble that is illuminated when viewed in the dark with infrared radiation or black light, but transparent in regular light.

Additionally, the aqueous solutions that are currently provided by manufacturers afford bubbles that do not withstand environmental stresses, such as wind, airborne particulate, or contact with a surface, among many other physically detrimental impediments. Contact with a surface generally causes the bubble to burst, decreasing the enjoyment one obtains from experiencing the bubble.

Traditional marking materials, such as paints, markers, pens, spray-paint, chalk, lipstick, and doll cosmetics, are given to children for drawing, painting, decorating, styling and coloring purposes. A drawback with traditional marking materials is that they tend to be messy and leave unwanted marks. Thus parents often limit the use of marking materials because of the possibility of household objects, such as carpet, furniture, skin and clothing, being stained by the marking materials. The unwanted marks are usually extremely difficult or impossible to remove. Accordingly, many manufactures and inventors have spent great time and money trying to create products and methods to reduce or eliminate staining. They have also spent an equal amount of effort to produce products that simulate marking but do not offer the creative freedom and flexibility of traditional marking materials. Most of the commercial marking materials labeled "washable" have been found to be difficult or impossible, for example, to remove from simple cotton t-shirts.

Cosmetics are generally applied to an area to affect a change in color, tone, appearance, suppleness, or other visible attributes. Generally, the application lasts for many hours, a day, or in the case of hair color, weeks or months. For children, it would be fun to have cosmetics that provide the desired change but do not last for a lengthy period of time.

Therefore, a need exists for new compositions that can provide one or more of: a change in color (or to colorless), water washable, do not leave a stain or residue, provide a wide range of color across the color spectrum, are water solubile, can be easily prepared in high yields with a simple procedure, and are non-toxic.

### BRIEF SUMMARY OF THE INVENTION

The present invention provides unique phthalein acid-base bubble compositions as detailed throughout the specification that are useful in paints, bubbles, markers and cosmetics. These phthalein acid-base compositions are previously unknown and can be used in any of the above-identified applications (and those listed throughout the specification). The phthalein compositions provide novel colors, previously not achievable by known synthetic procedures. Additionally, the colored phthalein compositions of the invention can be made to change to colorless, a first color to a second color, or colorless to a color under appropriate conditions.

In one embodiment, the present invention provides an aqueous composition that includes a surfactant and an acid-base indicator of the formula as herein described. The compositions provide a composition, such as a bubble, that is a uniformly colored composition. Alternatively, the uniformly colored composition can change to colorless under appropriate conditions. The compositions are non-toxic and/or washable.

Thus, viewed from a first aspect, the present invetion provides a substantially uniformly colored bubble composition comprising:
a surfactant;
water; and
an acid-base indicator comprising: 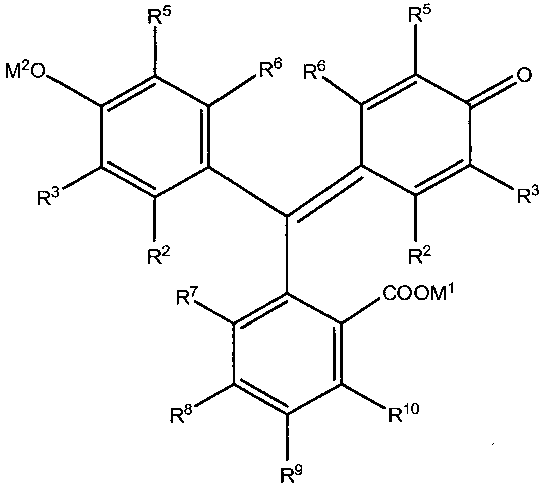 wherein
R² is selected from the group consisting of hydrogen, nitro, amino and alkyl;
R³ is selected from the group consisting of hydrogen, aryl, alkyl, nitro, acetamido and alkoxy;
R⁵ is selected from the group consisting of hydrogen, halo, alkoxy and alkyl;
R⁶ is selected from the group consisting of hydrogen and alkyl;
R⁷, R⁸, R⁹ and R¹⁰ are all hydrogen;
optionally, one of the carbons connected to R², R³, R⁵ or R⁶ can be substituted with a nitrogen atom; and
M¹ and M² are each independently a hydrogen atom, a metal ion or an ammonium ion, provided that at least one of M¹ or M² is a metal ion or an ammonium ion.

Prior to the present invention, it was generally considered extremely difficult if not impossible to make a broad spectrum of colored phthalein compositions which provide the entire spectrum of colors from yellow, orange, red, pink, magenta, purple, violet, blue, green and black. Additionally, preparation of phthaleins, up until the present invention did not generally provide high yield, high purity phthaleins by a simple process.

Up until the present invention, there also existed a need to develop color changing systems which were non-toxic and washable. Food dyes are non-toxic but do not change color and leave stains and are thus not washable. Most known dyes/pigments generate stains and leave residue or spots on the substrates. Therefore, the present invention provides phthaleins and compositions thereof that develop a color and which can be easily washable from skin, fabric, leather, hard/porous surfaces such as stones, brick, concrete, and wood (finished/unfinished).

The present invention overcomes some of the disadvantages of known dyes and pigments by providing color compositions which can change color and can provide the spectrum of colors from yellow, orange, red, pink, magenta, purple, violet, blue, green and black.

The present invention provides bubble compositions which include acid-base indicator dyes compositions that can change color from colored to colorless, one color to another color, or colorless to a colored under appropriate conditions.

The present invention surprisingly provides color changing, color disappearing bubble compositions, that have a uniform coloration about the bubble. Additionally, the present invention provides compositions that have film forming capabilities such that the resultant bubble can withstand physical contact with a surface. The film formed bubble can be colored, have a color changing composition, or can be without color.

The compositions of the present invention are aqueous compositions that include a surfactant and an acid-base indicator colorant according to claim 1. The compositions provide a bubble that begins as a uniformly colored bubble, but then changes coloration or the coloration disappears within a few seconds to within a few minutes. Further suitable colorants include leuco dyes and/or metal salts. When a leuco dye is included in the composition, an electron accepting compound or oxidizing agent is generally included. The compositions are non-toxic and/or washable, if necessary.

In another embodiment, the present invention provides compositions that provide film forming bubbles. The film forming compositions include a film forming resin and, optionally, a colorant as described herein. Generally the film forming resin is a polymeric material that can form a film about the surface of the bubble, such that the resultant bubble can withstand contact with a surface. The film forming compositions provide color changing bubbles. The compositions are non-toxic and/or washable, if necessary.

In still another embodiment, the present invention provides methods to prepare compositions that provide the various bubble producing solutions used throughout the present specification.

It should be understood, that the salt form of the indicator can be isolated prior to use or prepared in situ. Ideally, the salt is formed as a mono-salt or a di-salt, meaning that excess base is not present and either 1 or 2 equivalents of base react with the acidic protons of the indicator.

The following table provides phthaleins of particular interest.

| **R²** | **R³** | **R⁵** | **R⁶** | **Color** |
|---|---|---|---|---|
| H | phenyl | H | H | purple |
| H | *i*-propyl | *i*-propyl | H | violet |
| H | Me | H | Me | blue |
| H | OMe | OMe | H | teal |
| H | Me | Me | H | purple |
| H | Et | H | H | magenta |
| H | *i*-propyl | H | H | pink |
| H | OMe | H | H | blue |
| Me | Me | Me | H | teal |
| H | Me | H | H | magenta |
| H | *i*-propyl | H | Me | blue |
| H | Me | Br | H | purple |
| H | *i*-propyl | Br | Me | teal |
| H | *sec*-butyl | H | H | pink |
| H | NO₂ | H | H | yellow |

Additionally, substituted hydrazides are useful in the compositions of the invention and can have one of two formulae:

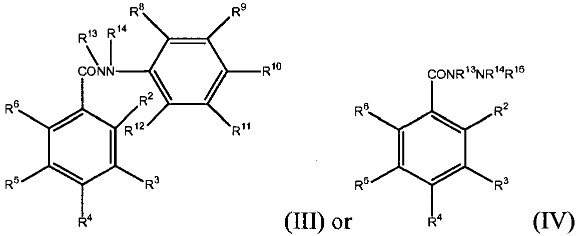

wherein R² through R⁶ are as defined above and R⁸ through R¹² are the same substituents as R² through R⁶. R¹³, R¹⁴ and R¹⁵ (if present) are each, independently of one another, a hydrogen atom, an alkyl group, a substituted alkyl group, any aryl group or a substituted aryl group.

In certain embodiments for compound formulae (III), R¹³, R¹⁴ and R¹⁵ are all hydrogen atoms.

In certain aspects, compounds of formulae (III) can have one or more hydroxyl groups, which can be deprotonated to form a salt. For example, formulae (IIIa) provides one isomer where a hydroxyl is present at the R² position as a salt. M² is as defined above for M¹. It should be understood that one or more of R² through R¹² could have a hydroxyl at that given position, and that hydroxyl could be in a salt form.

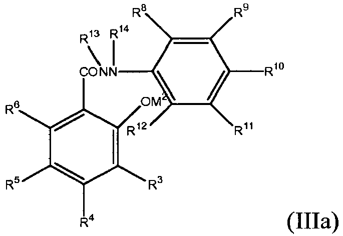

"Alkyl," by itself or as part of another substituent, refers to a saturated or unsaturated, branched, straight-chain or cyclic monovalent hydrocarbon radical derived by the removal of one hydrogen atom from a single carbon atom of a parent alkane, alkene or alkyne. Typical alkyl groups include, but are not limited to, methyl; ethyls such as ethanyl, ethenyl, ethynyl; propyls such as propan-1-yl, propan-2-yl, cyclopropan-1-yl, prop-1-en-1-yl, prop-1-en-2-yl, prop-2-en-1-yl (allyl), cycloprop-1-en-1-yl; cycloprop-2-en-1-yl, prop-1-yn-1-yl , prop-2-yn-1-yl, *etc*.; butyls such as butan-1-yl, butan-2-yl, 2-methyl-propan-1-yl, 2-methyl-propan-2-yl, cyclobutan-1-yl, but-1-en-1-yl, but-1-en-2-yl, 2-methyl-prop-1-en-1-yl, but-2-en-1-yl , but-2-en-2-yl, buta-1,3-dien-1-yl, buta-1,3-dien-2-yl, cyclobut-1-en-1-yl, cyclobut-1-en-3-yl, cyclobuta-1,3-dien-1-yl, but-1-yn-1-yl, but-1-yn-3-yl, but-3-yn-1-yl, *etc.*; and the like.

The term "alkyl" is specifically intended to include groups having any degree or level of saturation, *i.e*., groups having exclusively single carbon-carbon bonds, groups having one or more double carbon-carbon bonds, groups having one or more triple carbon-carbon bonds and groups having mixtures of single, double and triple carbon-carbon bonds. Where a specific level of saturation is intended, the expressions "alkanyl," "alkenyl," and "alkynyl" are used. Preferably, an alkyl group comprises from 1 to 15 carbon atoms (C₁-C₁₅ alkyl), more preferably from 1 to 10 carbon atoms (C₁-C₁₀ alkyl) and even more preferably from 1 to 6 carbon atoms (C₁-C₆ alkyl or lower alkyl).

"Alkanyl," by itself or as part of another substituent, refers to a saturated branched, straight-chain or cyclic alkyl radical derived by the removal of one hydrogen atom from a single carbon atom of a parent alkane. Typical alkanyl groups include, but are not limited to, methanyl; ethanyl; propanyls such as propan-1-yl, propan-2-yl (isopropyl), cyclopropan-1-yl, etc.; butanyls such as butan-1-yl, butan-2-yl (*sec*-butyl), 2-methyl-propan-1-yl (isobutyl), 2-methyl-propan-2-yl (*t*-butyl), cyclobutan-1-yl, *etc.*; and the like.

"Alkenyl," by itself or as part of another substituent, refers to an unsaturated branched, straight-chain or cyclic alkyl radical having at least one carbon-carbon double bond derived by the removal of one hydrogen atom from a single carbon atom of a parent alkene. The group may be in either the *cis* or *trans* conformation about the double bond(s). Typical alkenyl groups include, but are not limited to, ethenyl; propenyls such as prop-1-en-1-yl , prop-1-en-2-yl, prop-2-en-1-yl (allyl), prop-2-en-2-yl, cycloprop-1-en-1-yl; cycloprop-2-en-1-yl ; butenyls such as but-1-en-1-yl, but-1-en-2-yl, 2-methyl-prop-1-en-1-yl, but-2-en-1-yl , but-2-en-1-yl, but-2-en-2-yl, buta-1,3-dien-1-yl, buta-1,3-dien-2-yl, cyclobut-1-en-1-yl, cyclobut-1-en-3-yl, cyclobuta-1,3-dien-1-yl, *etc.*; and the like.

"Alkynyl," by itself or as part of another substituent refers to an unsaturated branched, straight-chain or cyclic alkyl radical having at least one carbon-carbon triple bond derived by the removal of one hydrogen atom from a single carbon atom of a parent alkyne. Typical alkynyl groups include, but are not limited to, ethynyl; propynyls such as prop-1-yn-1-yl, prop-2-yn-1-yl, etc.; butynyls such as but-1-yn-1-yl, but-1-yn-3-yl, but-3-yn-1-yl, *etc.*; and the like.

"Alkyldiyl" by itself or as part of another substituent refers to a saturated or unsaturated, branched, straight-chain or cyclic divalent hydrocarbon group derived by the removal of one hydrogen atom from each of two different carbon atoms of a parent alkane, alkene or alkyne, or by the removal of two hydrogen atoms from a single carbon atom of a parent alkane, alkene or alkyne. The two monovalent radical centers or each valency of the divalent radical center can form bonds with the same or different atoms. Typical alkyldiyl groups include, but are not limited to, methandiyl; ethyldiyls such as ethan-1,1-diyl, ethan-1,2-diyl, ethen-1,1-diyl, ethen-1,2-diyl; propyldiyls such as propan-1,1-diyl, propan-1,2-diyl, propan-2,2-diyl, propan-1,3-diyl, cyclopropan-1,1-diyl, cyclopropan-1,2-diyl, prop-1-en-1,1-diyl, prop-1-en-1,2-diyl, prop-2-en-1,2-diyl, prop-1-en-1,3-diyl, cycloprop-1-en-1,2-diyl, cycloprop-2-en-1,2-diyl, cycloprop-2-en-1,1-diyl, prop-1-yn-1,3-diyl, etc.; butyldiyls such as, butan-1,1-diyl, butan-1,2-diyl, butan-1,3-diyl, butan-1,4-diyl, butan-2,2-diyl, 2-methyl-propan-1,1-diyl, 2-methyl-propan-1,2-diyl, cyclobutan-1,1-diyl; cyclobutan-1,2-diyl, cyclobutan-1,3-diyl, but-1-en-1,1-diyl, but-1-en-1,2-diyl, but-1-en-1,3-diyl, but-1-en-1,4-diyl, 2-methyl-prop-1-en-1,1-diyl, 2-methanylidene-propan-1,1-diyl, buta-1,3-dien-1,1-diyl, buta-1,3-dien-1,2-diyl, buta-1,3-dien-1,3-diyl, buta-1,3-dien-1,4-diyl, cyclobut-1-en-1,2-diyl, cyclobut-1-en-1,3-diyl, cyclobut-2-en-1,2-diyl, cyclobuta-1,3-dien-1,2-diyl, cyclobuta-1,3-dien-1,3-diyl, but-1-yn-1,3-diyl, but-1-yn-1,4-diyl, buta-1,3-diyn-1,4-diyl, etc.; and the like. Where specific levels of saturation are intended, the nomenclature alkanyldiyl, alkenyldiyl and/or alkynyldiyl is used. Where it is specifically intended that the two valencies are on the same carbon atom, the nomenclature "alkylidene" is used. In preferred embodiments, the alkyldiyl group comprises from 1 to 6 carbon atoms (C1-C6 alkyldiyl). Also preferred are saturated acyclic alkanyldiyl groups in which the radical centers are at the terminal carbons, *e.g*., methandiyl (methano); ethan-1,2-diyl (ethano); propan-1,3-diyl (propano); butan-1,4-diyl (butano); and the like (also referred to as alkylenos, defined *infra*).

"Alkyleno," by itself or as part of another substituent, refers to a straight-chain saturated or unsaturated alkyldiyl group having two terminal monovalent radical centers derived by the removal of one hydrogen atom from each of the two terminal carbon atoms of straight-chain parent alkane, alkene or alkyne. The locant of a double bond or triple bond, if present, in a particular alkyleno is indicated in square brackets. Typical alkyleno groups include, but are not limited to, methano; ethylenos such as ethano, etheno, ethyno; propylenos such as propano, prop[1]eno, propa[1,2]dieno, prop[1]yno, etc.; butylenos such as butano, but[1]eno, but[2]eno, buta[1,3]dieno, but[1]yno, but[2]yno, buta[1,3]diyno, etc.; and the like. Where specific levels of saturation are intended, the nomenclature alkano, alkeno and/or alkyno is used. In preferred embodiments, the alkyleno group is (C1-C6) or (C1-C3) alkyleno. Also preferred are straight-chain saturated alkano groups, *e.g*., methano, ethano, propano, butano, and the like.

"Alkoxy," by itself or as part of another substituent, refers to a radical of the formula -OR, where R is an alkyl or cycloalkyl group as defined herein. Representative examples alkoxy groups include, but are not limited to, methoxy, ethoxy, propoxy, isopropoxy, butoxy, tert-butoxy, cyclopropyloxy, cyclopentyloxy, cyclohexyloxy and the like.

"Alkoxycarbonyl," by itself or as part of another substituent, refers to a radical of the formula -C(O)-alkoxy, where alkoxy is as defined herein.

"Alkylthio," by itself or as part of another substituent, refers to a radical of the formula -SR, where R is an alkyl or cycloalkyl group as defined herein. Representative examples of Alkylthio groups include, but are not limited to, methylthio, ethylthio, propylthio, isopropylthio, butylthio tert-butylthio, cyclopropylthio, cyclopentylthio, cyclohexylthio, and the like.

"Aryl," by itself or as part of another substituent, refers to a monovalent aromatic hydrocarbon group derived by the removal of one hydrogen atom from a single carbon atom of a parent aromatic ring system, as defined herein. Typical aryl groups include, but are not limited to, groups derived from aceanthrylene, acenaphthylene, acephenanthrylene, anthracene, azulene, benzene, chrysene, coronene, fluoranthene, fluorene, hexacene, hexaphene, hexalene, *as*-indacene, *s*-indacene, indane, indene, naphthalene, octacene, octaphene, octalene, ovalene, penta-2,4-diene, pentacene, pentalene, pentaphene, perylene, phenalene, phenanthrene, picene, pleiadene, pyrene, pyranthrene, rubicene, triphenylene, trinaphthalene and the like. Preferably, an aryl group comprises from 6 to 20 carbon atoms (C₆-C₂₀ aryl), more preferably from 6 to 15 carbon atoms (C₆-C₁₅ aryl) and even more preferably from 6 to 10 carbon atoms (C₆-C₁₀ aryl).

"Arylalkyl," by itself or as part of another substituent, refers to an acyclic alkyl group in which one of the hydrogen atoms bonded to a carbon atom, typically a terminal or *sp*³ carbon atom, is replaced with an aryl group as, as defined herein. Typical arylalkyl groups include, but are not limited to, benzyl, 2-phenylethan-1-yl, 2-phenylethen-1-yl, naphthylmethyl, 2-naphthylethan-1-yl, 2-naphthylethen-1-yl, naphthobenzyl, 2-naphthophenylethan-1-yl and the like. Where specific alkyl moieties are intended, the nomenclature arylalkanyl, arylalkenyl and/or arylalkynyl is used. Preferably, an arylalkyl group is (C₆-C₃₀) arylalkyl, *e.g*., the alkanyl, alkenyl or alkynyl moiety of the arylalkyl group is (C₁-C₁₀) alkyl and the aryl moiety is (C₆-C₂₀) aryl, more preferably, an arylalkyl group is (C₆-C₂₀) arylalkyl, *e.g*., the alkanyl, alkenyl or alkynyl moiety of the arylalkyl group is (C₁-C₈) alkyl and the aryl moiety is (C₆-C₁₂) aryl, and even more preferably, an arylalkyl group is (C₆-C₁₅) arylalkyl, *e.g*., the alkanyl, alkenyl or alkynyl moiety of the arylalkyl group is (C₁-C₅) alkyl and the aryl moiety is (C₆-C₁₀) aryl.

"Aryloxy," by itself or as part of another substituent, refers to a radical of the formula -O-aryl, where aryl is as defined herein.

"Arylalkyloxy, by itself or as part of another substituent, refers to a radical of the formula -O-arylalkyl, where arylalkyl is as defined herein.

"Aryloxycarbonyl," by itself or as part of another substituent, refers to a radical of the formula -C(O)-O-aryl, where aryl is as defined herein.

"Carbamoyl," by itself or as part of another substituent, refers to a radical of the formula -C(O)NR'R", where R' and R" are each, independently of one another, selected from the group consisting of hydrogen, alkyl and cycloalkyl as defined herein, or alternatively, R' and R", taken together with the nitrogen atom to which they are bonded, form a 5-, 6- or 7-membered cycloheteroalkyl ring as defined herein, which may optionally include from 1 to 4 of the same or different additional heteroatoms selected from the group consisting of O, S and N.

"Compounds of the invention" refers to compounds encompassed by the various descriptions and structural formulae disclosed herein. The compounds of the invention may be identified by either their chemical structure and/or chemical name. When the chemical structure and chemical name conflict, the chemical structure is determinative of the identity of the compound. The compounds of the invention may contain one or more chiral centers and/or double bonds and therefore may exist as stereoisomers, such as double-bond isomers (*i.e*., geometric isomers), rotamers, enantiomers or diastereomers. Accordingly, when stereochemistry at chiral centers is not specified, the chemical structures depicted herein encompass all possible configurations at those chiral centers including the stereoisomerically pure form (*e.g*., geometrically pure, enantiomerically pure or diastereomerically pure) and enantiomeric and stereoisomeric mixtures. Enantiomeric and stereoisomeric mixtures can be resolved into their component enantiomers or stereoisomers using separation techniques or chiral synthesis techniques well known to the skilled artisan. The compounds of the invention may also exist in several tautomeric forms including the enol form, the keto form and mixtures thereof. Accordingly, the chemical structures depicted herein encompass all possible tautomeric forms of the illustrated compounds. The compounds of the invention may also include isotopically labeled compounds where one or more atoms have an atomic mass different from the atomic mass conventionally found in nature. Examples of isotopes that may be incorporated into the compounds of the invention include, but are not limited to, ²H, ³H, ¹¹C, ¹³C, ¹⁴C, ¹⁵N, ¹⁸O, ¹⁷O, ³¹P, ³²P, ³⁵S, ¹⁸F and ³⁶Cl. Compounds of the invention may exist in unsolvated forms as well as solvated forms, including hydrated forms and as N-oxides. In general, the hydrated, solvated and N-oxide forms are within the scope of the present invention. Certain compounds of the present invention may exist in multiple crystalline or amorphous forms. In general, all physical forms are equivalent for the uses contemplated by the present invention and are intended to be within the scope of the present invention.

"Cycloalkyl," by itself or as part of another substituent, refers to a saturated or unsaturated cyclic alkyl radical, as defined herein. Where a specific level of saturation is intended, the nomenclature "cycloalkanyl" or "cycloalkenyl" is used. Typical cycloalkyl groups include, but are not limited to, groups derived from cyclopropane, cyclobutane, cyclopentane, cyclohexane, and the like. Preferably, the cycloalkyl group comprises from 3 to 10 ring atoms (C₃-C₁₀ cycloalkyl) and more preferably from 3 to 7 ring atoms (C₃-C₇ cycloalkyl).

"Cycloheteroalkyl," by itself or as part of another substituent, refers to a saturated or unsaturated cyclic alkyl radical in which one or more carbon atoms (and optionally any associated hydrogen atoms) are independently replaced with the same or different heteroatom. Typical heteroatoms to replace the carbon atom(s) include, but are not limited to, N, P, O, S, Si, *etc.* Where a specific level of saturation is intended, the nomenclature "cycloheteroalkanyl" or "cycloheteroalkenyl" is used. Typical cycloheteroalkyl groups include, but are not limited to, groups derived from epoxides, azirines, thiiranes, imidazolidine, morpholine, piperazine, piperidine, pyrazolidine, pyrrolidone, quinuclidine, and the like. Preferably, the cycloheteroalkyl group comprises from 3 to 10 ring atoms (3-10 membered cycloheteroalkyl) and more preferably from 5 to 7 ring atoms (5-7 membered cycloheteroalkyl).

A cycloheteroalkyl group may be substituted at a heteroatom, for example, a nitrogen atom, with a lower alkyl group. As specific examples, N-methyl-imidazolidinyl, N-methyl-morpholinyl, N-methyl-piperazinyl, N-methyl-piperidinyl, N-methyl-pyrazolidinyl and N-methyl-pyrrolidinyl are included within the definition of "cycloheteroalkyl." A cycloheteralkyl group may be attached to the remainder of the molecule *via* a ring carbon atom or a ring heteroatom.

"Dialkylamino" or "Monoalkylamino," by themselves or as part of other substituents, refer to radicals of the formula -NRR and -NHR, respectively, where each R is independently selected from the group consisting of alkyl and cycloalkyl, as defined herein. Representative examples of dialkylamino groups include, but are not limited to, dimethylamino, methylethylamino, di-(1-methylethyl)amino, (cyclohexyl)(methyl)amino, (cyclohexyl)(ethyl)amino, (cyclohexyl)(propyl)amino and the like. Representative examples of monalkylamino groups include, but are not limited to, methylamino, ethylamino, propylamino, isopropylamino, cyclohexylamino, and the like.

"Halogen" or "Halo," by themselves or as part of another substituent, refer to a fluoro, chloro, bromo and/or iodo radical.

"Haloalkyl," by itself or as part of another substituent, refers to an alkyl group as defined herein in which one or more of the hydrogen atoms is replaced with a halo group. The term "haloalkyl" is specifically meant to include monohaloalkyls, dihaloalkyls, trihaloalkyls, *etc*. up to perhaloalkyls. The halo groups substituting a haloalkyl can be the same, or they can be different. For example, the expression "(C₁-C₂) haloalkyl" includes 1-fluoromethyl, 1-fluoro-2-chloroethyl, difluoromethyl, trifluoromethyl, 1-fluoroethyl, 1, 1-difluoroethyl, 1, 2-difluoroethyl, 1,1,1-trifluoroethyl, perfluoroethyl, etc. "Haloalkyloxy," by itself or as part of another substituent, refers to a group of the formula -O-haloalkyl, where haloalkyl is as defined herein.

"Heteroalkyl," "Heteroalkanyl," "Heteroalkenyl," "Heteroalkynyl," "Heteroalkyldiyl" and "Heteroalkyleno," by themselves or as part of other substituents, refer to alkyl, alkanyl, alkenyl, alkynyl, alkyldiyl and alkyleno groups, respectively, in which one or more of the carbon atoms (and optionally any associated hydrogen atoms), are each, independently of one another, replaced with the same or different heteroatoms or heteroatomic groups. Typical heteroatoms or heteroatomic groups which can replace the carbon atoms include, but are not limited to, O, S, N, Si, -NH-, -S(O)-, -S(O)₂-, -S(O)NH-, -S(O)₂NH- and the like and combinations thereof. The heteroatoms or heteroatomic groups may be placed at any interior position of the alkyl, alkenyl or alkynyl groups. Examples of such heteroalkyl, heteroalkanyl, heteroalkenyl and/or heteroalkynyl groups include -CH₂-CH₂-O-CH₃, -CH₂-CH₂-NH-CH₃, -CH₂-CH₂-N(CH₃)-CH₃, -CH₂-S-CH₂,-CH₃, -CH₂-CH₂-S(O)-CH₃, -CH₂-CH₂-S(O)₂-CH₃, -CH=CH-O-CH₃, -CH₂-CH=N-O-CH₃, and - CH₂-CH₂-O-C=CH. For heteroalkyldiyl and heteroalkyleno groups, the heteratom or heteratomic group can also occupy either or both chain termini. For such groups, no orientation of the group is implied.

"Heteroaryl," by itself or as part of another substituent, refers to a monovalent heteroaromatic radical derived by the removal of one hydrogen atom from a single atom of a parent heteroaromatic ring systems, as defined herein. Typical heteroaryl groups include, but are not limited to, groups derived from acridine, β-carboline, chromane, chromene, cinnoline, furan, imidazole, indazole, indole, indoline, indolizine, isobenzofuran, isochromene, isoindole, isoindoline, isoquinoline, isothiazole, isoxazole, naphthyridine, oxadiazole, oxazole, perimidine, phenanthridine, phenanthroline, phenazine, phthalazine, pteridine, purine, pyran, pyrazine, pyrazole, pyridazine, pyridine, pyrimidine, pyrrole, pyrrolizine, quinazoline, quinoline, quinolizine, quinoxaline, tetrazole, thiadiazole, thiazole, thiophene, triazole, xanthene, and the like. Preferably, the heteroaryl group comprises from 5 to 20 ring atoms (5-20 membered heteroaryl), more preferably from 5 to 10 ring atoms (5-10 membered heteroaryl). Preferred heteroaryl groups are those derived from furan, thiophene, pyrrole, benzothiophene, benzofuran, benzimidazole, indole, pyridine, pyrazole, quinoline, imidazole, oxazole, isoxazole and pyrazine.

"Heteroarylalkyl" by itself or as part of another substituent refers to an acyclic alkyl group in which one of the hydrogen atoms bonded to a carbon atom, typically a terminal or *sp³* carbon atom, is replaced with a heteroaryl group. Where specific alkyl moieties are intended, the nomenclature heteroarylalkanyl, heteroarylakenyl and/or heteroarylalkynyl is used. In preferred embodiments, the heteroarylalkyl group is a 6-21 membered heteroarylalkyl, *e.g*., the alkanyl, alkenyl or alkynyl moiety of the heteroarylalkyl is (C1-C6) alkyl and the heteroaryl moiety is a 5-15-membered heteroaryl. In particularly preferred embodiments, the heteroarylalkyl is a 6-13 membered heteroarylalkyl, *e.g*., the alkanyl, alkenyl or alkynyl moiety is (C1-C3) alkyl and the heteroaryl moiety is a 5-10 membered heteroaryl.

"Parent Aromatic Ring System" refers to an unsaturated cyclic or polycyclic ring system having a conjugated π electron system. Specifically included within the definition of "parent aromatic ring system" are fused ring systems in which one or more of the rings are aromatic and one or more of the rings are saturated or unsaturated, such as, for example, fluorene, indane, indene, phenalene, *etc*. Typical parent aromatic ring systems include, but are not limited to, aceanthrylene, acenaphthylene, acephenanthrylene, anthracene, azulene, benzene, chrysene, coronene, fluoranthene, fluorene, hexacene, hexaphene, hexalene, *as*-indacene, *s*-indacene, indane, indene, naphthalene, octacene, octaphene, octalene, ovalene, penta-2,4-diene, pentacene, pentalene, pentaphene, perylene, phenalene, phenanthrene, picene, pleiadene, pyrene, pyranthrene, rubicene, triphenylene, trinaphthalene and the like.

"Parent Heteroaromatic Ring System" refers to a parent aromatic ring system in which one or more carbon atoms (and optionally any associated hydrogen atoms) are each independently replaced with the same or different heteroatom. Typical heteroatoms to replace the carbon atoms include, but are not limited to, N, P, O, S, Si, *etc.* Specifically included within the definition of "parent heteroaromatic ring system" are fused ring systems in which one or more of the rings are aromatic and one or more of the rings are saturated or unsaturated, such as, for example, benzodioxan, benzofuran, chromane, chromene, indole, indoline, xanthene, *etc*. Typical parent heteroaromatic ring systems include, but are not limited to, arsindole, carbazole, β-carboline, chromane, chromene, cinnoline, furan, imidazole, indazole, indole, indoline, indolizine, isobenzofuran, isochromene, isoindole, isoindoline, isoquinoline, isothiazole, isoxazole, naphthyridine, oxadiazole, oxazole, perimidine, phenanthridine, phenanthroline, phenazine, phthalazine, pteridine, purine, pyran, pyrazine, pyrazole, pyridazine, pyridine, pyrimidine, pyrrole, pyrrolizine, quinazoline, quinoline, quinolizine, quinoxaline, tetrazole, thiadiazole, thiazole, thiophene, triazole, xanthene and the like.

"Metal ion" or "Metal Salt" refers to a salt of a compound of the invention which is made with counterions understood in the art to be generally acceptable for pharmaceutical uses and which possesses the desired pharmacological activity of the parent compound. Such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl) benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid and the like; or (2) salts formed when an acidic proton present in the parent compound is replaced by a metal ion, *e.g*., an alkali metal ion, an alkaline earth ion, or an aluminum ion; or coordinates with an organic base such as ethanolamine, diethanolamine, triethanolamine, N-methylglucamine, morpholine, piperidine, dimethylamine, diethylamine and the like. Also included are salts of amino acids such as arginates and the like, and salts of organic acids like glucurmic or galactunoric acids and the like (*see*, *e.g.,* Berge et al., 1977, J. Pharm. Sci. 66:1-19).

"Pharmaceutically acceptable vehicle" refers to a diluent, adjuvant, excipient or carrier with which a compound of the invention is administered.

"Substituted," when used to modify a specified group or radical, means that one or more hydrogen atoms of the specified group or radical are each, independently of one another, replaced with the same or different substituent(s). Substituent groups useful for substituting saturated carbon atoms in the specified group or radical include, but are not limited to -R^{a}, halo, -O-, =O, -OR^{b}, -SR^{b}, -S⁻, =S, -NR^{c}R^{c}, =NR^{b}, =N-OR^{b}, trihalomethyl, -CF₃, -CN, -OCN, -SCN, -NO, -NO₂, =N₂, -N₃, -S(O)₂R^{b}, -S(O)₂O⁻, -S(O)₂OR^{b}, -OS(O)₂R^{b}, -OS(O)₂O⁻, -OS(O)₂OR^{b}, -P(O)(O⁻)₂, -P(O)(OR^{b})(O⁻), -P(O)(OR^{b})(OR^{b}), -C(O)R^{b}, -C(S)R^{b}, -C(NR^{b})R^{b}, -C(O)O-, -C(O)OR^{b}, -C(S)OR^{b}, -C(O)NR^{c}R^{c}, -C(NR^{b})NR^{c}R^{c}, -OC(O)R^{b}, -OC(S)R^{b}, -OC(O)O-, -OC(O)OR^{b}, -OC(S)OR^{b}, -NR^{b}C(O)R^{b}, -NR^{b}C(S)R^{b}, -NR^{b}C(O)O⁻, -NR^{b}C(O)OR^{b}, -NR^{b}C(S)OR^{b}, -NR^{b}C(O)NR^{c}R^{c}, -NR^{b}C(NR^{b})R^{b} and -NR^{b}C(NR^{b})NR^{c}R^{c}, where R^{a} is selected from the group consisting of alkyl, cycloalkyl, heteroalkyl, cycloheteroalkyl, aryl, arylalkyl, heteroaryl and heteroarylalkyl; each R^{b} is independently hydrogen or R^{a}; and each R^{c} is independently R^{b} or alternatively, the two R^{c}s are taken together with the nitrogen atom to which they are bonded form a 5-, 6- or 7-membered cycloheteroalkyl which may optionally include from 1 to 4 of the same or different additional heteroatoms selected from the group consisting of O, N and S. As specific examples, -NR^{c}R^{c} is meant to include NH₂, -NH-alkyl, N-pyrrolidinyl and N-morpholinyl.

Similarly, substituent groups useful for substituting unsaturated carbon atoms in the specified group or radical include, but are not limited to, -R^{a}, halo, -O⁻, -OR^{b}, -SR^{b}, -S⁻, -NR^{c}R^{c}, trihalomethyl, -CF₃, -CN, -OCN, -SCN, -NO, -NO₂, -N₃, -S(O)₂R^{b}, -S(O)₂O⁻, -S(O)₂OR^{b}, -OS(O)₂R^{b}, -OS(O)₂O⁻, -OS(O)₂OR^{b}, -P(O)(O⁻)₂, -P(O)(OR^{b})(O⁻), -P(O)(OR^{b})(OR^{b}), -C(O)R^{b}, -C(S)R^{b}, -C(NR^{b})R^{b}, -C(O)O⁻, -C(O)OR^{b}, -C(S)OR^{b}, -C(O)NR^{c}R^{c}, -C(NR^{b})NR^{c}R^{c}, -OC(O)R^{b}, -OC(S)R^{b}, -OC(O)O⁻, -OC(O)OR^{b}, -OC(S)OR^{b}, -NR^{b}C(O)R^{b}, -NR^{b}C(S)R^{b}, -NR^{b}C(O)O⁻, -NR^{b}C(O)OR^{b}, -NR^{b}C(S)OR^{b}, -NR^{b}C(O)NR^{c}R^{c}, -NR^{b}C(NR^{b})R^{b} and -NR^{b}C(NR^{b})NR^{c}R^{c}, where R^{a}, R^{b} and R^{c} are as previously defined.

Substituent groups useful for substituting nitrogen atoms in heteroalkyl and cycloheteroalkyl groups include, but are not limited to, -R^{a}, -O⁻, -OR^{b}, -SR^{b}, -S⁻, -NR^{c}R^{c}, trihalomethyl, -CF₃, -CN, -NO, -NO₂, -S(O)₂R^{b}, -S(O)₂O⁻, -S(O)₂OR^{b}, -OS(O)₂R^{b}, -OS(O)₂O⁻, -OS(O)₂OR^{b}, -P(O)(O⁻)₂, -P(O)(OR^{b})(O⁻), -P(O)(OR^{b})(OR^{b}), -C(O)R^{b}, -C(S)R^{b}, -C(NR^{b})R^{b}, -C(O)OR^{b}, -C(S)OR^{b}, -C(O)NR^{c}R^{c}, -C(NR^{b})NR^{c}R^{c}, -OC(O)R^{b}, -OC(S)R^{b}, -OC(O)OR^{b}, -OC(S)OR^{b}, -NR^{b}C(O)R^{b}, -NR^{b}C(S)R^{b}, -NR^{b}C(O)OR^{b}, -NR^{b}C(S)OR^{b}, -NR^{b}C(O)NR^{c}R^{c}, -NR^{b}C(NR^{b})R^{b} and -NR^{b}C(NR^{b})NR^{c}R^{c}, where R^{a}, R^{b} and R^{c} are as previously defined.

Substituent groups from the above lists useful for substituting other specified groups or atoms will be apparent to those of skill in the art.

The substituents used to substitute a specified group can be further substituted, typically with one or more of the same or different groups selected from the various groups specified above.

"Sulfamoyl," by itself or as part of another substituent, refers to a radical of the formula -S(O)₂NR'R", where R' and R" are each, independently of one another, selected from the group consisting of hydrogen, alkyl and cycloalkyl as defined herein, or alternatively, R' and R", taken together with the nitrogen atom to which they are bonded, form a 5-, 6- or 7-membered cycloheteroalkyl ring as defined herein, which may optionally include from 1 to 4 of the same or different additional heteroatoms selected from the group consisting of O, S and N.

### Methods of Synthesis

The particular phthaleins described above can be obtained *via* synthetic methods illustrated below. It should be understood that in R², R³, R⁵, R⁶, R⁷, R⁸, R⁹ and R¹⁰, are as previously defined for structural formula (I).

Starting materials useful for preparing compounds of the invention and intermediates thereof are commercially available or can be prepared by well-known synthetic methods (*see, e.g.,* Harrison et al., "Compendium of Synthetic Organic Methods", Vols. 1-8 (John Wiley and Sons, 1971-1996); "Beilstein Handbook of Organic Chemistry," Beilstein Institute of Organic Chemistry, Frankfurt, Germany; Feiser et al., "Reagents for Organic Synthesis," Volumes 1-21, Wiley Interscience; Trost et al., "Comprehensive Organic Synthesis," Pergamon Press, 1991; "Theilheimer's Synthetic Methods of Organic Chemistry," Volumes 1-45, Karger, 1991; March, "Advanced Organic Chemistry," Wiley Interscience, 1991; Larock "Comprehensive Organic Transformations," VCH Publishers, 1989; Paquette, "Encyclopedia of Reagents for Organic Synthesis," 3d Edition, John Wiley & Sons, 1995). Other methods for synthesis of the compounds described herein and/or starting materials are either described in the art or will be readily apparent to the skilled artisan.

A typical synthesis is depicted in Scheme I, wherein 2 equivalents of a phenol or phenol equivalent are condensed with 1 equivalent of a phthalic anhydride or equivalent under essentially acid anhydrous conditions.

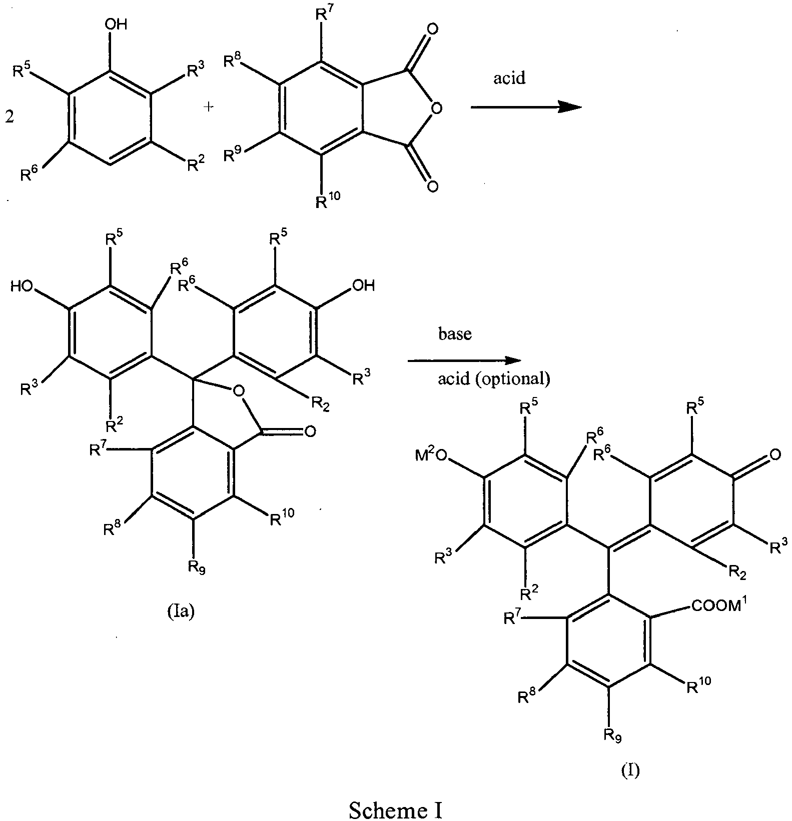

Generally, the phenol and anhydride are condensed in the presence of an acid under anhydrous conditions. For example, polyphosphoric acid and zinc chloride can be utilized. The carbon atom at 4-position -position with respect to the aromatic hydroxyl group must not be substituted as it is necessary for reaction. Polyphosphoric acid acts as a condensing agent as well as reaction medium. The reaction with only polyphosphoric acid afforded tarry products but when very small amount of zinc chloride was added to polyphosphoric acid, clean product was isolated. Very small amount of zinc chloride was found to increase yield and purity of the product. Polyphosphoric acid can be replaced with orthophosphoric acid, chlorosulfonic acid, methane sulfonic acid, trifluoroacetic acid or other acids under anhydrous conditions. Suitable solvents include nonprotic solvents known in the art such as tetrahydrofuran, dioxane, methylene chloride, ether, etc.

The reaction proceeds with the formation of an isobenzofuranone (Ia), which is then treated with a base under aqueous conditions. The salt can be isolated or the solution can be acidified to produce the protonated phenol/carboxylic acid. For example, one molar equivalent of Ia was condensed with either two molar equivalent of sodium hydroxide in 85% ethanol or two molar equivalent of sodium ethoxide in ethanol. The products are generally solids and can be easily purified via filtration, crystallization, and other methods known in the art.

Suitable phenols include, but are not limited to 2-nitrophenol, 3-nitrophenol, 2-chlorophenol, 3-chlorophenol, 2-bromophenol, 3-bromophenol, 2-iodophenol, 3-iodophenol, 2-fluorophenol, 3-fluorophenol, 2-aminophenol, 3-aminophenol, 2-acetamidophenol, 3-acetamidophenol, 2-cyanophenol, 3-cyanophenol, 2-methylphenol, 3-methylphenol, 2-ethylphenol, 3-ethylphenol, 2-proylphenol, 3-proylphenol, 2-isoproylphenol, 3-isoproylphenol, 2-butylphenol, 3-butylphenol, 2-isobutylphenol, 3-isobutylphenol, 2-pentylphenol, 3-pentylphenol 2-hexylphenol, 3-hexylphenol, 2-heptylphenol, 3-heptylphenol, 2-octylphenol, 3-octylphenol, 2-nonylphenol, 3-nonylphenol, 2-decylphenol, 3-decylphenol, 2-decylphenol, 2-methoxyphenol, 3-methoxyphenol, 2-ethoxyphenol, 3-ethoxyphenol, 2-propoxyphenol, 3-propoxyphenol, 2-isopropoxyphenol, 3-isopropoxyphenol, 2-butoxyphenol, 3-butoxyphenol, 2-isobutoxyphenol, 3-isobutoxylphenol, 2-allylphenol, 3-allylphenol, 2-vinylphenol, 3-vinylphenol, 2-phenylphenol, 3-phenylphenol, 2-phenoxyphenol, 3-phenoxyphenol, 2-cyclopropylphenol, 3-cyclopropylphenol, 2-cyclobutylphenol, 3-cyclobutylphenol, 2-cyclopentylphenol, 3-cyclopentylphenol, 2-cyclohexylphenol, 3-cyclohexylphenol, 2-cycloheptylphenol, 3-cycloheptylphenol, 2-cyclooctylphenol, 3-cyclooctylphenol, 2-cyclononylphenol, 3-cyclononylphenol, 2-cyclodecylphenol, 3-cyclodecylphenol, 2,3-dinitrophenol, 2,5-dinitrophenol, 2,6-dinitrophenol, 2,3-dimethylphenol, 2,5-dimethylphenol, 2,6-dimethylphenol, 2,3-diethylphenol, 2,5-diethylphenol, 2,6-diethylphenol, 2,3-diproplylphenol, 2,5-dipropylphenol, 2,6-dipropylphenol, 2,3-diisoproplylphenol, 2,5-diisopropylphenol, 2,6-diisopropylphenol, 2,3-dibutylphenol, 2,5-dibutylphenol, 2,6-dibutylphenol, 2,3-diisobutylphenol, 2,5-diisobutylphenol, 2,6-diisobutylphenol, 2,3-dipentylphenol, 2,5-dipentylphenol, 2,6-dipentylphenol, 2,3-dihexylphenol, 2,5-dihexylphenol, 2,6-dihexylphenol, 2,3-diheptylphenol, 2,5-diheptylphenol, 2,6-diheptylphenol, 2,3-dioctylphenol, 2,5-dioctylphenol, 2,6-dioctylphenol, 2,3-dinonylphenol, 2,5-dinonylphenol, 2,6-dinonylphenol, 2,3-didecylphenol, 2,5-didecylphenol, 2,6-didecylphenol, 2,3-dimethoxyphenol, 2,5-dimethoxyphenol, 2,6-dimethoxyphenol, 2,3-diethoxyphenol, 2,5-diethoxyphenol, 2,6-diethoxyphenol, 2,3-dipropoxyphenol, 2,5-dipropoxyphenol, 2,6-dipropoxyphenol, 2,3-diisopropoxyphenol, 2,5-diisopropoxyphenol, 2,6-diisopropoxyphenol, 2,3-dibutoxyphenol, 2,5-dibutoxyphenol, 2,6-dibutoxyphenol, 2,3-diisobutoxyphenol, 2,5-diisobutoxyphenol, 2,6-diisobutoxyphenol, 2,3-dipentoxyphenol, 2,5-dipentoxyphenol, 2,6-dipentoxyphenol, 2,3-dihexoxyphenol, 2,5-dihexoxyphenol, 2,6-dihexoxyphenol, 2,3-diheptoxyphenol, 2,5-diheptoxyphenol, 2,6-diheptoxyphenol, 2,3-dioctoxyphenol, 2,5-dioctoxyphenol, 2,6-dioctoxyphenol, 2,3-dinonoxyphenol, 2,5-dinonoxyphenol, 2,6-dinonoxyphenol, 2,3-didecyloxyphenol, 2,5-didecyloxyphenol, 2,6-didecyloxyphenol, 2,3-dichlorophenol, 2,5-dichlorophenol, 2,6-dichlorophenol, 2,3-dibromophenol, 2,5-dibromophenol, 2,6-dibromophenol, 2,3-diiodophenol, 2,5-diiodophenol, 2,6-diiodophenol, 2,3-difluorophenol, 2,5-difluorophenol, 2,6-difluorophenol, 2,3-diaminophenol, 2,5-diaminophenol, 2,6-diaminophenol, 2,3-diacetamidophenol, 2,5-diacetamidophenol, 2,6-diacetamidophenol, 2,3-dicyanophenol, 2,5-dicyanophenol, 2,6-dicyanophenol, 2,3-diallylphenol, 2,5-diallylphenol, 2,6-diallylphenol, 2,3-divinylphenol, 2,5-divinylphenol, 2,6-divinylphenol, 2,3-diphenylphenol, 2,5-diphenylphenol, 2,6-diphenylphenol, 2,3-diphenoxyphenol, 2,5-diphenoxyphenol, 2,6-diphenoxyphenol, 2,3-dicycloproylphenol, 2,5-dicyclopropylphenol, 2,6-dicyclopropylphenol, 2,3-dicyclobutylphenol, 2,5-dicyclobutylphenol, 2,6-dicyclobutylphenol, 2,3-dicyclopentylphenol, 2,5-dicyclopentylphenol, 2,6-dicyclopentylphenol, 2,3-dicyclohexylphenol, 2,5-dicyclohexylphenol, 2,6-dicyclohexylphenol, 2,3-dicycloheptylphenol, 2,5-dicycloheptylphenol, 2,6-dicycloheptylphenol, 2,3-dicyclooctylphenol, 2,5-dicyclooctylphenol, 2,6-dicyclooctylphenol, 2,3-dicyclononylphenol, 2,5-dicyclononylphenol, 2,6-dicyclononylphenol, 2,3-dicyclodecylphenol, 2,5-dicyclodecylphenol, 2,6-dicyclodecylphenol, 2,3,5-trimethylphenol, 2,3,6-trimethylphenol 2,3,5-triethylphenol, 2,3,6-triethylphenol, 2,3,5-tripropylphenol, 2,3,6-tripropylphenol, 2,3,5-tributylphenol, 2,3,6-tributylphenol, 2,3,5-trichlorophenol, 2,3,6-trichlorophenol, 2,3,5-tribromophenol, 2,3,6-tribromophenol, 2,3,5-triiodophenol, 2,3,6-triiodophenol, 2,3,5-trifluorophenol, 2,3,6-trifluorophenol, 2,3,5-trivinylphenol, 2,3,6-trivinylphenol, 2,3,5-triallylphenol, 2,3,6-triallylphenol, 2,3,5-triphenylphenol, 2,3,6-triphenylphenol, 2,3,5-triphenoxyphenol, 2,3,6-triphenoxyphenol, 2,3,5-trimethoxyphenol, 2,3,6-trimethoxyphenol, 2,3,5-triethoxyphenol, 2,3,6-triethoxyphenol, 2,3,5-tripropoxyphenol, 2,3,6-tripropoxyphenol, 2,3,5-tributoxyphenol, 2,3,6-tributoxyphenol, 2,3,5-trinitrophenol, 2,3,6-trinitrophenol, 2,3,5-triaminophenol, 2,3,6-triaminophenol, 2,3,5-triacetamidophenol, 2,3,6-triacetamidophenol, 2,3,5-tricyanophenol, 2,3,6-tricyanophenol, 3-(N,N-diethylamino)phenol, 2-tert-butyl-5-methylphenol, 2-tert-butyl-6-methylphenol, 3-methyl-2-nitrophenol, 5-methyl-2-nitrophenol, 6-methyl-2-nitrophenol, 3-ethyl-2-nitrophenol, 5-ethyl-2-nitrophenol, 6-ethyl-2-nitrophenol, 3-methoxyl-2-nitrophenol, 5-methoxy-2-nitrophenol, 6-methoxy-2-nitrophenol, 1-naphthaol, 2-naphthaol, 2-nitro-1-naphthol, 3-nitro-1-naphthol, 5-nitro-1-naphthol, 6-nitro-1-naphthol, 7-nitro-1-naphthol, 8-nitro-1-naphthol, 2-methyl-1-naphthol, 3-methyl-1-naphthol, 5-methyl-1-naphthol, 6-methyl-1-naphthol, 7-methyl-1-naphthol, 8-methyl-1-naphthol, 2-methoxy-1-naphthol, 3-methoxy-1-naphthol, 5-methoxy-1-naphthol, 6-methoxy-1-naphthol, 7-methoxy-1-naphthol, 8-methoxy-1-naphthol, 2-chloro-1-naphthol, 3-chloro-1-naphthol, 5-chloro-1-naphthol, 6-chloro-1-naphthol, 7-chloro-1-naphthol, 8-chloro-1-naphthol, 2-bromo-1-naphthol, 3-bromo-1-naphthol, 5-bromo-1-naphthol, 6-bromo-1-naphthol, 7-bromo-1-naphthol, 8-bromo-1-naphthol, 2-iodo-1-naphthol, 3-iodo-1-naphthol, 5-iodo-1-naphthol, 6-iodo-1-naphthol, 7-iodo-1-naphthol, 8-iodo-1-naphthol, 2-fluoro-1-naphthol, 3-fluoro-1-naphthol, 5-fluoro-1-naphthol, 6-fluoro-1-naphthol, 7-bromo-1-naphthol, 8-fluoro-1-naphthol, 2-cyano-1-naphthol, 3-cyano-1-naphthol, 5-cyano-1-naphthol, 6-cyano-1-naphthol, 7-cyano-1-naphthol, 8-cyano-1-naphthol, 8-hydroxyquinaldine and 2-quinoxalinol.

The term "phenol equivalent" is intended to include those compounds where, as described above, R² and R³, for example, form an aromatic, heterocyclic, or non-aromatic ring. Suitable compounds include naphthols for example.

Suitable phthalic anhydrides include but are not limited to phthalic anhydride, 3-nitrophthalic anhydride, 4-nitrophthalic anhydride, 5-nitrophthalic anhydride, 6-nitrophthalic anhydride, 3-chlorophthalic anhydride, 4-chlorophthalic anhydride, 5-chlorophthalic anhydride, 6-chlorophthalic anhydride, 3-bromophthalic anhydride, 4-bromophthalic anhydride, 5-bromophthalic anhydride, 6-bromophthalic anhydride, 3-iodophthalic anhydride, 4-iodophthalic anhydride, 5-iodophthalic anhydride, 6-iodophthalic anhydride, 3-fluorophthalic anhydride, 4-fluorophthalic anhydride, 5-fluorophthalic anhydride, 6-fluorophthalic anhydride, 3-methylphthalic anhydride, 4-methylphthalic anhydride, 5-methylphthalic anhydride, 6-methylphthalic anhydride, 3-ethylphthalic anhydride, 4-ethylphthalic anhydride, 5-ethylphthalic anhydride, 6-ethylphthalic anhydride, 3-methoxyphthalic anhydride, 4-methoxyphthalic anhydride, 5-methoxyphthalic anhydride, 6-methoxyphthalic anhydride, 3-cyanophthalic anhydride, 4-cyanophthalic anhydride, 5-cyanophthalic anhydride, 6-cyanophthalic anhydride, 3-aminophthalic anhydride, 4-aminophthalic anhydride, 5-aminophthalic anhydride, 6-aminophthalic anhydride, 3-acetamidophthalic anhydride, 4-acetamidophthalic anhydride, 5-acetamidophthalic anhydride, 6-acetamidophthalic anhydride, 3,4,5,6-tetrachlorophthalic anhydride, 3,4,5,6-tetrabromophthalic anhydride, 3,4,5,6-tetraiodophthalic anhydride, 3,4,5,6-tetrafluorophthalic anhydride, 3,4,5,6-tetranitrophthalic anhydride, 3,4,5,6-tetramethylphthalic anhydride, 3,4,5,6-tetraethylphthalic anhydride, 3,4,5,6-tetramethoxyphthalic anhydride, 3,4,5,6-tetracyanophthalic anhydride, 3,4,5,6-tetraaminophthalic anhydride, 3,4,5,6-tetraacetamidophthalic anhydride, naphthalic anhydride, 2-chloronaphthalic anhydride, 3-chloronaphthalic anhydride, 4-chloronaphthalic anhydride, 5-chloronaphthalic anhydride, 6-chloronaphthalic anhydride, 7-chloronaphthalic anhydride, 2-bromonaphthalic anhydride, 3-bromonaphthalic anhydride, 4-bromonaphthalic anhydride, 5-bromonaphthalic anhydride, 6-bromonaphthalic anhydride, 7-bromonaphthalic anhydride, 2-iodonaphthalic anhydride, 3-iodonaphthalic anhydride, 4-iodonaphthalic anhydride, 5-iodonaphthalic anhydride, 6-iodonaphthalic anhydride, 7-iodonaphthalic anhydride, 2-fluoronaphthalic anhydride, 3-fluoronaphthalic anhydride, 4-fluoronaphthalic anhydride, 5-fluoronaphthalic anhydride, 6-fluoronaphthalic anhydride, 7-fluoronaphthalic anhydride, 2-nitronaphthalic anhydride, 3-nitronaphthalic anhydride, 4-nitronaphthalic anhydride, 5-nitronaphthalic anhydride, 6-nitronaphthalic anhydride and 7-nitronaphthalic anhydride.

The term "phthalic anhydride equivalent" is intended to include those compounds where, as described above, R⁷ and R⁸, for example, form an aromatic, heterocyclic, or non-aromatic ring. Suitable compounds include naphthols for example.

### Synthesis of Phenols and Hydrazides

The compounds of the invention may be obtained *via* synthetic methods illustrated below. It should be understood that in R², R³, R⁴, R⁵, R⁶, R⁷, R⁸, R⁹, R¹⁰, R¹¹, R¹², R¹³, R¹⁴ and R¹⁵ are as previously defined for structural formulae (II), (III), (IIIa) and (IV).

Starting materials useful for preparing compounds of the invention and intermediates thereof are commercially available or can be prepared by well-known synthetic methods (*see, e.g.,* Harrison et al., "Compendium of Synthetic Organic Methods", Vols. 1-8 (John Wiley and Sons, 1971-1996); "Beilstein Handbook of Organic Chemistry," Beilstein Institute of Organic Chemistry, Frankfurt, Germany; Feiser et al., "Reagents for Organic Synthesis," Volumes 1-21, Wiley Interscience; Trost et al., "Comprehensive Organic Synthesis," Pergamon Press, 1991; "Theilheimer's Synthetic Methods of Organic Chemistry," Volumes 1-45, Karger, 1991; March, "Advanced Organic Chemistry," Wiley Interscience, 1991; Larock "Comprehensive Organic Transformations," VCH Publishers, 1989; Paquette, "Encyclopedia of Reagents for Organic Synthesis," 3d Edition, John Wiley & Sons, 1995). Other methods for synthesis of the compounds described herein and/or starting materials are either described in the art or will be readily apparent to the skilled artisan.

A typical synthesis for substituted phenols is depicted in Scheme II, wherein a phenol is treated with a base to form the phenolic salt. Advantageously, the phenolic salts are water soluble, which is useful in the applications detailed throughout the specification.

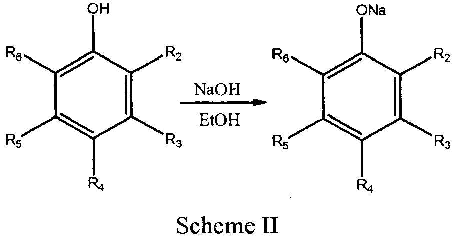

Generally, the phenol mixed with the base and the salt is formed. The solution may be heated to facilitate the rate of reaction.

Suitable phenols include, but are not limited to 2-nitrophenol, 3-nitrophenol, 4-nitrophenol, 2-chlorophenol, 3-chlorophenol, 4-chlorophenol, 2-bromophenol, 3-bromophenol, 4-bromophenol, 2-iodophenol, 3-iodophenol, 4-iodophenol, 2-aminophenol, 3-aminophenol, 4-aminophenol, 2-cyanophenol, 3-cyanophenol, 4-cyanophenol, 2-vinylphenol, 3-vinylphenol, 4-vinylphenol, 2,3-dichlorophenol, 2,4-dichlorophenol, 2,5-dichlorophenol, 2,6-dichlorophenol, 2,3-dibromophenol, 2,4-dibromophenol, 2,5-dibromophenol, 2,6-dibromophenol, 2,3-diiodophenol, 2,4-diiodophenol, 2,5-diiodophenol, 2,6-diiodophenol, 2,3-diaminophenol, 2,4-diaminophenol, 2,5-diaminophenol, 2,6-diaminophenol, 2,3-dicyanophenol, 2,4-dicyanophenol, 2,5-dicyanophenol, 2,6-dicyanophenol, 2,3-divinylphenol, 2,4-divinylphenol, 2,5-divinylphenol, 2,6-divinylphenol, 2,3-diphenylphenol, 2,3,4-trichlorophenol, 2,3,5-trichlorophenol, 2,3,6-trichlorophenol, 2,3,4-tribromophenol, 2,3,5-tribromophenol, 2,3,6-tribromophenol, 2,3,4-triiodophenol, 2,3,5-triiodophenol, 2,3,6-triiodophenol, 2,3,4-trivinylphenol, 2,3,5-trivinylphenol, 2,3,6-trivinylphenol, 2,3,4-trinitrophenol, 2,3,5-trinitrophenol, 2,3,6-trinitrophenol, 2,3,4-triaminophenol, 2,3,5-triaminophenol, 2,3,6-triaminophenol, 2,3,4-tricyanophenol, 2,3,5-tricyanophenol, 2,3,6-tricyanophenol, 3-(N,N-diethylamino)phenol, 3-methyl-2-nitrophenol, 5-methyl-2-nitrophenol, 6-methyl-2-nitrophenol, 3-ethyl-2-nitrophenol, 5-ethyl-2-nitrophenol, 6-ethyl-2-nitrophenol, 3-methoxyl-2-nitrophenol, 5-methoxy-2-nitrophenol, 6-methoxy-2-nitrophenol, 2-nitro-1-naphthol, 3-nitro-1-naphthol, 4-nitro-1-naphthol, 5-nitro-1-naphthol, 6-nitro-1-naphthol, 7-nitro-1-naphthol, 8-nitro-1-naphthol, 2-chloro-1-naphthol, 3-chloro-1-naphthol, 4-chloro-1-naphthol, 5-chloro-1-naphthol, 6-chloro-1-naphthol, 7-chloro-1-naphthol, 8-chloro-1-naphthol, 2-bromo-1-naphthol, 3-bromo-1-naphthol, 4-bromo-1-naphthol, 5-bromo-1-naphthol, 6-bromo-1-naphthol, 7-bromo-1-naphthol, 8-bromo-1-naphthol, 2-iodo-1-naphthol, 3-iodo-1-naphthol, 4-iodo-1-naphthol, 5-iodo-1-naphthol, 6-iodo-1-naphthol, 7-iodo-1-naphthol, 8-iodo-1-naphthol, 2-cyano-1-naphthol, 3-cyano-1-naphthol, 4-cyano-1-naphthol, 5-cyano-1-naphthol, 6-cyano-1-naphthol, 7-cyano-1-naphthol, 8-cyano-1-naphthol and 8-hydroxyquinaldine.

The term "phenol equivalent" is intended to include those compounds where, as described above, R² and R³, for example, form an aromatic, heterocyclic, or non-aromatic ring. Suitable compounds include naphthols for example.

A typical synthesis of hydrazines is depicted in Scheme III, where a hydrazine (NH₂NH-R¹⁵, wherein R¹⁵ can be a hydrogen atom or as described above) and an ester are condensed to form the hydrazide.

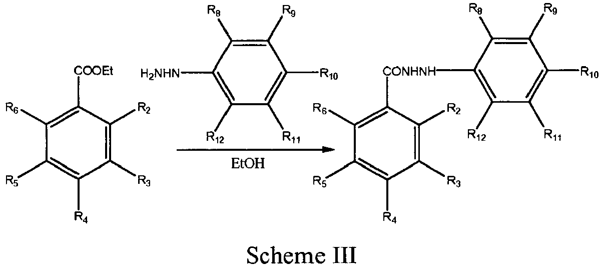

Typically the ester and the hydrazine are combined in a solvent, such as a protic solvent, e.g., an alcohol, such as ethanol, and heated, e.g., to reflux. Upon cooling, the hydrazide generally precipitates from solution and can be collected.

Suitable salicylic derivatives include, but not limited to salicylic acid, 3-methylsalicylic acid, 4-methylsalicylic acid, 5-methylsalicylic acid, 6-methylsalicylic acid, 3-ethylsalicylic acid, 4-ethylsalicylic acid, 5-ethylsalicylic acid, 6-ethylsalicylic acid, 3-propylsalicylic acid, 4-propylsalicylic acid, 5-propylsalicylic acid, 6-propylsalicylic acid, 3-isopropylsalicylic acid, 4-isopropylsalicylic acid, 5-isopropylsalicylic acid, 6-isopropylsalicylic acid, 3-butylsalicylic acid, 4-butylsalicylic acid, 5-butylsalicylic acid, 6-butylsalicylic acid, 3-isobutylsalicylic acid, 4-isobutylsalicylic acid, 5-isobutylsalicylic acid, 6-isobutylsalicylic acid, 3-methoxysalicylic acid, 4-methoxysalicylic acid, 5-methoxysalicylic acid, 6-methoxysalicylic acid, 3-ethoxysalicylic acid, 4-ethoxysalicylic acid, 5-ethoxysalicylic acid, 6-ethoxysalicylic acid, 3-propoxysalicylic acid, 4-propoxysalicylic acid, 5-propoxysalicylic acid, 6-propoxysalicylic acid, 3-butoxysalicylic acid, 4-butoxysalicylic acid, 5-butoxysalicylic acid, 6-butoxysalicylic acid, 3-nitrosalicylic acid, 4-nitrosalicylic acid, 5-nitrosalicylic acid, 6-nitrosalicylic acid, 3-chlorosalicylic acid, 4-chlorosalicylic acid, 5-chlorosalicylic acid, 6-chlorosalicylic acid, 3-bromosalicylic acid, 4-bromosalicylic acid, 5-bromosalicylic acid, 6-bromosalicylic acid, 3-iodosalicylic acid, 4-iodosalicylic acid, 5-iodosalicylic acid, 6-iodoosalicylic acid, 3-fluorosalicylic acid, 4-fluorosalicylic acid, 5-fluorosalicylic acid, 6-fluorosalicylic acid, 3-aminosalicylic acid, 4-aminosalicylic acid, 5-aminosalicylic acid, 6-aminosalicylic acid, 3-acetamidosalicylic acid, 4-acetamidosalicylic acid, 5-acetamidosalicylic acid, 6-acetamidosalicylic acid, 3-cyanosalicylic acid, 4-cyanosalicylic acid, 5-cyanosalicylic acid, 6-cyanosalicylic acid, 3-sulfosalicylic acid, 4-sulfosalicylic acid, 5-sulfosalicylic acid, 6-sulfosalicylic acid, 3,5-dimethylsalicylic acid, 3,5-diethylsalicylic acid, 3,5-dipropylsalicylic acid, 3,5-dibutylsalicylic acid, 3,5-dimethoxysalicylic acid, 3,5-diethoxysalicylic acid, 3,5-dipropoxysalicylic acid, 3,5-dibutoxysalicylic acid, 3,5-dichlorosalicylic acid, 3,5-dibromosalicylic acid, 3,5-diiodosalicylic acid, 3,5-difluorosalicylic acid, 3,5-dinitrosalicylic acid, 3,5-diaminosalicylic acid, 3,5-diacetamidosaticylic acid, 3,5-dicyanosalicylic acid, 3,5-disulfosalicylic acid, substituted/unsubstituted alkyl salicylic acid, substituted/unsubstituted alkoxy salicylic acid, substituted/unsubstituted aryl salicylic acid, substituted/unsubstituted cycloalkyl salicylic acid and substituted/unsubstituted hetaryl salicylic acid.

Suitable hydrazines include but not limited to hydrazine hydrate, 4-nitrophenylhydrazine, 3-nitrophenylhydrazine, 2-nitrophenylhydrazine, 4-nitrobenzoic hydrazide, 3-nitrobenzoic hydrazide, 2-nitrobenzoic hydrazide, p-toluenesulfonylhydrazide, m-toluenesulfonylhydrazide, o-toluenesulfonyl-hydrazide, 2,4-dinitrophenylhydrazine (2,4-DNP), 1-naphthoic hydrazide, 2-naphthoic hydrazide, nicotinic hydrazide, substituted/unsubstituted alkyl hydrazide, substituted/unsubstituted alkoxy hydrazide, substituted/unsubstituted aryl hydrazide, substituted/unsubstituted cycloalkyl hydrazide and substituted/unsubstituted hetaryl hydrazide.

Representative examples of typical film forming agents/thickeners (binders) which may be employed in the bubble compositions of the invention include, but are not limited to, polyvinylalcohol, polyvinylpyrrolidone, polyoxyethylene, polyvinyl acetate, gelatin, gum arabic, rosin, rosin-modified maleic acid resins, rosin-modified phenol resins, rosin esters, hydrogenated rosins, various cellulosic resins, methyl cellulose, ethyl cellulose, acetyl cellulose, propyl cellulose, butyl cellulose, hydroxyethyl cellulose, hydroxypropyl cellulose, higher alkyl cellulose, sodium carboxymethyl cellulose, cellulose ethers, cellulose esters, cellulose acetobutyrate, petroleum resins, various phenol resins, starches and derivatives thereof, acrylics such as polyacrylates and/or polymethacrylates, polyurethanes, styrenic polymers, acrylate/sytrenic copolymers, metharcylate/sytrenic copolymers, polyvinyl chloride, vinylchloride/styrenic copolymers, alkyds, polyesters, epoxy polymers, polyamides, polyimide, alkylated ureas, benzoguanamines, melamine-formaldehydes, phenolic resins and the like. Mixtures of two or more of these film forming agents can also be employed in the present invention.

Suitable polymerized vinyl containing mateials include, for example, polyolefins, poly(vinyl halides), vinyl halide copolymers, poly(vinyl) esters, poly(vinyl) alcohol, poly(vinyl) acetals, poly(vinyl) acetate, poly(vinyl) chloride, poly(vinyl) formal, polyvinyl) butyrals, poly(vinyl) ethers and polystyrenes.

Suitable alkyd resins include, for example, styrene modified alkyd resins, vinyl modified alkyd resins, silicon modified alkyd resins, thixotropic alkyd resins, urethane alkyd resins, alkyd resins with highly branched carboxylic acids and waterborne alkyd resins.

In particular, film forming agents include, but are not limited to PVP K-15, PVP K-30, PVP K-90, polyvinylpyrrolidone, manufactured by ISP (International Specialty Products), Wayne, NJ, Elvanol 40-05, Elvanol 40-16, Elvanol 40-40, Elvanol 70-14, Elvanol 70-27, Elvanol 60-30, Elvanol 70-03, Elvanol 70-04, Elvanol 70-06, Elvanol 70-20, Elvanol 70-30, Elvanol 70-62, Elvanol 70-63, Elvanol 70-75, Elvanol 71-30, Elvanol 90-50, Elvanol 50-14, Elvanol 50-26, Elvanol 50-42, Elvanol 51-03, Elvanol 51-04, Elvanol 51-05, Elvanol 51-08, Elvanol 52-22, Elvanol 75-15, Elvanol 85-82, Elvanol 85-91, polyvinylalcohol, manufactured by DuPont, Wilmington, DE, Celvol 125, Celvol 165, Celvol 350, Celvol 325, Celvol 310, Celvol 305, Celvol 103, Celvol 107, Celvol 203, Celvol 205, Celvol 418, Celvol 425, Celvol 443, Celvol 502, Celvol 504, Celvol 508, Celvol 513, Celvol 518, Celvol 523, Celvol 530, Celvol 540, Celvol 805, Celvol 823, Celvol 840, polyvinylalcohol, manufactured by Celanese Chemicals, Dallas TX, Polyox WSR N-10, Polyox WSR N-80, Polyox WSR N-750, Polyox WSR N-3000, Polyox WSR 205, Polyox WSR -1105, Polyox WSR N-12K, Polyox WSR N-60K, Polyox WSR-301, Polyox WSR-303, Polyox WSR-308, polyethylene oxide, manufactured by Dow Chemical Co., Midland, MI, K4484, water soluble starch, manufactured by National Starch & Chemicals, Bridgewater, NJ, Dri-sweet 42, water soluble starch, manufactured by American Maize and ICB 3000, water soluble starch, manufactured by Staley.

The film forming agent/thickener may be present in the bubble composition of the invention in a range from about 2% up to about 99% by weight, in particular from about 5% to about 90% by weight and more particularly from about 10% to about 80% by weight.

In one aspect, film forming agents/thickeners useful in the compositions of the invention include, but are not limited to, cellulose ethers or mixtures with other surface-active agents, which are water soluble. Cellulose ether surface-active agents have unique thickening and film forming properties. Cellulose ethers used in the present invention include methyl cellulose, ethyl cellulose, propyl cellulose, butyl cellulose, higher alkyl, aryl, alkoxy, cycloalkyl celluloses, hydroxypropyl cellulose, hydroxybutyl cellulose or mixtures thereof.

Commercial cellulose ether film forming agents/thickeners include, but are not limited to, Methocel A4M, methyl cellulose, Methocel F4M, hydroxypropyl methylcellulose, Methocel K4M, hydroxypropyl methylcellulose, manufactured by Dow Chemical Co., Mildland, MI; Natrosol, hydroxyethyl cellulose, Klucel, hydroxypropyl cellulose, Aqualon Cellulose Gum, sodium carboxymethyl cellulose, Hercules Inc., Wilmington, DE; Elfacos CD 481, ethyl 2-hydroxyethyl ether cellulose, manufactured by Akzo Nobel, Chicago, IL.

Cellulose ether film forming agents/thickeners are generally present in amounts from about 2% up to about 99% by weight in the compositions of the invention. Suitable concentrations of cellulose ether surface-active agentss are in the range of about 5% to about 90% by weight and from about 10% to about 80% by weight. A particularly useful cellulosic ether film forming agent in the painting compositions is Methocel A4M.

The compositions of the invention include surface active agent(s) (surfactants). Suitable surface-active agents include anionic, cationic, nonionic or zwitterionic compounds and combinations thereof. The surface-active agent can be either polymeric or non-polymeric.

The term "surface active agent" is recognized in the relevant art to include those compounds which modify the nature of surfaces, e.g. reducing the surface tension of water. Surface active agents are generally classified into four types: cationic (e.g. modified onium salts, where part of the molecule is hydrophilic and the other consists of straight or branches long hydrocarbon chains such as hexadecyltrimethyl bromide), anionic, also known as amphiphatic agents (e.g., alkyl or aryl or alkylarylsulfonates, carboxylates, phosphates), nonionic (e.g., polyethylene oxides, alcohols) and ampholytic or amphoteric (e.g. dodecyl-beta-alanine, such that the surface-active agent contains a zwitterionic group). One or more surface-active agents can be used in the present invention.

Cationic surface-active agents useful as surface tension reducing agents in the present invention include long chain hydrocarbons which contain quaternarized heteroatoms, such as nitrogen. Suitable cationic surface-active agents include quaternary ammonium compounds in which typically one of the groups linked to the nitrogen atom is a C12-C18 alkyl group and the other three groups are short chained alkyl groups.

Anionic surface-active agents (amphiphatic agents) are characterized by a single lipophilic chain and a polar head group which can include sulfate, sulfonate, phosphate, phosphonate and carboxylate. Exemplary compounds include linear sodium alkyl benzene sulfonate (LAS), linear alkyl sulfates and phosphates, such as sodium lauryl sulfate (SLS) and linear alkyl ethoxy sulfates. Additional examples of anionic surface-active agents include substituted ammonium (e.g., mono-, di-, and tri-ethanolammonium), alkali metal and alkaline earth metal salts of C6-C20 fatty acids and rosin acids, linear and branched alkyl benzene sulfonates, alkyl ether sulfates, alkane sulfonates, olefin sulfonates, hydroxyalkane sulfonates, fatty acid monoglyceride sulfates, alkyl glyceryl ether sulfates, acyl sarcosinates. acyl N-methyltaurides, and alkylaryl sulfonated surface-active agents, such as alkylbenezene sulfonates.

Nonionic surface-active agents do not dissociate but commonly derive their hydrophilic portion from polyhydroxy or polyalkyloxy structures. Suitable examples of polyhydroxy (polyhydric) compounds include ethylene glycol, butylene glycol,1,3-butylene glycol, propylene glycol, glycerine, 2-methyl-1,3-propane diol, glycerol, mannitol, corn syrup, beta-cyclodextrin, and amylodextrin. Suitable examples of polyalkyloxy compounds include diethylene glycol, dipropylene glycol, polyethylene glycols, polypropylene glycols and glycol derivatives.

Other suitable nonionic surface-active agentss include other linear ethoxylated alcohols with an average length of 6 to 16 carbon atoms and averaging about 2 to 20 moles of ethylene oxide per mole of alcohol; linear and branched, primary and secondary ethoxylated, propoxylated alcohols with an average length of about 6 to 16 carbon atoms and averaging 0-10 moles of ethylene oxide and about 1 to 10 moles of propylene oxide per mole of alcohol; linear and branched alkylphenoxy (polyethoxy) alcohols, otherwise known as ethoxylated alkylphenols, with an average chain length of 8 to 16 carbon atoms and averaging 1.5 to 30 moles of ethylene oxide per mole of alcohol; and mixtures thereof.

Additionally, suitable nonionic surface-active agents include polyoxyethylene carboxylic acid esters, fatty acid glycerol esters, fatty acid and ethoxylated fatty acid alkanolamides. Block copolymers of propylene oxide and ethylene oxide, and block polymers of propylene oxide and ethylene oxide with propoxylated ethylene diamine are also included as acceptable nonionic surface-active agents. Semi-polar nonionic surface-active agents like amine oxides, phosphine oxides, sulfoxides, and their ethoxylated derivatives are included within the scope of the invention.

Suitable amphoteric and zwitterionic surface-active agents which contain an anionic water-solubilizing group, a cationic group and a hydrophobic organic group include amino carboxylic acids and their salts, amino dicarboxylic acids and their salts, alkylbetaines, alkyl aminopropylbetaines, sulfobetaines, alkyl imidazolinium derivatives, certain quaternary ammonium compounds, certain quaternary phosphonium compounds and certain tertiary sulfonium compounds.

Examples of anionic, nonionic, cationic and amphoteric surface-active agents that are suitable for use in the present invention are described in Kirk-Othmer, Encyclopedia of Chemical Technology, Third Edition, Volume 22, pages 347-387, and McCutcheon's Detergents and Emulsifiers, North American Edition, 1983, both of which are incorporated herein by reference.

Typical concentration ranges of surface-active agent that are useful in the present compositions are from about 0.1 parts by weight to about 15 parts by weight, from about 0.5 part by weight to about 10 parts by weight, and from about 1 parts by weight to about 5 parts by weight. Surface-active agents do not act as film forming agents.

In another aspect, alkanolamide or a mixture with other surface-active agents can be used in the compositions of the invention. Alkanolamides are commercially available and are the reaction products of one or more fatty acids having 12 or more carbon atoms and a lower alkanolamime. Typical alkanolamides are formed by reaction between stearic, mystiric, lauric acid or mixtures thereof with mono-, di-, and/or iso-propanolamine.

Alkanolamides can be present in the compositions of the invention in the ranges generally described throughout the application but generally are present in amounts from about 0% up to about 10% by weight. Suitable ranges include from about 1% to about 6% by weight and in particular from about 1.5% to about 4% by weight.

In one embodiment, the alkanolamide surface-active agents of the present invention include, but are not limited to, Ninol 55LL, diethanolamine, Ninol 40CO, cocamide DEA, Ninol 30LL, lauramide DEA, manufactured by Stepan Co., Northfield, IL; Colamid C, cocamide DEA, Colamid 0071-J, alkanolamide, manufactured by Colonial Chemical Inc., S. Pittsburgh, TN. In one aspect, the alkanolamides are Ninol 55LL, and Colamid C.

Exemplary sulfosuccinates that can be employed in the present painting compositions include, but are not limited to, Stepan-Mild SL3-BA, disodium laureth sulfosuccinate, Stepan-Mild LSB, sodium lauryl sulfosuccinate, manufactured by Stepan Co., Northfield, IL, Lankropol 4161L, sodium fatty alkanolamide sulfosuccinate and Colamate-DSLS, disodium laureth sulfosuccinate, manufactured by Colonial Chemical Inc., S. Pittsburgh, TN.

Suitable betaines that can be employed in the present painting compositions include, but are not limited to, Miracare BC-27, cocamidopropyl betaine and Miranol Ultra C-37, sodium cocoampho acetate, manufactured by J & S Chemical Co., Weston, FL.

Suitable sulfates that can be employed in the present painting compositions include Rhodapex ES-2, sodium laureth sulfate, J & S Chemical Co., Weston, FL; Witcolate WAQ, sodium alkyl sulfate, manufactured by Akzo Nobel, Chicago, I and Colonial-SLS, sodium lauryl sulfate, manufactured by Colonial Chemical Inc., S. Pittsburgh, TN. Colonial-SLS surfactant is a combination of lauryl sulfate, C10-C16 alkyl alcohols, sodium salts and C10-C16 alcohols.

A suitable nonionic surface-active agent that can be employed in the present painting compositions is Triton H-66, alkyl aryl alkoxy potassium salt, manufactured by Dow Chemical Co., Mildland, MI.

In another embodiment, the surface-active agent used is a combination of an alkanolamide and a mixture of an alkyl betaine and/or an alkyl sulfonate.

In a particular embodiment, the surface-active agent is a combination of Colamid C and Miracare BC27 which is a mixture of Surface-active agent blend include sodium trideceyl sulfate, water, PEG 80 sorbitant laurate, cocamidopropyl betaine, sodium lauroamphoacetate, PEG 150 distearate, sodium laureth-13 carboxylate, glycerin, citric acid, tetrasodium EDTA, quaternium-15. Generally, the combination of the alkanolamide and alkylsulfonate/betaine is in the range of between about 1:1 to about 1:7, more particularly between about 1:1 to about 2:7 and more particularly about 2:7. Generally, the combination of the two surface-active agents comprises a concentration between about 3 and about 10 percent by weight of the total weight of the composition, and more particularly between about 5 and about 10 percent by weight of the total weight of the composition, and in particular about 9 percent of the total weight of the composition.

The aqueous compositions throughout the specification can further include a solvent or other additives as described throughout the present application. Suitable solvents include, for example, alcohols having a carbon chain length of from about 1 carbon atom to about 12 carbon atoms. Typically, methanol and ethanol are not included due to their generally recognized properties, especially in view of use with children.

Suitable optional additives to the compositions mentioned throughout the invention include, preservatives, fragrance, etc.

Representative examples of preservatives useful with the compositions throughout the specification include, but are not limited to, glutaraldehyde, bicyclic oxazolidones, hydroxybenzoic acid esters, 3-iodo-2-propynyl butyl carbamate, methyl p-hydroxybenzoate, and a biocide comprising 2-methyl-4-isothiazolin-3-one and 5-chloro-2-methyl-4-isothiazolin-3-one. The preservatives often serves as both a bactericide and a fungicide.

In particular, compositions of the invention include preservatives that are selected from, but not limited to, Liquid Germall Plus, iodopropynyl butyl carbamate, Germall II, diazolidinyl urea, Nuosept 95, bicyclic oxazolidines solution, manufactured by ISP (International Specialty Products), Wayne, NJ, Troysan 395, dihydroxy-dimethyl hydantoin, manufactured by Troy Chemical Corporation, Florham park, NJ and Kathon PFM, isothiazolinones, manufactured by Rohm & Haas Co., Philadelphia, PA

Preservatives, when present in the compositions of the invention, are generally present in amounts from about 0.01% to about 10% by weight, in particular from about 0.05% to about 5% by weight, and particularly from about 0.1% to about 2.5% by weight. In one aspect, the preservative is one of Liquid Germall Plus, Tryosan 395 or Nuosept 95.

Representative fragrances include those pleasing to children such as flowers, candy, popcorn, fruit, bubble gum and the like. A fragrance, when present in the compositions of the invention, is generally present in amounts from about 0.1 % to about 10% by weight of the total weight of the composition.

The present invention further includes kits that include the compositions of the invention.

It should be understood, that additional colorants described infra, are within the scope of the compositions. These colorants useful in paint applications, include, for example, leuco dyes, metal salts, etc. as described infra.

### Bubbles

Prior to the present invention, it was generally considered extremely difficult if not impossible to make a colored bubble with uniform color intensity throughout the bubble: A bubble's wall is only a few millionths of an inch thick and up until the present invention was considered that the bubble wall was incapable of being colored. Generally, a bubble's rainbow color is the result of reflects color from its surroundings.

Traditionally, when a light wave hits the surface of a bubble, part of the light is reflected back to a viewer's eye from the outer surface and part of the light is reflected from the inner surface which is a few millionths of an inch further. As the two waves of light travel back, they interfere with one another causing what we visualize as color. When the waves reinforce each other, the color is more intense. When the waves get close to canceling each other out, there is almost no color. As a bubble wall gets thinner, either from a weakened solution or because gravity has pulled the additives to the bottom of the bubble, the distance between the inner surface and the outer surface of the bubble becomes less and less until the two reflected waves of light start to coincide and cancel each other out. The result is that the bubble loses its color and can become nearly invisible.

Prior to the present invention, it has proven extremely difficult, if not impossible, to develop a colored bubble composition with uniform color intensity throughout the bubble. When the dyes are added to the soap/bubble solution, they form colored solution but when the bubbles are blown, the resulting bubbles are colorless.

The present invention surprisingly provides coloration changing, color disappearing bubble compositions, that have substantially uniform coloration about the bubble. Additionally, the present invention provides compositions that have film forming capabilities such that the resultant bubble can withstand physical contact with a surface. The film formed bubble can be colored, have a color changing composition, have a color disappearing composition, or can be without color. The bubbles can have a wide range of opacity, colors and scents. The compositions and resultant bubbles are non-toxic and/or washable.

The present invention further provides compositions and methods for producing bubbles, as described herein, having a wide range of opacities, ranging from essentially translucent to semi-transparent to opaque. The bubbles can be intrinsically colored; the composition from which the bubbles are formed itself is colored.

In some embodiments, the bubbles have substantially uniform color intensity. In other embodiments, the bubbles can have non-uniform color intensity and/or dispersion.

The phrases "substantially uniform" or "substantially uniformly" are intended to refer to coloration about the bubble such that the coloration intensity is approximately equal from the top of the bubble to the bottom of the bubble. In such an embodiment, the coloration in the bubble is dispersed evenly throughout the bubble and coloration streaking or having an increased concentration of color at the bottom of the bubble is substantially avoided.

The phrases "non-uniform" or "non-uniformly" are intended to refer to coloration about the bubble such that the coloration intensity is concentrated, for example, more at the top and bottom of the bubble. Such fanciful bubbles can be very interesting to children.

The phrase "colored bubble" is intended to refer to a bubble that can be uniformly or non-uniformly colored, as described herein, but does not have a change in coloration over a given period of time and does not have the coloration disappear from the bubble. Colored bubbles retain their coloration throughout the period of time the bubble exists, generally from about a few seconds to about a few minutes. The colored bubbles related to the present invention are formed with film forming technology described herein.

In one aspect, the colored bubbles and compositions of the invention are excluded from those described in US Provisional Application 60/581,294, filed on June 17, 2004, by Tim Kehoe, entitled "Composition and Method for Producing Colored Bubbles", the contents of which are incorporated herein by reference in their entirety.

Color Changing or Color Disappearing Compositions and Bubbles

In one embodiment, the present invention pertains to an aqueous composition that includes a surfactant, an acid-base indicator and a base, such as a volatile base, such as an amine. A preferred base is a metal hydroxide. The composition can be used to prepare bubbles that have color disappearing characteristics and/or color changing characteristics.

The phrase "color changing" refers to the ability of the dye or pigment to change coloration due to a change in physical propert(ies) of the dye or pigment. The change in color can be a result of oxidation, change in pH, or some other physical attribute of the dye or pigment that is altered upon, for example, the evaporation of water, solvent or some other component from the bubble surface. Alternatively, the change can occur when the concentration of an ingredient increases within the bubble surface due to loss of another component, for example, due to evaporation. Generally the color change occurs over a period between about 1 second and about 10 minutes, more particularly between about 2 seconds to about 5 minutes and more particularly between about 5 seconds and about 1 minute.

The phrase "color disappearing" refers to the ability of the dye or pigment to lose or have a diminishment in coloration due to a change in physical propert(ies). The disappearance of color can be a result of oxidation, change in pH, or some other physical attribute of the dye or pigment that is altered upon, for example, upon the evaporation of water, solvent or other component from the bubble surface. Alternatively, the coloration loss or diminishment can occur when the concentration of an ingredient increases within the bubble surface due to loss of another component, for example, due to evaporation. Generally the coloration loss or diminishment occurs over a period between about 1 second and about 10 minutes, more particularly between about 2 seconds to about 5 minutes and more particularly between about 5 seconds and about 1 minute.

The aqueous solutions of the present invention generally contain between about 1 and about 90 parts water, in particular between about 10 and about 80, and more particularly between about 20 and about 70 percent based on a total weight percentage of the final composition. In one aspect, the water utilized can be ordinary tap water or spring water. In another aspect the water can be deionized water or water purified by reverse osmosis.

The compositions of the invention include a surfactant. Suitable surfactants include anionic, cationic, nonionic or zwitterionic compounds and combinations thereof described throughout the specification. The surfactant can be either polymeric or non-polymeric.

In one particular embodiment, the surfactant utilized to form the bubble is Colonial SLS.

Typical concentration ranges of surfactant that are useful in the preparation of bubble composition are from about 0.01 parts by weight to about 90 parts by weight, from about 0.5 part by weight to about 50 parts by weight, and from about 1 parts by weight to about 20 parts by weight. A preferred surfactant is a combination of lauryl sulphate, C10-C16 alkyl alcohols, sodium salts and C10-C16 alcohols.

In one aspect, surfactants useful in the bubble compositions of the invention include, but are not limited to, cellulose ethers or mixtures with other surfactants, which are water soluble. Cellulose ether surfactants have unique foaming and film forming properties which make them ideal of colored bubble applications. Cellulose ethers used in the present invention include methyl cellulose, ethyl cellulose, propyl cellulose, butyl cellulose, higher alkyl, aryl, alkoxy, cycloalkyl celluloses, hydroxypropyl cellulose, hydroxybutyl cellulose or mixtures thereof.

Cellulose ether surfactants are generally present in bubble composition in amounts from about 1% up to about 40% by weight in the compositions of the invention. Suitable concentrations of cellulose ether surfactants are in the range of about 2% to about 30% by weight and from about 3% to about 8% by weight. A particularly useful cellulosic ether surfactant in the compositions is Methocel A4M.

Alkanolamides can be present in the bubble compositions of the invention in the ranges generally described throughout the application but generally are present in amounts from about 0% up to about 10% by weight. Suitable ranges include from about 1% to about 6% by weight and in particular from about 1.5% to about 4% by weight.

Suitable optional additives to the compositions of the invention include, humectants, preservatives, fragrance, dye blockers, cleaners, etc.

The term "humectant" is known and helps to retard the evaporation of water from the composition of the invention, thus avoiding premature drying during the application. Not to be limited by theory, it is believed that the presence of a humectant helps to strengthen the bubble formation, enhances even distribution of the dye throughout the bubble and increases life of bubble in the air.

Representative examples of humectants include, but are not limited to, glycerin, ethylene glycol, propylene glycol, diethylene glycol, polyethylene glycol, hydroxylated starches and mixtures of these materials. Any effective amount of humectant may be used although a generally useful concentration range for these humectants is from about 5% to about 35% by weight of the total composition. Particular ranges of the humectant include a range of from about 8% to about 30% by weight of the composition and from about 10% to about 25% by weight of the composition. In one particular aspect, the humectant is glycerin.

Preservatives, when present in the bubble compositions of the invention, are generally present in amounts from about 0.01% to about 6% by weight, in particular from about 0.05% to about 5% by weight, and particularly from about 0.1 % to about 2.5% by weight. In one aspect, the preservative is one of Liquid Germall Plus, Tryosan 395 or Nuosept 95.

Representative fragrances include those pleasing to children such as flowers, candy, popcorn, fruit, bubble gum and the like. A fragrance, when present in the bubble compositions of the invention, is generally present in amounts from about 0.1 % to about 10% by weight of the total weight of the composition.

Dye blockers or cleaners can be optionally added in the compositions of the invention, including bubbles, to remove dye from hard/porous surfaces such as wood, stone, brick, leather, cloth, concrete, skin, fabric, etc. Up until the present invention, contact with a solution having a dye could stain a surface.

Suitable dye blockers include, but are not limited to, Bio-Terge PAS-8S, sodium octane sulfonate, Stepanate SXS, sodium xylenesulfonate, Steposol DG, fatty alcohol ethoxylate, manufactured by Stepan Co., Northfield, IL, Dowfax 8390, disodium hexadecyldiphenyloxide disulfonate, Dowfax 2A1, benzene-1,1-oxybis-tetrapropylene sulfonated sodium, Dowfax 3B2, decyl-sulfophenoxy-benzenesulfonic acid-disodium, Dowfax C10L, decyl-sulfophenoxybenzenesulfonicacid disodium, Triton X-15, octylphenoxypolyethoxyethanol, manufactured by Dow Chemical Co., Mildland, MI, Tamol SN, sodium salt of naphthalene-formaldehyde condensate, Tamol 731, sodium salt of carboxylated polyelectrolyte, manufactured by Rohm & Haas Co., Philadelphia, PA, Darvan 2, sodium lignin sulfonate, manufactured by R. T. Vanderbilt & Co., Norwalk, CT, Aqua-Cleen GP, polyethoxylated tert-dodecyl sulfur compound, TZ-Paint Prep, phosphorous/sulfur containing builders, and TAZ-B300, sulfur/oxygen/nitrogen containing surface active agents, manufactured by Chemical Products Industries, Oklahoma City, OK

Dye blockers or cleaners are usually effective in the compositions of the invention when present in any amount but generally are present in ranges from about 5% up to about 50% by weight, from 10% to about 40% by weight or from about 12% to about 25% by weight.

The bubble compositions of the invention include acid-base indicators as described throughout the application. These acid-base indicators used in the present invention are generally colored under basic condition and change color or fade to clear in non-basic condition. Acid-base indicators which are colored on alkaline pH side (pH >7) and turn clear on acidic pH (pH <7) are most useful. Typically, the acid-base indicators are colored at pH between about 9 and 10, and turn clear at pH between about 6 and 8.

Representative examples of acid-base indicators useful in the bubble compositions of the present invention include those in which R² is selected from the group consisting of hydrogen and methyl; R³ is selected from the group consisting of hydrogen phenyl, isopropyl, methyl ethyl and methoxy; R⁵ is selected from the group consisting of hydrogen, bromo, methoxy, isopropyl and methyl; and R⁶ is selected from the group consisting of hydrogen and methyl.

In certain bubble embodiments, R² is selected from the group consisting of hydrogen, nitro, amino and alkyl; R³ is selected from the group consisting of hydrogen, phenyl, alkyl, nitro, acetamido and alkoxy; R⁵ is selected from the group consisting of hydrogen, halo, and alkyl; and R⁶ is selected from the group consisting of hydrogen and alkyl.

In certain bubble embodiments, R² is selected from the group consisting of hydrogen and methyl; R³ is selected from the group consisting of hydrogen, phenyl, isopropyl, methyl, ethyl, sec-butyl, nitro and methoxy; R⁵ is selected from the group consisting of hydrogen, bromo, methoxy, isopropyl and methyl; and R⁶ is selected from the group consisting of hydrogen and methyl.

In other bubble embodiments, R², R³, R⁵, R⁶, R⁷, R⁸, R⁹ and R¹⁰ are all hydrogen atoms; R² is hydrogen, R³ is Me, and R⁵, R⁶, R⁷, R⁸, R⁹ and R¹⁰ are all hydrogen atoms; R² is Me, R³ is a hydrogen atom, R⁵ is an iso-propyl group and R⁶, R⁷, R⁸, R⁹ and R¹⁰ are all hydrogen atoms; R² is H, R³ is Me, R⁵ is Br and R⁶, R⁷, R⁸, R⁹ and R¹⁰ are all hydrogen atoms; and R² is Me, R³ is Br, R⁵ is an isopropyl and R⁶, R⁷, R⁸, R⁹ and R¹⁰ are all hydrogen atoms. In certain embodiments, one or more of these compounds may be excluded from certain aspects of the invention.

In still other bubble embodiments, R² is H, R³ is phenyl and R⁵, R⁶, R⁷, R⁸, R⁹ and R¹⁰ are all hydrogen atoms; R² is H, R³ and R⁵ are isopropyl and R⁶, R⁷, R⁸, R⁹ and R¹⁰ are all hydrogen atoms; R² is H, R³ is methyl, R⁵ is H, R⁶ is methyl, R⁷, R⁸, R⁹ and R¹⁰ are all hydrogen atoms; R² is H, R³ and R⁵ are methoxy and R⁶, R⁷, R⁸, R⁹ and R¹⁰ are all hydrogen atoms; R² is H, R³ and R⁵ are methyl and R⁶, R⁷, R⁸, R⁹ and R¹⁰ are all hydrogen atoms; R² is H, R³ is ethyl and R⁵, R⁶, R⁷, R⁸, R⁹ and R¹⁰ are all hydrogen atoms; R² is H, R³ is isopropyl and R⁵, R⁶, R⁷, R⁸, R⁹ and R¹⁰ are all hydrogen atoms; R² is H, R³ is methoxide and R⁵, R⁶, R⁷, R⁸, R⁹ and R¹⁰ are all hydrogen atoms; and R², R³ and R⁵ are all methyl and R⁶, R⁷, R⁸, R⁹ and R¹⁰ are all hydrogen atoms, or R², R⁵, R⁶, R⁷, R⁸, R⁹, R¹⁰ are all hydrogen atoms and R³ is sec-butyl, or R², R⁵, R⁶, R⁷, R⁸, R⁹, R¹⁰ are all hydrogen atoms and R³ is nitro.

At least one of M¹ or M² is a metal or an ammonium ion.

Additionally, the phenols and hydrazines described throughout are useful in the bubble compositions as well. Additional colorants useful in the various compositions of the application (e.g., paints, bubbles, markers and cosmetics) include those listed throughout the application, including those which follow.

With the suitable selection of acid-base indicators, it is possible to produce any color. The acid-base indicators are preferably in the form of a salt, such as a sodium salt generated by reacting the indicator, such as a phthalein, with sodium hydroxide, so as to permit its solubilization into the present composition. Generally, 2 equivalents of base are added per mole of phthalein, however, less tha 2 equivalents will produce a color, and it is possible that 1 equivalent will impart color to the phthalein salt. Additionally, combinations of two or more indicators may be used.

Acid-base indicators are usually effective when present in small amounts in the bubble compositions of the invention but generally are present in amounts from about 0.1% up to about 20% by weight, from about 0.5% to about 10% by weight and from about 0.8% to about 8% by weight of the total weight of the composition.

Selection of an appropriate basic material is important for color change of acidic dye indicators in the colored compositions of the present invention. Desirable basic reagents, which should readily volatilize at ambient temperatures for use in the present compositions, include, but are not limited to, aminoalcohols, such as alkylamines, such as methylamine, dimethylamine, ethylamine, diethylamine, triethylamine, ethyleneamine, diethyleneamine, morpholine, ammonia, triethanolamine.

The selection of the kind and the amount of basic reagent used enables control of fading time of the color after a colored bubble is blown. Suitable basic reagents which readily volatilize at ambient temperatures, typically have a vapor pressure higher than about 10 mm Hg at 20°C. The selection of the base also depends on solubility in water, toxicity and odor. Therefore, aminoalcohols useful in the compositions of the present invention include, but are not limited to triethanolamine (TEA) and/or diethylamine. TEA, for example, is clear, non-toxic and does not emit a noxious odor.

The basic reagent(s) is generally present in the bubble composition of the invention in an amount from about 0.1% up to about 20% by weight, from about 0.2% to about 10% by weight and from about 0.5% to about 5% by weight.

The aqueous bubble compositions may include a surfactant, leuco dye and an electron acceptor or oxidizing agent. The composition can be used to prepare bubbles that have color disappearing characteristics and/or color changing characteristics. Optionally, one or more of the above-identified solvents, humectants, preservatives, fragrances, etc. as described throughout the application can be included in the composition.

The bubble compositions can include one or more of the surfactant(s) described throughout the present application in combination with at least one leuco dye and an electron acceptor or oxidizing agent.

The term "leuco dye" is intended to mean a colorless or lightly colored dye which can be oxidized to a colored form. Leuco dyes are generally electron donating chromatic organic compounds which are generally colorless and produce color upon reaction with acidic materials.

Suitable examples of leuco dyes that can be incorporated into compositions of the invention include, but are not limited to, diaryl phthalide, polyarylcarbinols, leuco-auramines, acylauramines, arylauramines, rhodamine B lactams, indolines, spiropyrans, fluorans, phenothiazines, triarylmethanes, 3,3-bis(p-dimethylaminophenyl)-phthalide, 3,3-bis(p-dimethylaminophenyl)-6-dimethylaminophthalide (crystal violet lactone), 3,3-bis(p-dimethylaminophenyl)-6-diethylaminophthalide, 3,3-bis(p-dimethylaminophenyl)-6-chlorophthalide, 3,3-bis(p-dibutylaminophenyl)-phthalide, 3-(2'-hydroxy-4'-dimethylaminophenyl)-3-(2'-methoxy-5'-chlorophenyl)-phthalide, 3-(2'-hydroxy-4'-dimethylaminophenyl)-3-(2'-methoxy-5'-nitrophenyl)-phthalide, 3-(2'-hydroxy-4'-diethylaminophenyl)-3-(2'-methoxy-5'-methylphenyl)-phthalide, 3-(2'-methoxy-4'-dimethylaminophenyl)-3-(2'-hydroxy-4'-chloro-5'-methylphenyl)-phthalide, malachite green lactone, mishler's hydrol, crystal violet carbinol, malachite green carbinol, N-(2,3-dichlorophenyl)-leuco auramine, N-benzoyl auramine, N-acetyl auramine, N-phenyl auramine, rhodamine B lactam, 2-(phenyliminoethylidene)-3,3-dimethylindoline, N-3,3-trimethyl-indolinobenzospiropyran, 8'-methoxy-N-3,3-trimethyl-indolino-benzospiropyran, 6'-chloro-8'-methoxy-benzoindolino-spiropyran, 6'-bromo-3'-methoxy-benzoindolino-spiropyran, 2[3,6-bis(diethylamino)-9-(o-chloroanilino)xanthylbenzoic acid lactam, benzoyl leuco methylene blue, 3-amino-5-phenyl-8-methylfluoran, 2-bromo-6-cyclohexylaminofluoran, 2-chloro-3-(N-methyltoluidino)-7-(p-n-butylanilino)fluoran, 3-cyclohexylamino-6-chlorofluoran, 3-dimethylamino-6-benzyloxyfluoran, 3-dimethylamino-5,7-dimethylfluoran, 3-diethylamino-7-methylfluoran, 3-diethylamino-7-methoxyfluoran, 3-diethylamino-7-chlorofluoran, 3-diethylamino-7-anilinofluoran, 3-diethylamino-7-acetamido-fluoran, 3-diethylamino-7-p-toluidino-fluoran, 3-diethylamino-7-piperidinofluoran, 3-diethylamino-7-(o-chloroanilino)-fluoran, 3-diethylamino-7-(o-methoxycarbonylphenylamino)-fluoran, 3-diethylamino-7,8-benzofluoran, 6-diethylamino-1,2-benzofluoran, 3-diethylamino-6-methyl-7-chlorofluoran, 3-diethylamino-6-methyl-7-anilinofluoran, 3-diethylamino-6-methyl-7-mesidino-4',5'-benzofluoran, 3-diethylamino-6-methyl-7-(2',4'-dimethylanilino)fluoran, 3-diethylamino-6-methyl-7-(m-trichloroanilino)fluoran, 3-diethylamino-5-chloro-7-(N-benzyltrifluoromethyl-anilino)-fluoran, 3-diethylamino-5-chloro-7-(α-phenylethylamino)fluoran, 3-diethylamino-5-methyl-7-(α-phenylethylamino)fluoran, 3-dibutylamino-7-(o-chloroanilino)-fluoran, 2,3-dibutylene-6-di-n-butylaminofluoran, 2,7-dichloro-3-methyl-6-n-butylaminofluoran, 3,6-di-p-toluidino-4,5-dimethylfluoran-phenylhydrazide-γ-lactam, 3-N-ethyl-p-toluidino-7-(α-phenylethylamino)-fluoran, 3-N-methyl-N-amylamino-6-methyl-7-anilinofluoran, 3-N-methyl-N-cyclohexylamino-6-methyl-7-anilinofluoran, 3-(N-benzyl-N-cyclohexylamino)-5,6-benzo-7-α-naphthylamino-4'-bromofluoran, 3-(N-p-tolyl-N-ethylamino)-6-methyl-7-anilinofluoran, 2'-[N-(3'-trifluoromethylphenyl)amino]-6-diethylaminofluoran, 3-(N,N-diethylamino)-5-methyl-7-(N,N-dibenzylamino)fluoran, 3-morpholino-7-(N-propyltrifluoromethylanilino)fluoran, 3-pyrrolidino-6-methyl-7-anilinofluoran, 3-pyrrolidino-7-(di-p-chlorophenyl)-methylaminofluoran, 3-pyrrolidino-7-trifluoromethylanilinofluoran, and salts or mixtures thereof.

Typically the leuco dye concentration in the compositions of the invention range from about 0.1% up to about 20% by weight, in particular from about 0.5 to about 15 by weight, and most particular from about 1 to about 10 by weight.

As the color developers for use in combination with the above leuco dyes in the present invention, a variety of electron acceptors or oxidizing agents capable of inducing color formation with the leuco dyes can be employed.

Suitable examples of electron acceptors or oxidizing agents that can be incorporated into compositions of the invention include, but are not limited to, inorganic acids, organic acids, phenolic materials, phenolic resins, bentonite, zeolite, acidic terra alba, activated clay, silica gel, alkyl-monophenols, alkyl-diphenols, thio-bis- alkyl-phenols, alkyl-phenylphenols, alkylcatechols, hydroxyl-alkylnaphthalenes, dihydroxyl-alkylnaphthalenes (any alkyl moiety having 1 to 12 carbon atoms), gallic acid alcohol esters, p-oxybenzoic acid alcohol esters, procatechuric acid alcohol esters, phenol-formaldehyde prepolymer, tert-butyl phenol, nonyl phenol, dodecyl phenol, styrenated phenol, 2,2'-methylene-bis-(4-methyl-6-tert-butyl phenol), α-naphthol, β-naphthol, hydroquinone monomethyl ether, guaiacol, eugenol, p-chlorophenol, p-bromophenol, o-chlorophenol, o-bromophenol, o-phenylphenol, p-phenylphenol, o-(o-chlorophenyl)phenol, p-(p-chlorophenyl)phenol, methyl p-hydroxybenzoate, ethyl p-hydroxybenzoate, propyl p-hydroxybenzoate, isopropyl p-hydroxybenzoate, octyl p-hydroxybenzoate, benzyl p-hydroxybenzoate, dodecyl phenol 3-isopropyl-catechol, p-tert-butyl catechol, 4,4'-methylene diphenol, 4,4'-thio-bis-(6-tert-butyl-3-methylphenol), bisphenol A, 1,2-dihydroxynaphthalene, 2,3-dihydroxynaphthalene, chlorocatechol, bromocatechol, 2,4-dihydroxybenzophenone, phenol, phthalein, o-cresol phthalein, methyl protocatechuate, ethyl protocatechuate, propyl protocatechuate, octyl protocatechuate, benzyl protocatechuate, dodecyl protocatechuate, 2,4,6-trihydroxymethylbenzene, methyl gallate, ethyl gallate, propyl gallate, butyl gallate, hexyl gallate, octyl gallate, dodecyl gallate, cetyl gallate, stearyl gallate, lauryl gallate, 2,4,6-trihydroxynaphthalene, tannic acid, phenol-formaldehyde prepolymers, resorcin, phloroglucin, 4,4'-isopropylidenebisphenol, 4,4'-isopropylidenebis-(o-methylphenol), 4,4'-isopropylidenebis-(2-tert-butylphenol), 4,4'- isopropylidenebis-(2-chlorophenol)phenol, 4,4'- sec-butylidene-bisphenol, 4,4'-cyclohexylidenebisphenol, 4,4'-butylidene-bis-(6-tert-butyl-2-methyl)phenol, 4,4'-thiobis- (6-tert-butyl-2-methyl)phenol, 2,2'-methylene-bis-(4-methyl-6-tert-butyl)phenol, 2,2'-methylene-bis-(4-ethyl-6-tert-butyl)phenol, 1,1,3-tris-(2-methyl-4-hydroxy-5-tert-butylphenyl)-butane, 1,1,3-tris-(2-methyl-4-hydroxy-5-cyclohexylphenyl)butane, 1,7-bis-(4-hydroxyphenylthio)-3,5-dioxaheptane, 1,5-bis-(4-hydroxyphenylthio)-3-oxapentane, 1,3-bis-(4-hydroxyphenylthio)-propane, 1,3-bis-(4-hydroxyphenylthio)-2-hydroxypropane, 2,2'-methylene-bis-(4-ethyl-6-tert-butylphenol), N,N'-diphenylthiourea, N,N'-di(m-chlorophenyl)thiourea, salicylanilide, 5-chloro- salicylanilide, salicyl-o-chloroanilide, 2-hydroxy-3-naphthoic acid, 2-hydroxy-1-naphthoic acid, 1-hydroxy-2-naphthoic acid, zinc salt of 1-acetoxy-3-naphthoic acid, zinc hydroxynaphthoic acid, aluminum hydroxynaphthoic acid, calcium hydroxynaphthoic acid, bis(4-hydroxyphenyl)methyl acetate, bis(4-hydroxyphenyl)benzyl acetate, 1,3-bis(4-hydroxy)cumyl benzene, 1,4-bis(4-hydroxy)cumyl benzene, 4,4'-diphenylsulfone, 4,2'-diphenylsulfone, 2,4'-diphenylsulfone, 4,4'-diphenylsulfoxide, 3,3'-diallyl-4,4'-diphenylsulfone, 3,4-dihydroxy-4'-methyldiphenylsulfone, 4-isopropoxy-4'-hydroxydiphenylsulfone, 4-benzyloxy-4'-hydroxydiphenylsulfone, tetrabromobisphenol·A, tetrabromobisphenol S and salts or mixtures thereof.

Typically the electron acceptor compound or oxidizing agent concentration in the compositions of the invention range from about 0.1% up to about 25% by weight, in particular from about 0.5 to about 15 by weight, and most particular from about 1 to about 10 by weight.

An additive which acts as a reaction medium or a solvent to help control the degree of color change is selected from long chain aliphatic alcohols. Specific examples used in the compositions of the present invention include, for example and are not limited to, 1-dodecanol, 1-decanol, 1-docosanol, 1-nonanol, 1-tetradecanol, 1-hexadecanol, 1-octadecanol, (2)-9-octadec-1-ol, 3-pentadecylcatechol and mixture thereof. Generally alcohols which have carbon atoms from 12 to 18 or having molecular weight greater than 100 are most useful. The reaction medium can also include polyvinyl alcohol which can be partially hydrolyzed (86-89 %), fully hydrolyzed (98-99 %) or super hydrolyzed (99.3+ %). The molecular weight range of the polyvinyl alcohol is from 50,000 to > 186,000.

Typically the long chain aliphatic alcohol, is incorporated into the compositions of the invention range from about 1% up to about 90% by weight, in particular from about 2 to about 80 by weight, and most particular from about 5 to about 75 by weight.

An additional additive to the surfactant, leuco dye and electron acceptor or oxidizing agent can be an alcohol ester, with a molecular weight range from 150 to about 1000. Suitable examples include, but are not limited to, octyl caprylate, decyl caprylate, octyl caprate, decyl caprate, cetyl caprate, stearyl caprate, butyl laurate, octyl laurate, lauryl laurate, stearyl laurate, butyl myristate, decyl myristate, myristyl myristate, cetyl myristate, octyl palmitate, butyl stearate, decyl stearate, lauryl stearate, stearyl stearate and 12-hydroxy stearic acid triglyceride.

Typically the alcohol ester, is incorporated into the compositions of the invention range from about 1% up to about 20% by weight, in particular from about 0.5 to about 15 by weight, and most particular from about 0.1 to about 5 by weight.

A typical ratio of long chain aliphatic alcohol to alcohol ester is about 9:1

The aqueous composition may include a surfactant and a metal salt. The composition can be used to prepare bubbles that have color disappearing characteristics and/or color changing characteristics. Optionally, one or more of the above-identified solvents, humectants, preservatives, fragrances, etc. as described throughout the application can be included in the composition.

The compositions can include one or more of the surfactant(s) described throughout the present application in combination with at least one water soluble metal salt.

Typically the metallic salt is selected from transition metals. Transition elements, undergo a color change with the loss of a water of hydration or when complexes are formed with organic materials. Salts of transition metals include those salts formed from Sc, Ti, V, Cr, Mn, Fe, Co , Ni, Cu, Zn, Y, Zr, Nb, Mo, Tc, Ru, Th, Pd, Ag, Cd, La, Hf, Ta, W, Re, Os, Ir, Pt, Au, Hg, Ac, Ce, Pr, Nd, Pm Sm, Eu, Gd, Tb, Dy, Ho, Er, Tm, Yb, Lu, Th and Pa. For example, color change can occur upon heating the metallic salts with long chain alcohols or polyvinyl alcohols. Representative examples of suitable transition metal salts include, but are not limited to, cobalt (II) chloride, cobalt (III) chloride, cobalt (II) bromide, cobalt (III) bromide, cobalt (II) nitrate, cobalt (III) nitrate, nickel (II) chloride, nickel (III) chloride, nickel (II) bromide, nickel (III) bromide, nickel (II) nitrate, nickel (III) nitrate, nickel (II) sulfate, nickel (II) sulfamate, nickel (II) oxalate, nickel (II) phthalocyanine, iron (II) chloride, iron (III) chloride, iron (II) bromide, iron (III) bromide, iron (II) nitrate, iron (III) nitrate, aluminium potassium sulfate.

Inorganic salts such as cobalt (II) chloride in combination with certain long chain alcohol produce the unusual effect of coloration due to the complex formation on heating and fade color on cooling. Specific examples of long chain alcohols that can interact with the transition metal to form a colored complex include, but are not limited to, 1-dodecanol, 1-tetradecanol, 1-hexadecanol, 1-docosanol, 1-decanol, 1-octadecanol, (2)-9-octadecen-1-ol, 1-nonanol, 3-pentadecylcatechol. Generally, suitable alcohols are those which have a carbon atom chain length from about 12 to about 18 carbon atoms or have molecular weight greater than 100. The reaction medium or solvent can also include polyvinyl alcohol which can be partially hydrolyzed (86-89 %), fully hydrolyzed (98-99 %) or super hydrolyzed (99.3+ %). The molecular weight range of polyvinyl alcohol is from 50,000 to > 186,000.

For example, when metal salts are heated with a long chain aliphatic alcohol or a polyvinyl alcohol, colored complexes are formed, and hence generate color. The color fades away when they are cooled. The color either disappears (color to colorless) or changes color (one color to another color).

### Colored, Color Disappearing or Color Changing Film Forming Compositions and Bubbles

The color changing and/or color disappearing compositions described herein can be combined with film forming technology as described infra. Additionally, substantially uniformly colored bubble compositions can be prepared with the film forming technology described infra. Suitable colored bubble compositions, such as those described in US Provisional Application No. 60/581,294, filed June 17, 2004, by Tim Kehoe, entitled "Composition and Method for Producing Colored Bubbles", the contents of which are incorporated herein in their entirety, can therefore be incorporated with the film forming technology described herein. The following are therefore preferred aspects of the invention:

In one aspect, the present invention pertains to an aqueous film forming color changing composition that includes a surfactant, an acid-base indicator according to claim 1, a base, such as a volatile base, and a film forming resin. In one embodiment, the composition provides a film in which the color changes or the color disappears. In another embodiment, the composition provides a colored bubble in which the color changes or the color disappears.

In still another aspect, the present invention pertains to an aqueous film forming color changing composition including a surfactant, a water soluble metal salt and a film forming resin. In one embodiment, the composition provides a film in which the color changes or the color disappears. In another embodiment, the composition provides a colored bubble in which the color changes or the color disappears.

The present invention provides the advantage(s) that a bubble that forms a strong film in the air permits the bubble to be caught, handled, stacked, and/or popped. The thin film of the bubble, for example, captures the color thus making the removal of the bubble or color from the bubble (if any) easy

The present invention further provides colored bubbles for films with uniform color intensity, large numbers of bubble, long lasting bubbles, bubbles that generate noise upon bursting, and in some cases, bubbles that are interconnected.

The compositions of the invention may include a resin which is water soluble, non-toxic and forms a film at room temperature. The water soluble film forming resins provide the compositions with a suitable viscosity for generating films or bubbles.

Representative examples of typical film forming resins employed in the compositions of the invention include, but are not limited to, polyvinylalcohol, polyvinylpyrrolidone, polyoxyethylene, polyvinyl acetate, gelatin, gum arabic, rosin, rosin-modified maleic acid resins, rosin-modified phenol resins, rosin esters, hydrogenated rosins, various cellulosic resins, methyl cellulose, ethyl cellulose, acetyl cellulose, propyl cellulose, butyl cellulose, hydroxypropyl cellulose, higher alkyl cellulose, petroleum resins, various phenol resins, starches and derivatives thereof. Mixtures of two or more of these film forming agents can also be employed in the present invention.

In particular, film forming resin include, but are not limited to PVP K-15, PVP K-30, PVP K-90, polyvinylpyrrolidone, manufactured by ISP (International Specialty Products), Wayne, NJ, Elvanol 40-05, Elvanol 40-16, Elvanol 40-40, Elvanol 70-14, Elvanol 70-27, Elvanol 60-30, Elvanol 70-03, Elvanol 70-04, Elvanol 70-06, Elvanol 70-20, Elvanol 70-30, Elvanol 70-62, Elvanol 70-63, Elvanol 70-75, Elvanol 71-30, Elvanol 90-50, Elvanol 50-14, Elvanol 50-26, Elvanol 50-42, Elvanol 51-03, Elvanol 51-04, Elvanol 51-05, Elvanol 51-08, Elvanol 52-22, Elvanol 75-15, Elvanol 85-82, Elvanol 85-91, polyvinylalcohol, manufactured by DuPont, Wilmington, DE, Celvol 125, Celvol 165, Celvol 350, Celvol 325, Celvol 310, Celvol 305, Celvol 103, Celvol 107, Celvol 203, Celvol 205, Celvol 418, Celvol 425, Celvol 443, Celvol 502, Celvol 504, Celvol 508, Celvol 513, Celvol 518, Celvol 523, Celvol 530, Celvol 540, Celvol 805, Celvol 823, Celvol 840, polyvinylalcohol, manufactured by Celanese Chemicals, Dallas TX, Polyox WSR N-10, Polyox WSR N-80, Polyox WSR N-750, Polyox WSR N-3000, Polyox WSR 205, Polyox WSR -1105, Polyox WSR N-12K, Polyox WSR N-60K, Polyox WSR-301, Polyox WSR-303, Polyox WSR-308, polyethylene oxide, manufactured by Dow Chemical Co., Midland, MI, K4484, water soluble starch, manufactured by National Starch & Chemicals, Bridgewater, NJ, Dri-sweet 42, water soluble starch, manufactured by American Maize and ICB 3000, water soluble starch, manufactured by Staley.

The film forming resin present in the composition of the invention are generally in a range from about 0.1% up to about 40% by weight, in particular from about 1% to about 35% by weight and more particularly from about 2% to about 30% by weight.

The combination of film forming resins, dye blockers and/or cleaners in the colored composition of the present invention provide enhanced washability on skin, fabrics, and hard surfaces such as finished/unfinished wood, stone, brick, leather, etc.

For films and bubbles that have a colorant that does not lose or changes color, various dyes and pigments can be incorporated into the compositions of the invention.

Suitable dyes can be selected from various dye classes that include, but are not limited to acid dyes, basic dyes, direct dyes, reactive dyes, sulfur dyes, fluorescent dyes, food dyes (FD&C) cosmetic dyes (D & C), solvent dyes and polymeric dyes.

The terms "acid dye" or "acidic dye" are recognized in the art and are intended to include those water soluble anionic dyes that are applied to a material from neutral to acid solution. Attachment to the material is attributed, at least partly, to salt formation between anionic groups in the dyes and cationic groups in the material. Generally, acid dyes have functional groups such as azo, triaryl methane or anthraquinone that include acid substituents such as nitro, carboxy or sulfonic acid groups.

Representative examples of acid dyes useful in the present compositions include, but are not limited to, Acid Black 1, Acid Black 2, Acid Black 24, Acid Black 48, Acid Blue 1, Acid Blue 7, Acid Blue 9, Acid Blue 25, Acid Blue 29, Acid Blue 40, Acid Blue 45, Acid Blue 74, Acid Blue 80, Acid Blue 83, Acid Blue 90, Acid Blue 92, Acid Blue 113, Acid Blue 120, Acid Blue 129, Acid Blue 147, Acid Green 1, Acid Green 3, Acid Green 5, Acid Green 25, Acid Green 27, Acid Green 50, Acid Orange 6, Acid Orange 7, Acid Orange 8, Acid Orange 10, Acid Orange 12, Acid Orange 51, Acid Orange 51, Acid Orange 63, Acid Orange 74, Acid Red 1, Acid Red 4, Acid Red 8, Acid Red 14, Acid Red 17, Acid Red 18, Acid Red 26, Acid Red 27, Acid Red 29, Acid Red 37, Acid Red 44, Acid Red 50, Acid Red 51, Acid Red 52, Acid Red 66, Acid Red 73, Acid Red 87, Acid Red 88, Acid Red 91, Acid Red 92, v Acid Red 94, Acid Red 97, Acid Red 103, Acid Red 114, Acid Red 150, Acid Red 151, Acid Red 183, Acid Violet 7, Acid Violet 9, Acid Violet 17, Acid Violet 19, Acid Yellow 1, Acid Yellow 3, Acid Yellow 9, Acid Yellow 11, Acid Yellow 17, Acid Yellow 23, Acid Yellow 25, Acid Yellow 29, Acid Yellow 34, Acid Yellow 36, Acid Yellow 42, Acid Yellow 54, Acid Yellow 73, Acid Yellow 76 and Acid Yellow 99.

The terms "base dye" or "basic dye" are recognized in the art and are intended to include those water soluble cationic dyes that are applied to a material from neutral to basic solution. Generally, basic dyes have functional groups such as sulfonium, oxonium, or quarternary ammonium functional groups. Attachment to the material is attributed, at least partly, to salt formation between cationic groups in the dyes and anionic groups in the material.

Representative examples of basic dyes useful in the present compositions include, but are not limited to, Basic Black 2, Basic Blue 3, Basic Blue 6, Basic Blue 7, Basic Blue 9, Basic Blue 11, Basic Blue 12, Basic Blue 16, Basic Blue 17, Basic Blue 24, Basic Blue 26, Basic Blue 41, Basic Blue 66, Basic Blue 140, Basic Brown 1, Basic Brown 4, Basic fuchsin, Basic Green 1, Basic Green 4, Basic Green 5, Basic Orange 2, Basic Orange 14, Basic Orange 21, Basic Red 1, Basic Red 2, Basic Red 5, Basic Red 9, Basic Red 29, Basic Violet 1, Basic Violet 2, Basic Violet 3, Basic Violet 4, Basic Violet 10, Basic Yellow 1 and Basic Yellow 2.

The term "direct dye" is recognized in the art and is intended to include those water soluble dyes that adsorb onto a material. Bonding is believed to occur through hydrogen bonding and/or Van der Waals forces between the dye and the substrate.

Representative examples of direct dyes useful in the present compositions include, but are not limited to, Direct Blue 1, Direct Blue 14, Direct Blue 53, Direct Blue 71, Direct Red 2, Direct Red 23, Direct Red 28, Direct Red 75, Direct Red 80, Direct Red 81, Direct Violet 51, Direct Yellow 4, Direct Yellow 7, Direct Yellow 8, Direct Yellow 9, Direct Yellow 12, Direct Yellow 27, Direct Yellow 50, Direct Yellow 59, Direct Yellow 62.

The term "reactive dye" is recognized in the art and is intended to include those dyes that contain a reactive group, for example, either a haloheterocycle or an activated double bond, that, when applied to a surface in a weakly alkaline solution, forms a chemical bond with a hydroxyl or amino group on the substrate.

Representative examples of reactive dye compounds useful in the present compositions include, but are not limited to, Procion red, blue, orange and yellow (ICI), Levafix E Yellow (Bayer), Remazol Yellow (Hoechst), Cibacron (Ciba), Drimarene X, R, K (Sandoz), Reactive Black 5, Reactive Blue 2, Reactive Blue 4, Reactive Blue 13, Reactive orange 16 and Reactive Yellow 4.

The term "sulfur dye" is recognized in the art and is intended to include those dyes that contain sulfide linkages and are absorbed by a substrate and are insolubilised within or on the substrate by oxidation. During this process the sulfur dye forms complex larger molecules which are the basis of their good wash-fastness.

Representative examples of sulfur dyes useful in the present compositions include, but are not limited to, Sulfur Black 1 (Sulfur Black T) and Sulfur Blue (Patent Blue VF).

The term "fluorescent dye" is recognized in the art and is intended to include those dyes which give fluorescence either in solid phase or in liquid form. The color of compound can be different from the fluorescence in liquid form.

Representative examples of fluorescent dyes useful in the present compositions include, but are not limited to, Fluorescein, fluorescein diacetate, carboxyfluorescein, carboxyfluorescein diacetate, rhodamine B, sulforhodamine B, cotadecyl rhodamine B, rhodamine 6G, rhodamine 110, rhodaine 123, coumarine, resorufin, quinoxalines, pyrido[1,2-a]benzimidazoles, acridine, acriflavin, acridine orange, nonyl acridine orange, xanthene, eosin Y, pyronine Y, texas red, calcein, quinacrine, ethidium bromide, propidium iodide, resazurin, nile, crystal violet, DiO6(3), JC-1, YOYO-1, DAPI, Hoechst 33342, FM 1-43, thiazole orange, primuline, thioflavin T, calcein blue, morin, and fura-2.

The term "solvent dye"" is recognized in the art. Solubility in an organic solvent or solvents is a characteristics physical property of a solvent dye.

Representative examples of solvent dyes useful in the present compositions include, but are not limited to, Solvent Black 3, Solvent Black 5, Solvent Blue 14, Solvent Blue 35, Solvent Blue 38, Solvent Blue 43, Solvent Blue 59, Solvent Brown 1, Solvent Green 1, Solvent Green 3, Solvent Green 7, Solvent Green 11, Solvent Orange 1, Solvent Orange 2, Solvent Orange 7, Solvent Orange 15, Solvent Red 19, Solvent Red 23, Solvent Red 24, Solvent Red 26, Solvent Red 27, Solvent Red 41, Solvent Red 43, Solvent Red 45, Solvent Red 49, Solvent Red 72, Solvent Violet 8, Solvent Yellow 2, Solvent Yellow 3, Solvent Yellow 7, Solvent Yellow 14, Solvent Yellow 33, Solvent Yellow 94, manufactured by Sigma-Aldrich, St. Louis, MO; and Special Fluorescent Yellow 3G (Solvent Green 7), manufactured by Lanxess Corporation, Pittsburgh, PA.

The terms "FD&C" and "D&C" dyes are recognized in the art. In the United States, colorants for food, drugs and cosmetics are regarded as "color additives". The Federal Food, Drug & Cosmetic (FD&C) Act of 1938 made food color additive certification mandatory. Since then the Food and Drug Administration (FDA) has been responsible for regulating all color additives used in food, drugs and cosmetics. Each batch to be sold in the United States has to be certified by the FDA. To avoid confusing color additives used in food with those manufactured for other uses, 3 categories of certifiable color additives were created: 1) FD&C (Food, Drug & Cosmetics) color additives with applications in food, drug & cosmetics; 2) D&C (Drug & Cosmetics) color additives with applications in drug & cosmetics; 3) External D&C (External Drug & Cosmetics) color additives with applications in externally applied drugs & in externally applied cosmetics. The use of all food colors approved for use in the United States are listed in 21 CFR (Code of Federal Regulation), parts 70 through 82 dealing with color additives.

Representative examples of FD&C dyes useful in compositions of the invention include, but are not limited to FD&C Blue 1, FD&C Blue 2, FD&C Green 3, FD&C Red 3, FD&C Red 40, FD&C Yellow 5, FD&C Yellow 6, Fast Emerald Green, and mixtures thereof, manufactured by Sensient Colors Inc., St. Louis, MO, Vitasyn Tetrazine X 90, Vitasyn Orange RGL 90, Vitasyn Quinoline Yellow 70, Vitasyn Ponceau 4RC 82, Vitasyn Blue AE 90, Vitasyn Patent Blue V 85 01, Sanolin Flavin 8GZ, Sanolin Yellow BG, Sanolin Red NBG, Sanolin Rhodamine B, Sanolin Violet E2R, Sanolin Violet FBL, Sanolin Blue NBL, Sanolin Blue EHRL, Sanolin Blue EHRL Liquid, and mixtures thereof, manufactured by Clariant Corp., Coventry, RI.

The term "polymeric colorant" is recognized in the art and polymeric colorants are a group of intermediate or high molar mass compounds that are intrinsically colored. Polymeric dyes may be defined through their applications as polymers and dyes, which possess suitably high tinctorial strength. Polymeric dyes are characterized by having polymeric chains covalently bonded to a chromophore (dye) molecule.

Representative examples of polymeric dyes useful in compositions of the invention include, but are not limited to , Palmer Orange B113, Palmer Blue B232, Palmer Magenta, Palmer Fluorescent Red, Palmer Yellow R, Palmer Scarlett, Palmer Black B57, Palmer Patent Blue, LiquiTone Magenta 418, Polytint Violet X80LT, Polytint Orange X96, Polytint Yellow X15, Polytint Black X41LV, Polytint Red X64, Polytint Blue X3LV, & mixtures thereof, manufactured by Milliken & Co., Spartanburg, SC.

Alternatively, pigments can be incorporated into the compositions of the invention. Suitable examples of pigments include those known as Hydrus™ (available from Salis International Inc./Dr. Ph. Martin's). Currently there are 24 Hydrus™ colors that can be used within the scope of the present invention.

Colorants (dyes and pigments) that do not change color or have color dissipation are included in the compositions of the invention in ranges from about 1% to about 50% by weight, more particularly from about 3% to about 30% by weight and in particular from about 5% to about 15% by weight.

The compositions of the present invention can be used with any simple or complex bubble making device, apparatus or machine to generate bubbles.

The compositions of the present invention provide bubbles that have at least average bubble integrity and lifespan. In particular embodiments, the compositions that do not contain a film forming resin, resultant bubbles maintain integrity and/or lifespan for 1 second to about 30 minutes, more particularly from about 2 seconds to about 20 minutes and most particularly from about 5 seconds to about 5 minutes. In particular embodiments, where the compositions include a film forming resin, resultant bubbles maintain integrity and/or lifespan for 1 second to about 30 days, more particularly from about 2 seconds to about 25 days and most particularly from about 5 seconds to about 20 days.

The compositions of the present invention can be prepared by the following general method. Either 1%, 3% or 5% indicator, 2 equivalent of sodium hydroxide, 16g Colonial SLS (30%) solution with the reaminder being remaining DI water to make total 100g. When polyvinyl alcohol was added, heating was required but otherwise reaction was stirred at room temperature. Additional additives such as deionized water, surfactant, preservatives, amine, dye blockers and polyvinyl alcohol solution (in case of film forming bubbles) are added and the reaction mixture further stirred for 2 hours at room temperature.

In certain experiments were polyvinyl alcohol in prepared in situ, the following procedure can be used. Polyvinyl alcohol is added with stirring to deionized water at room temperature. After the addition is complete, the mixture is heated at 90°C for 30 min, cooled to room temperature followed by addition of dye, humectant (glycerin or triethanolamine), surfactant, base, dye blockers and/or preservatives.

After cooling, the compositions may be bottled. Alternately, the solution may be bottled without cooling.

The present invention further includes kits that include the compositions of the invention and instructions how to use the compositions to form films or bubbles. Typically, the solution is spread onto a surface to form a film or a suitable object is dipped into the solution and subjected to air flow to form a bubble or bubbles.

Aspects of the present teachings can be further understood in light of the following examples, which should not be construed as limiting the scope of the present teachings in any way.

### Examples

### Synthesis of acid-base indicators (precursors to those suitable for the invention, example 10 is not within the scope of the invention):

### EXAMPLE 1

### Synthesis of 3,3-bis-(4-hydroxy-3-isopropylphenyl)-1-(3H)-isobenzofuranone

A mixture of 2-isopropylphenol (0.2M), phthalic anhydride (0.1M), polyphosphoric acid (0.25M), and zinc chloride (0.01M), was stirred and heated at 100C for 3 hours. The reaction mixture was cooled to room temperature and added to ice-water mixture when the product precipitated. The product was filtered, thoroughly washed with water and dried. Recrystallization from ethanol with charcoal treatment furnished pure 3,3-bis-(4-hydroxy-3-isopropylphenyl)-1-(3H)-isobenzofuranone in 96% yield.

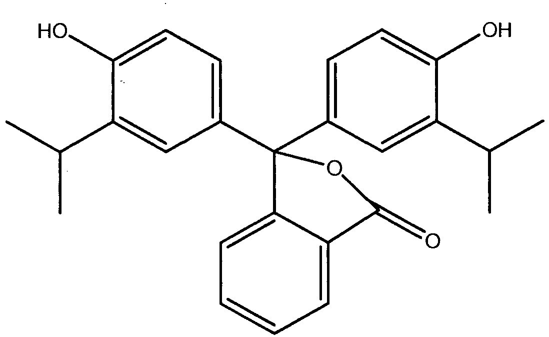

### EXAMPLE 2

### Synthesis of 3,3-bis-(4-hydroxy-3,5-diisopropylphenyl)-1-(3H)-isobenzofuranone

A mixture of 2,6-diisopropylphenol (0.2M), phthalic anhydride (0.1M), polyphosphoric acid (0.25M), and zinc chloride (0.01M), was stirred and heated at 100C for 3 hours. The reaction mixture was cooled to room temperature and added to ice-water mixture when the product precipitated. The product was filtered, thoroughly washed with water and dried. Recrystallization from ethanol with charcoal treatment furnished pure 3,3-bis-(4-hydroxy-3,5-diisopropylphenyl)-1-(3H)-isobenzofuranone in 98% yield.

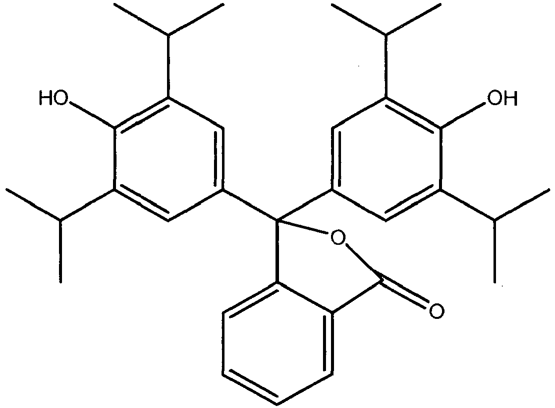

### EXAMPLE 3

### Synthesis of 3,3-bis-(4-hydroxy-2-nitrophenyl)-1-(3H)-isobenzofuranone

A mixture of 3-nitrophenol (0.2M), phthalic anhydride (0.1M), polyphosphoric acid (0.25M), and zinc chloride (0.01M), was stirred and heated at 100C for 3 hours. The reaction mixture was cooled to room temperature and added to ice-water mixture when the product precipitated. The product was filtered, thoroughly washed with water and dried. Recrystallization from ethanol with charcoal treatment furnished pure 3,3-bis-(4-hydroxy-2-nitrophenyl)-1-(3H)-isobenzofuranone in 81% yield.

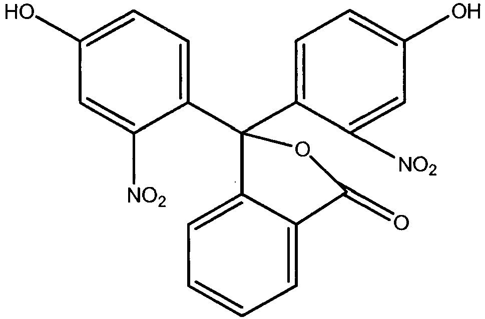

### EXAMPLE 4

### Synthesis of 3,3-bis-(4-hydroxy-3-nitrophenyl)-1-(3H)-isobenzofuranone

A mixture of 2-nitrophenol (0.2M), phthalic anhydride (0.1M), polyphosphoric acid (0.25M), and zinc chloride (0.01M), was stirred and heated at 100C for 3 hours. The reaction mixture was cooled to room temperature and added to ice-water mixture when the product precipitated. The product was filtered, thoroughly washed with water and dried. Recrystallization from ethanol with charcoal treatment furnished pure 3,3-bis-(4-hydroxy-3-nitrophenyl)-1-(3H)-isobenzofuranone in 89% yield.

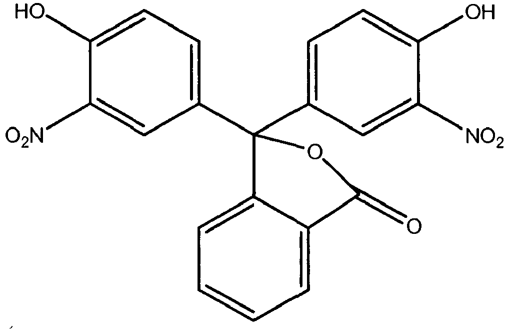

### EXAMPLE 5

### Synthesis of 3,3-bis-[4-hydroxy-2-(N,N-diethylamino)phenyl]-1-(3H)-isobenzofuranone

A mixture of 3-(N,N-diethylamino)phenol (0.2M), phthalic anhydride (0.1M), polyphosphoric acid (0.25M), and zinc chloride (0.01M), was stirred and heated at 100C for 3 hours. The reaction mixture was cooled to room temperature and added to ice-water mixture when the product precipitated. The product was filtered, thoroughly washed with water and dried. Recrystallization from ethanol with charcoal treatment furnished pure 3,3-bis-[4-hydroxy-2-(N,N-diethylamino)phenyl]-1-(3H)-isobenzofuranone in 93% yield.

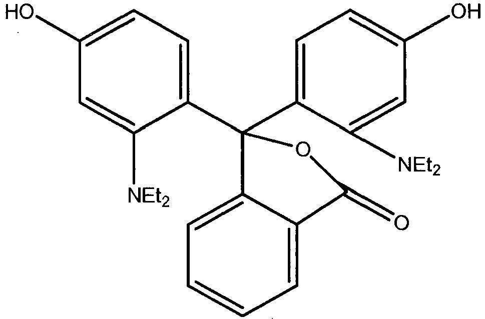

### EXAMPLE 6

### Synthesis of 3,3-bis-(4-hydroxy-3-ethylphenyl)-1-(3H)-isobenzofuranone

A mixture of 2-ethylphenol (0.2M), phthalic anhydride (0.1M), polyphosphoric acid (0.25M), and zinc chloride (0.01M), was stirred and heated at 100C for 3 hours. The reaction mixture was cooled to room temperature and added to ice-water mixture when the product precipitated. The product was filtered, thoroughly washed with water and dried. Recrystallization from ethanol with charcoal treatment furnished pure 3,3-bis-(4-hydroxy-3-ethylphenyl)-1-(3H)-isobenzofuranone in 92% yield.

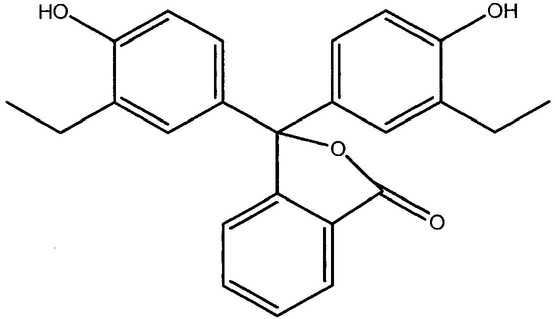

### EXAMPLE 7

### Synthesis of 3,3-bis-(4-hydroxy-3-ethoxyphenyl)-1-(3H)-isobenzofuranone

A mixture of 2-ethoxyphenol (0.2M), phthalic anhydride (0.1M), polyphosphoric acid (0.25M), and zinc chloride (0.01M), was stirred and heated at 100C for 3 hours. The reaction mixture was cooled to room temperature and added to ice-water mixture when the product precipitated. The product was filtered, thoroughly washed with water and dried. Recrystallization from ethanol with charcoal treatment furnished pure 3,3-bis-(4-hydroxy-3-ethoxyphenyl)-1-(3H)-isobenzofuranone in 85% yield.

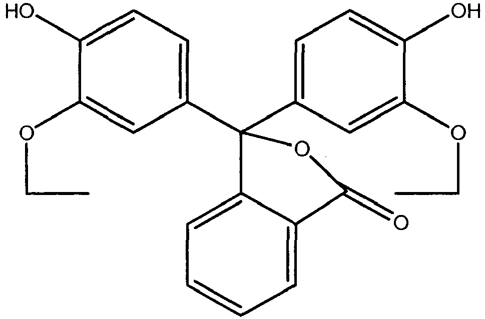

### EXAMPLE 8

### Synthesis of 3,3-bis-(4-hydroxy-3-acetamidophenyl)-1-(3H)-isobenzofuranone

A mixture of 2-acetamidophenol (0.2M), phthalic anhydride (0.1M), polyphosphoric acid (0.25M), and zinc chloride (0.01M), was stirred and heated at 100C for 3 hours. The reaction mixture was cooled to room temperature and added to ice-water mixture when the product precipitated. The product was filtered, thoroughly washed with water and dried. Recrystallization from ethanol with charcoal treatment furnished pure 3,3-bis-(4-hydroxy-3-acetamidophenyl)-1-(3H)-isobenzofuranone in 83% yield.

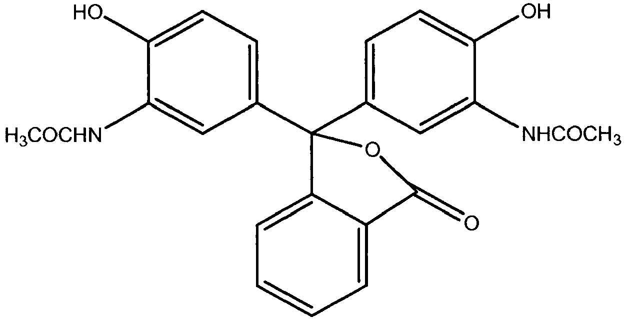

### EXAMPLE 9

### Synthesis of 3,3-bis-(4-hydroxy-6-methyl-3-nitrophenyl)-1-(3H)-isobenzofuranone

A mixture of 5-methyl-2-nitrophenol (0.2M), phthalic anhydride (0.1M), polyphosphoric acid (0.25M), and zinc chloride (0.01M), was stirred and heated at 100C for 3 hours. The reaction mixture was cooled to room temperature and added to ice-water mixture when the product precipitated. The product was filtered, thoroughly washed with water and dried. Recrystallization from ethanol with charcoal treatment furnished pure 3,3-bis-(4-hydroxy-6-methyl-3-nitrophenyl)-1-(3H)-isobenzofuranone in 81% yield.

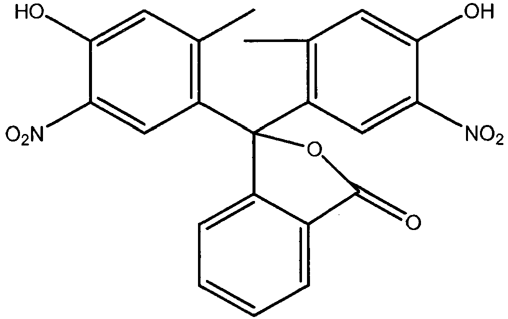

### EXAMPLE 10

### Synthesis of 3,3-bis-(4-hydroxy-6-methyl-5-quinolin-1-yl)-1-(3H)-isobenzofuranone

A mixture of 8-hydroxyquinaldine (0.2M), phthalic anhydride (0.1M), polyphosphoric acid (0.25M), and zinc chloride (0.01M), was stirred and heated at 100C for 3 hours. The reaction mixture was cooled to room temperature and added to ice-water mixture when the product precipitated. The product was filtered, thoroughly washed with water and dried. Recrystallization from ethanol with charcoal treatment furnished pure 3,3-bis-(4-hydroxy-6-methyl-5-quinolin-1-yl)-1-(3H)-isobenzofuranone in 88% yield.

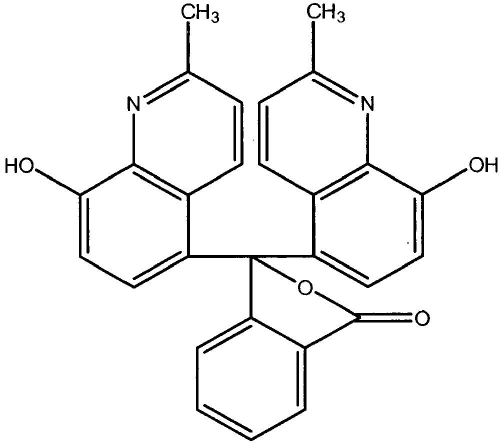

### EXAMPLE 11

### Synthesis of 3,3-bis-(4-hydroxy-3-pyridin-1-yl)-1-(3H)-isobenzofuranone

A mixture of 2-hydroxypyridine (0.2M), phthalic anhydride (0.1M), polyphosphoric acid (0.25M), and zinc chloride (0.01M), was stirred and heated at 100C for 3 hours. The reaction mixture was cooled to room temperature and added to ice-water mixture when the product precipitated. The product was filtered, thoroughly washed with water and dried. Recrystallization from ethanol with charcoal treatment furnished pure 3,3-bis-(4-hydroxy-3-pyridin-1-yl)-1-(3H)-isobenzofuranone in 80% yield.

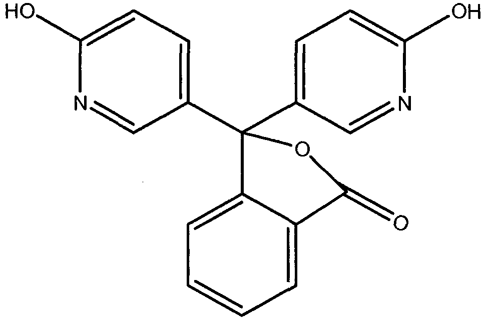

### EXAMPLE 12

### Synthesis of 3,3-bis-(4-hydroxy-2-pyridin-1-yl)-1-(3H)-isobenzofuranone

A mixture of 3-hydroxypyridine (0.2M), phthalic anhydride (0.1M), polyphosphoric acid (0.25M), and zinc chloride (0.01M), was stirred and heated at 100C for 3 hours. The reaction mixture was cooled to room temperature and added to ice-water mixture when the product precipitated. The product was filtered, thoroughly washed with water and dried. Recrystallization from ethanol with charcoal treatment furnished pure 3,3-bis-(4-hydroxy-2-pyridin-1-yl)-1-(3H)-isobenzofuranone in 82% yield.

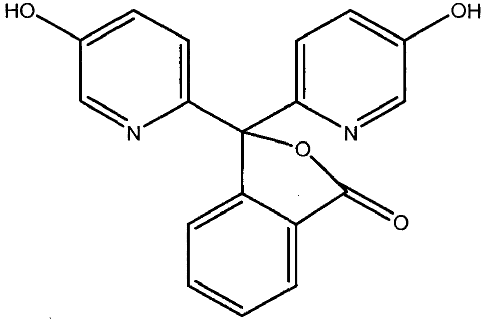

### EXAMPLE 13

### Synthesis of 3,3-bis-(4-hydroxy-3-phenylphenyl)-1-(3H)-isobenzofuranone

A mixture of 2-phenylphenol (0.133mol), phthalic anhydride (0.074mol), reaction medium (0.416mol), and Lewis acid (0.029mol), was stirred and heated at 90°C for 5 hours. The reaction mixture was cooled to room temperature and added to ice-water mixture when the product precipitated. The product was filtered, thoroughly washed with water and dried. Recrystallization from methanol with charcoal treatment furnished pure 3,3-bis-(4-hydroxy-3-phenylphenyl)-1-(3H)-isobenzofuranone as white crystals in 94% yield. ¹H-NMR (DMSO-d₆, 300MHz): δ 9.89 (s, 2H, 2OH), 6.97-7.18 (m, 6H, aromatic), 7.26-7.47 (m, 10H, aromatic), 7.63-7.92 (m, 4H, aromatic) ppm. Mass spectra: m/z 470 (M⁺).

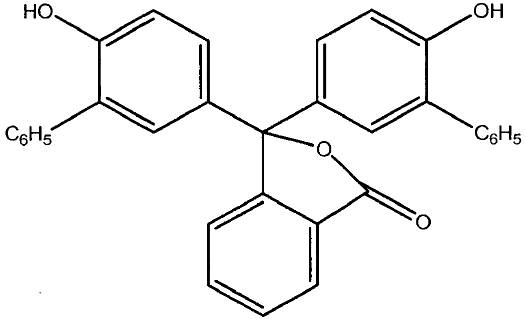

### EXAMPLE 14

### Synthesis of 3,3-bis-(4-hydroxy-3,5-diisopropylphenyl)-1-(3H)-isobenzofuranone

A mixture of 2,6-diisopropylphenol (0.133mol), phthalic anhydride (0.074mol), reaction medium (0.416mol), and Lewis acid (0.029mol), was stirred and heated at 90°C for 5 hours. The reaction mixture was cooled to room temperature and added to ice-water mixture when the product precipitated. The product was filtered, thoroughly washed with water and dried. Recrystallization from methanol with charcoal treatment furnished pure 3,3-bis-(4-hydroxy-3,5-diisopropylphenyl)-1-(3H)-isobenzofuranone as white crystals in 89% yield. IR (KBr): 3506, 1734, 1609 cm⁻¹. ¹H-NMR (DMSO-d₆): δ 9.56 (s, 2H, 2OH), 1.02-1.05 (dd, 24H, 8CH₃), 3.22-3.31 (heptate, 4H, 4CH), 6.74-7.00 (m, 4H, aromatic), 7.59-7.92 (m, 4H, aromatic) ppm. Mass spectra: m/z 486 (M⁺).

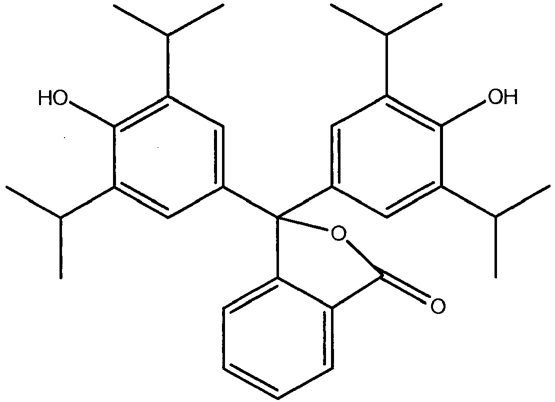

### EXAMPLE 15

### Synthesis of 3,3-bis-(4-hydroxy-3,5-dimethoxyphenyl)-1-(3H)-isobenzofuranone

A mixture of 2,6-dimethoxyphenol (0.133mol), phthalic anhydride (0.074mol), reaction medium (0.416mol), and Lewis acid (0.029mol), was stirred and heated at 90°C for 5 hours. The reaction mixture was cooled to room temperature and added to ice-water mixture when the product precipitated. The product was filtered, thoroughly washed with water and dried. Recrystallization from methanol with charcoal treatment furnished pure 3,3-bis-(4-hydroxy-3,5-dimethoxyphenyl)-1-(3H)-isobenzofuranone as white crystals in 84% yield. IR (KBr): 3388, 1769, 1606, 1369 cm⁻¹. ¹H-NMR (DMSO-d₆): δ 8.71 (s, 2H, 2OH), 3.66 (s, 12H, 4OCH₃), 7.65-7.68 (m, 4H, aromatic), 7.83-7.96 (m, 4H, aromatic) ppm. Mass spectra: m/z 438 (M⁺).

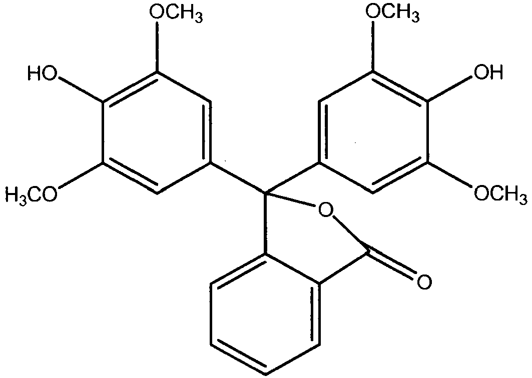

### EXAMPLE 16

### Synthesis of 3,3-bis-(4-hydroxy-3,5-dimethylphenyl)-1-(3H)-isobenzofuranone

A mixture of 2,6-dimethylphenol (0.133mol), phthalic anhydride (0.074mol), reaction medium (0.416mol), and Lewis acid (0.029mol), was stirred and heated at 90°C for 5 hours. The reaction mixture was cooled to room temperature and added to ice-water mixture when the product precipitated. The product was filtered, thoroughly washed with water and dried. Recrystallization from methanol with charcoal treatment furnished pure 3,3-bis-(4-hydroxy-3,5-dimethylphenyl)-1-(3H)-isobenzofuranone as white crystals in 91% yield. IR (KBr): 3582, 3386, 1746, 1605 cm⁻¹. ¹H-NMR (DMSO-d₆): δ 8.45 (s, 2H, 2OH), 2.10 (s, 12H, 4CH₃), 7.58-7.63 (m, 4H, aromatic), 7.78-7.87 (m, 4H, aromatic) ppm. Mass spectra: m/z 374 (M⁺).

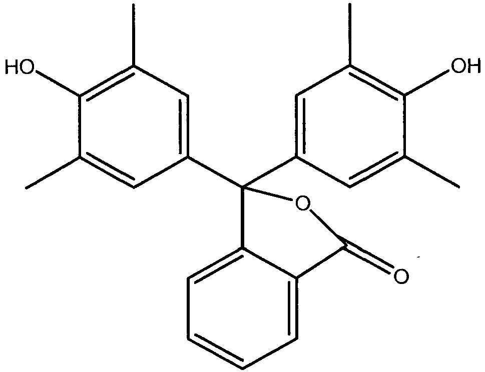

### EXAMPLE 17

### Synthesis of 3,3-bis-(4-hydroxy-3,6-diimethylphenyl)-1-(3H)-isobenzofuranone

A mixture of 2,5-dimethylphenol (0.133mol), phthalic anhydride (0.074mol), reaction medium (0.416mol), and Lewis acid (0.029mol), was stirred and heated at 90°C for 5 hours. The reaction mixture was cooled to room temperature and added to ice-water mixture when the product precipitated. The product was filtered, thoroughly washed with water and dried. Recrystallization from methanol with charcoal treatment furnished pure 3,3-bis-(4-hydroxy-3,6-diimethylphenyl)-1-(3H)-isobenzofuranone as pale yellow crystals in 85% yield. IR (KBr): 3393, 1729, 1611 cm⁻¹. ¹H-NMR (DMSO-d₆): δ 9.40 (s, 2H, 2OH), 1.95 (s, 12H, 4CH₃), 6.59-6.63 (m, 4H, aromatic), 7.46-7.91 (m, 4H, aromatic) ppm. Mass spectra: m/z 374 (M⁺).

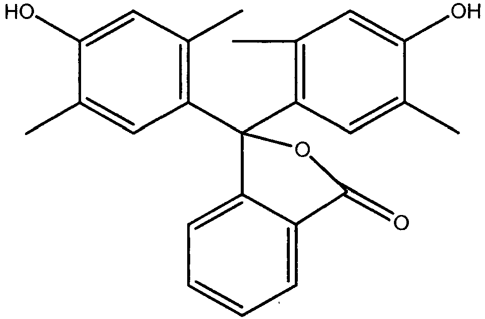

### EXAMPLE 18

### Synthesis of 3,3-bis-(4-hydroxy-3-ethylphenyl)-1-(3H)-isobenzofuranone

A mixture of 2-ethylphenol (0.133mol), phthalic anhydride (0.074mol), reaction medium (0.416mol), and Lewis acid (0.029mol), was stirred and heated at 90°C for 5 hours. The reaction mixture was cooled to room temperature and added to ice-water mixture when the product precipitated. The product was filtered, thoroughly washed with water and dried. Recrystallization from ethyl acetate:petroleum ether (1:1) with charcoal treatment furnished pure 3,3-bis-(4-hydroxy-3-ethylphenyl)-1-(3H)-isobenzofuranone as white crystals in 81% yield. IR (KBr): 3389, 1783, 1718, 1605 cm⁻¹. ¹H-NMR (DMSO-d₆): δ 9.54 (s, 2H, 2OH), 2.43-2.50 (q, 4H, 2CH₂), 1.00-1.05 (t, 6H, 2CH₃), 6.74-6.96 (m, 6H, aromatic), 7.57-7.89 (m, 4H, aromatic) ppm. Mass spectra: m/z 374 (M⁺).

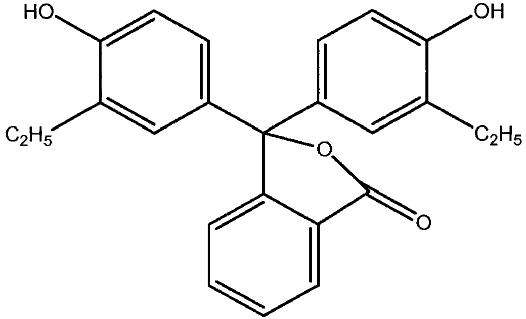

### EXAMPLE 19

### Synthesis of 3,3-bis-(4-hydroxy-3-isopropylphenyl)-1-(3H)-isobenzofuranone

A mixture of 2-isopropylphenol (0.133mol), phthalic anhydride (0.074mol), reaction medium (0.416mol), and Lewis acid (0.029mol), was stirred and heated at 90°C for 5 hours. The reaction mixture was cooled to room temperature and added to ice-water mixture when the product precipitated. The product was filtered, thoroughly washed with water and dried. Recrystallization from methanol:water (1:1) with charcoal treatment furnished pure 33,3-bis-(4-hydroxy-3-isopropylphenyl)-1-(3H)-isobenzofuranone as white crystals in 83% yield. IR (KBr): 3383, 1733, 1608 cm⁻¹. ¹H-NMR (DMSO-d₆): δ 9.57 (s, 2H, 2OH), 1.05-1.07 (dd, 12H, 4CH₃), 3.11-3.18 (heptate, 2H, 2CH), 6.75-7.01 (m, 6H, aromatic), 7.59-7.90 (m, 4H, aromatic) ppm. Mass spectra: m/z 402 (M⁺).

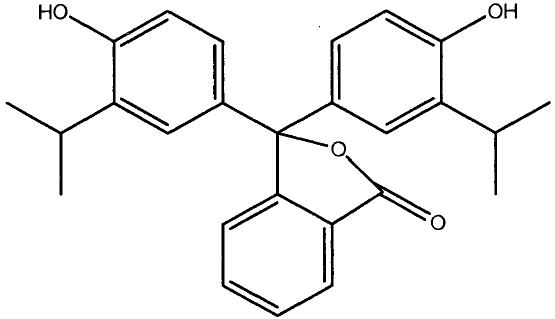

### EXAMPLE 20

### Synthesis of 3,3-bis-(4-hydroxy-3-methoxyphenyl)-1-(3H)-isobenzofuranone

A mixture of 2-methoxyphenol (0.133mol), phthalic anhydride (0.074mol), reaction medium (0.416mol), and Lewis acid (0.029mol), was stirred and heated at 90°C for 5 hours. The reaction mixture was cooled to room temperature and added to ice-water mixture when the product precipitated. The product was filtered, thoroughly washed with water and dried. Recrystallization from methanol with charcoal treatment furnished pure 3,3-bis-(4-hydroxy-3-methoxyphenyl)-1-(3H)-isobenzofuranone as white crystals in 79% yield. IR (KBr): 3517, 1747, 1701, 1279 cm⁻¹. ¹H-NMR (DMSO-d₆): δ 9.27 (s, 2H, 2OH), 3.66 (s, 6H, 2OCH₃), 6.65-6.78 (m, 6H, aromatic), 7.61-7.90 (m, 4H, aromatic) ppm. Mass spectra: m/z 378 (M⁺).

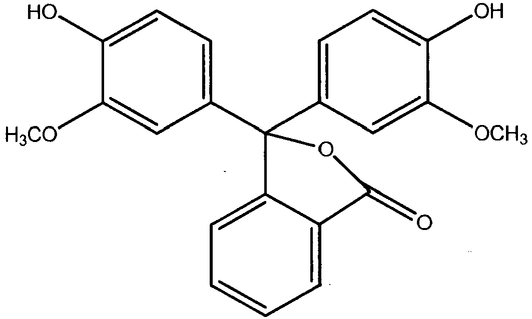

### EXAMPLE 21

### Synthesis of 3,3-bis-(4-hydroxy-2,3,5-trimethylphenyl)-1-(3H)-isobenzofuranone

A mixture of 2,3,6-trimethylphenol (0.133mol), phthalic anhydride (0.074mol), reaction medium (0.416mol), and Lewis acid (0.029mol), was stirred and heated at 90°C for 5 hours. The reaction mixture was cooled to room temperature and added to ice-water mixture when the product precipitated. The product was filtered, thoroughly washed with water and dried. Recrystallization from methanol with charcoal treatment furnished pure 3,3-bis-(4-hydroxy-2,3,5-trimethylphenyl)-1-(3H)-isobenzofuranone as white crystals in 73% yield. IR (KBr): 3510, 3390, 1746, 1609, cm⁻¹. ¹H-NMR (DMSO-d₆): δ 9.44 (s, 2H, 2OH), 2.05 (s, 18H, 6CH₃), 6.55 (s, 2H, aromatic), 7.46-7.90 (m, 4H, aromatic) ppm. Mass spectra: m/z 402 (M⁺).

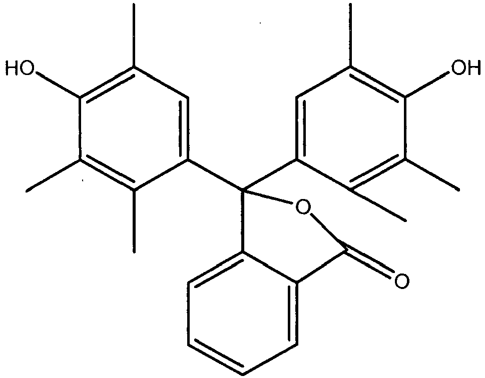

### EXAMPLE 22

### Synthesis of 3,3-bis-(4-hydroxy-3-sec-butylphenyl)-1-(3H)-isobenzofuranone

A mixture of 2-sec-butylphenol (0.133mol), phthalic anhydride (0.074mol), reaction medium (0.416mol), and Lewis acid (0.029mol), was stirred and heated at 90°C for 5 hours. The reaction mixture was cooled to room temperature and added to ice-water mixture when the product precipitated. The product was filtered, thoroughly washed with water and dried. Recrystallization from methanol with charcoal treatment furnished pure 3,3-bis-(4-hydroxy-3-sec-butylphenyl)-1-(3H)-isobenzofuranone as white crystals in 77% yield. IR (KBr): 3400, 1722, 1607 cm⁻¹. ¹H-NMR (DMSO-d₆): δ 9.50 (s, 2H, 2OH), 0.80 (t, 6H, 2CH₃), 1.35-1.39 (p, 4H, 2CH₂), 1.22 (d, 6H, 2CH₃), 2.89-2.97 (sextate, 2H, 2CH), 6.73-6.93 (m, 6H, aromatic), 7.59-7.90 (m, 4H, aromatic) ppm. Mass spectra: m/z 430 (M⁺).

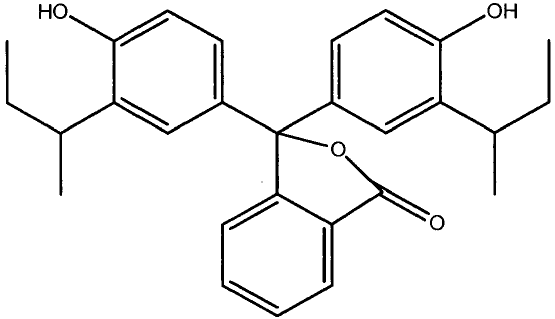

### EXAMPLE 23

### Synthesis of 3,3-bis-(4-hydroxy-3-nitrophenyl)-1-(3H)-isobenzofuranone

A mixture of phenolphthalein (0.062mol) in acetic acid (290mL) was stirred at 15°C. Concentrated nitric acid (0.136mol, 65%) in acetic acid (10mL) was slowly added to stirring mixture at 15°C. The reaction mixture was further stirred for 6 hours at room temperature and added to ice-water mixture when the yellow colored product precipitated. The product was filtered, thoroughly washed with water and dried. Recrystallization from ethanol with charcoal treatment furnished pure 3,3-bis-(4-hydroxy-3-nitrophenyl)-1-(3H)-isobenzofuranone as pale yellow crystals in 78% yield. IR (KBr): 3262, 1766, 1627, 1538, 1423 cm⁻¹. ¹H-NMR (DMSO-d₆): δ 9.67 (s, 2H, 2OH), 6.71-7.16 (m, 6H, aromatic), 7.46-7.98 (m, 4H, aromatic) ppm. Mass spectra: m/z 408 (M⁺).

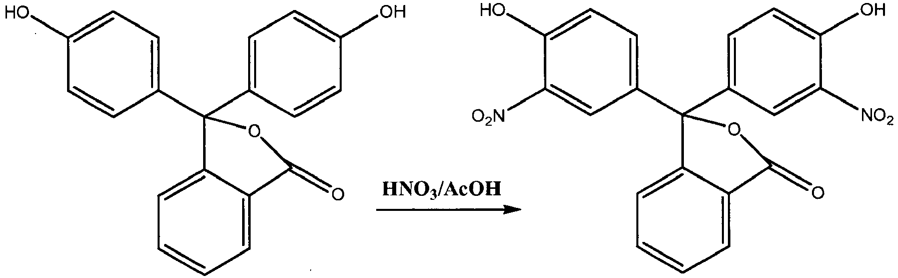

### Synthesis of disodium salts of acid-base indicators for water based systems (example 10 is not within the scope of the present invention):

### EXAMPLE 1

### Synthesis of disodium salt of 3,3-bis-(4-hydroxy-3-isopropylphenyl)-1-(3H)-isobenzofuranone

A mixture of 3,3-bis-(4-hydroxy-3-isopropylphenyl)-1-(3H)-isobenzofuranone (0.01M) in ethanol (50mL, 85%) was stirred followed by addition of sodium hydroxide (0.02M) in ethanol (50mL, 85%). The reaction mixture was stirred and refluxed for 2 hours, cooled to room temperature. The solvent was evaporated on rotary evaporator, isolated the crude product and dried. Recrystallization from ethanol furnished pure disodium salt in 98% yield.

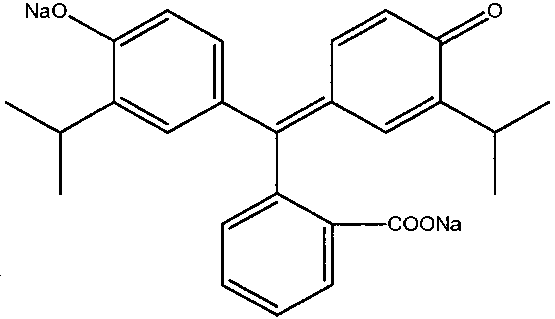

### EXAMPLE 2

### Synthesis of disodium salt of 3,3-bis-(4-hydroxy-3,5-diisopropylphenyl)-1-(3H)-isobenzofuranone

A mixture of 3,3-bis-(4-hydroxy-3,5-diisopropylphenyl)-1-(3H)-isobenzofuranone (0.01M) in ethanol (50mL, 85%) was stirred followed by addition of sodium hydroxide (0.02M) in ethanol (50mL, 85%). The reaction mixture was stirred and refluxed for 2 hours, cooled to room temperature. The solvent was evaporated on rotary evaporator, isolated the crude product and dried. Recrystallization from ethanol furnished pure disodium salt in 94% yield.

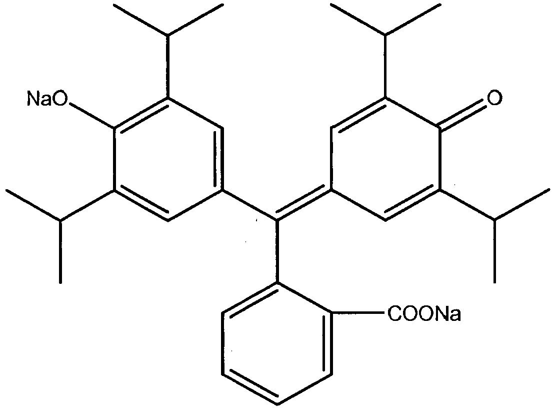

### EXAMPLE 3

### Synthesis of disodium salt of 3,3-bis-(4-hydroxy-2-nitrophenyl)-1-(3H)-isobenzofuranone

A mixture of 3,3-bis-(4-hydroxy-2-nitrophenyl)-1-(3H)-isobenzofuranone (0.01M) in ethanol (50mL, 85%) was stirred followed by addition of sodium hydroxide (0.02M) in ethanol (50mL, 85%). The reaction mixture was stirred and refluxed for 2 hours, cooled to room temperature. The solvent was evaporated on rotary evaporator, isolated the crude product and dried. Recrystallization from ethanol furnished pure disodium salt in 88% yield.

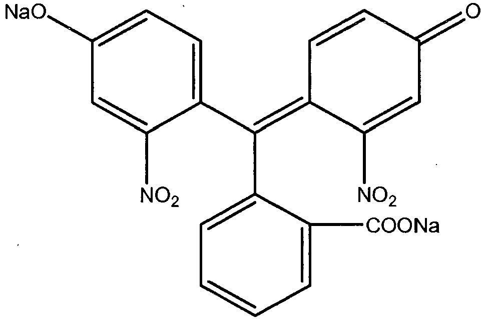

### EXAMPLE 4

### Synthesis of disodium salt of 3,3-bis-(4-hydroxy-3-nitrophenyl)-1-(3H)-isobenzofuranone

A mixture of 3,3-bis-(4-hydroxy-3-nitrophenyl)-1-(3H)-isobenzofuranone (0.01M) in ethanol (50mL, 85%) was stirred followed by addition of sodium hydroxide (0.02M) in ethanol (50mL, 85%). The reaction mixture was stirred and refluxed for 2 hours, cooled to room temperature. The solvent was evaporated on rotary evaporator, isolated the crude product and dried. Recrystallization from ethanol furnished pure disodium salt in 91 % yield.

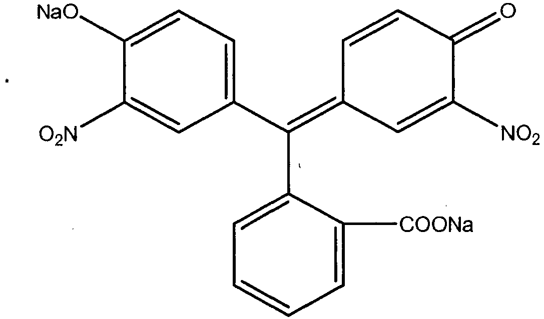

### EXAMPLE 5

### Synthesis of disodium salt of 3,3-bis-[4-hydroxy-2-(N,N-diethylamino)phenyl]-1-(3H)-isobenzofuranone

A mixture of 3,3-bis-[4-hydroxy-2-(N,N-diethylamino)phenyl]-1-(3H)-isobenzofuranone (0.01M) in ethanol (50mL, 85%) was stirred followed by addition of sodium hydroxide (0.02M) in ethanol (50mL, 85%). The reaction mixture was stirred and refluxed for 2 hours, cooled to room temperature. The solvent was evaporated on rotary evaporator, isolated the crude product and dried. Recrystallization from ethanol furnished pure, disodium salt in 89% yield.

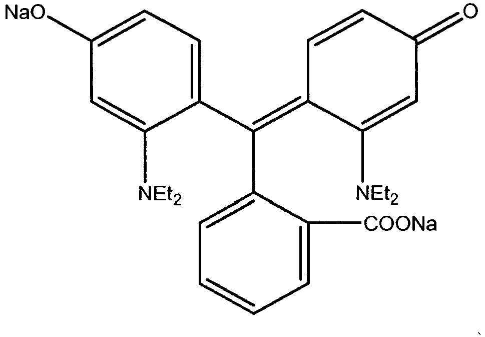

### EXAMPLE 6

### Synthesis of disodium salt of 3,3-bis-(4-hydroxy-3-ethylphenyl)-1-(3H)-isobenzofuranone

A mixture of 3,3-bis-(4-hydroxy-3-ethylphenyl)-1-(3H)-isobenzofuranone (0.01M) in ethanol (50mL, 85%) was stirred followed by addition of sodium hydroxide (0.02M) in ethanol (50mL, 85%). The reaction mixture was stirred and refluxed for 2 hours, cooled to room temperature. The solvent was evaporated on rotary evaporator, isolated the crude product and dried. Recrystallization from ethanol furnished pure disodium salt in 97% yield.

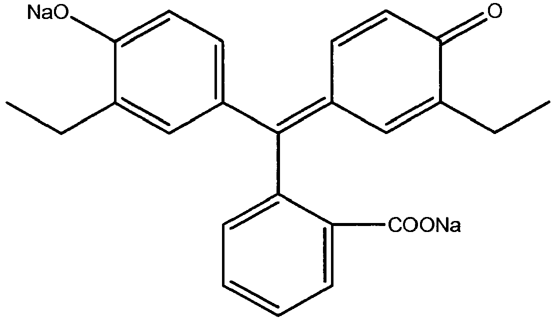

### EXAMPLE 7

### Synthesis of disodium salt of 3,3-bis-(4-hydroxy-3-ethoxyphenyl)-1-(3H)-isobenzofuranone

A mixture of 3,3-bis-(4-hydroxy-3-ethoxyphenyl)-1-(3H)-isobenzofuranone (0.01M) in ethanol (50mL, 85%) was stirred followed by addition of sodium hydroxide (0.02M) in ethanol (50mL, 85%). The reaction mixture was stirred and refluxed for 2 hours, cooled to room temperature. The solvent was evaporated on rotary evaporator, isolated the crude product and dried. Recrystallization from ethanol furnished pure disodium salt in 94% yield.

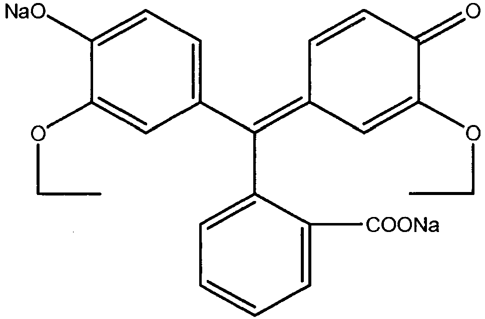

### EXAMPLE 8

### Synthesis of disodium salt of 3,3-bis-(4-hydroxy-3-acetamidophenyl)-1-(3H)-isobenzofuranone

A mixture of 3,3-bis-(4-hydroxy-3-acetamidophenyl)-1-(3H)-isobenzofuranone (0.01M) in ethanol (50mL, 85%) was stirred followed by addition of sodium hydroxide (0.02M) in ethanol (50mL, 85%). The reaction mixture was stirred and refluxed for 2 hours, cooled to room temperature. The solvent was evaporated on rotary evaporator, isolated the crude product and dried. Recrystallization from ethanol furnished pure disodium salt in 92% yield.

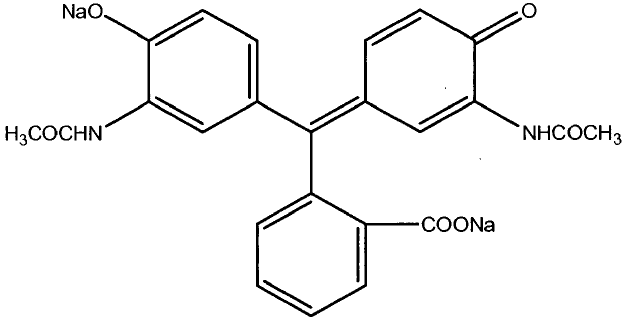

### EXAMPLE 9

### Synthesis of disodium salt of 3,3-bis-(4-hydroxy-6-methyl-3-nitrophenyl)-1-(3H)-isobenzofuranone

A mixture of 3,3-bis-(4-hydroxy-6-methyl-3-nitrophenyl)-1-(3H)-isobenzofuranone (0.01M) in ethanol (50mL, 85%) was stirred followed by addition of sodium hydroxide (0.02M) in ethanol (50mL, 85%). The reaction mixture was stirred and refluxed for 2 hours, cooled to room temperature. The solvent was evaporated on rotary evaporator, isolated the crude product and dried. Recrystallization from ethanol furnished pure disodium salt in 97% yield.

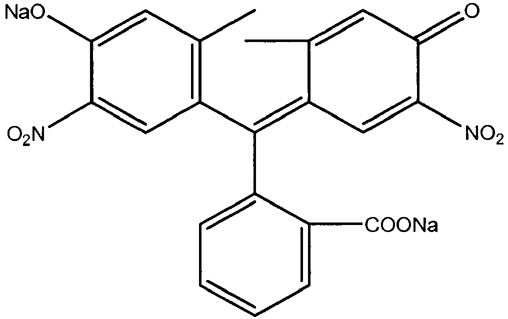

### EXAMPLE 10

### Synthesis of disodium salt of 3,3-bis-(4-hydroxy-6-methyl-5-quinolin-1-yl)-1-(3H)-isobenzofuranone

A mixture of 3,3-bis-(4-hydroxy-6-methyl-5-quinolin-1-yl)-1-(3H)-isobenzofuranone (0.01M) in ethanol (50mL, 85%) was stirred followed by addition of sodium hydroxide (0.02M) in ethanol (50mL, 85%). The reaction mixture was stirred and refluxed for 2 hours, cooled to room temperature. The solvent was evaporated on rotary evaporator, isolated the crude product and dried. Recrystallization from ethanol furnished pure disodium salt in 94% yield.

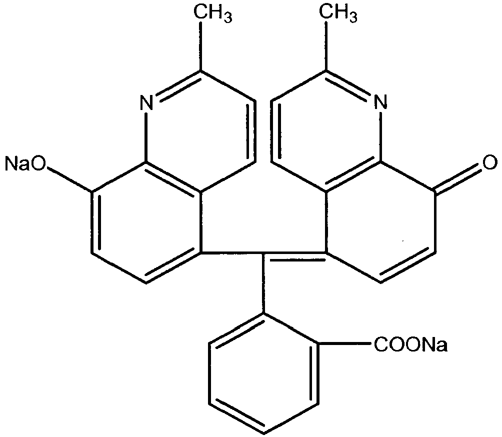

### EXAMPLE 11

### Synthesis of disodium salt of 3,3-bis-(4-hydroxy-3-pyridin-1-yl)-1-(3H)-isobenzofuranone

A mixture of 3,3-bis-(4-hydroxy-3-pyridin-1-yl)-1-(3H)-isobenzofuranone (0.01M) in ethanol (50mL, 85%) was stirred followed by addition of sodium hydroxide (0.02M) in ethanol (50mL, 85%). The reaction mixture was stirred and refluxed for 2 hours, cooled to room temperature. The solvent was evaporated on rotary evaporator, isolated the crude product and dried. Recrystallization from ethanol furnished pure disodium salt in 81 % yield.

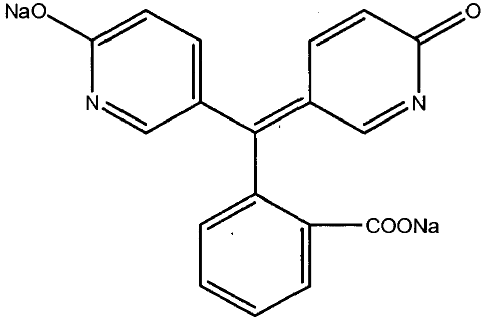

### EXAMPLE 12

### Synthesis of disodium salt of 3,3-bis-(4-hydroxy-2-pyridin-1-yl)-1-(3H)-isobenzofuranone

A mixture of 3,3-bis-(4-hydroxy-2-pyridin-1-yl)-1-(3H)-isobenzofuranone (0.01M) in ethanol (50mL, 85%) was stirred followed by addition of sodium hydroxide (0.02M) in ethanol (50mL, 85%). The reaction mixture was stirred and refluxed for 2 hours, cooled to room temperature. The solvent was evaporated on rotary evaporator, isolated the crude product and dried. Recrystallization from ethanol furnished pure disodium salt in 84% yield.

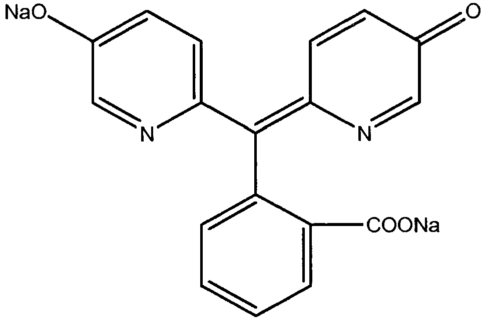

### EXAMPLE 13

### Synthesis of disodium salt of 3,3-bis-(4-hydroxy-3-phenylphenyl)-1-(3H)-isobenzofuranone

A mixture of 3,3-bis-(4-hydroxy-3-phenylphenyl)-1-(3H)-isobenzofuranone (0.01mol) in ethanol (50mL, 85%) was stirred followed by addition of sodium hydroxide (0.02mol) in ethanol (50mL, 85%). The reaction mixture was stirred and refluxed for 2 hours, cooled to room temperature. The solvent was evaporated on rotary evaporator, isolated the crude product and dried. Recrystallization from ethanol furnished pure disodium salt in 96% yield. ¹H-NMR (DMSO-d₆, 300MHz): δ 6.25-6.74 (m, 6H, aromatic), 6.88-7.45 (m, 10H, aromatic), 7.53-7.84 (m, 4H, aromatic) ppm. Mass spectra: m/z 514 (M⁺).

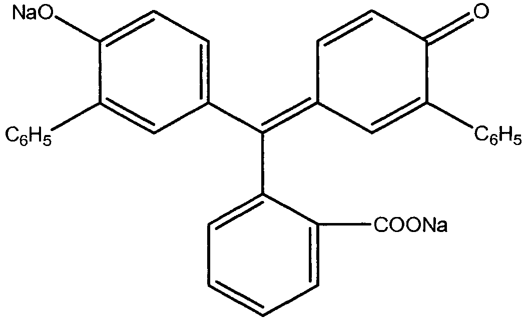

### EXAMPLE 14

### Synthesis of disodium salt of 3,3-bis-(4-hydroxy-3,5-diisopropylphenyl)-1-(3H)-isobenzofuranone

A mixture of 3,3-bis-(4-hydroxy-3,5-diisopropylphenyl)-1-(3H)-isobenzofuranone (0.01mol) in ethanol (50mL, 85%) was stirred followed by addition of sodium hydroxide (0.02mol) in ethanol (50mL, 85%). The reaction mixture was stirred and refluxed for 2 hours, cooled to room temperature. The solvent was evaporated on rotary evaporator, isolated the crude product and dried. Recrystallization from ethanol furnished pure disodium salt in 92% yield. ¹H-NMR (DMSO-d₆): δ 1.00-1.21 (dd, 24H, 8CH₃), 3.06-3.36 (heptate, 4H, 4CH), 6.74-6.96 (m, 4H, aromatic), 7.05-7.83 (m, 4H, aromatic) ppm. Mass spectra: m/z 530 (M⁺).

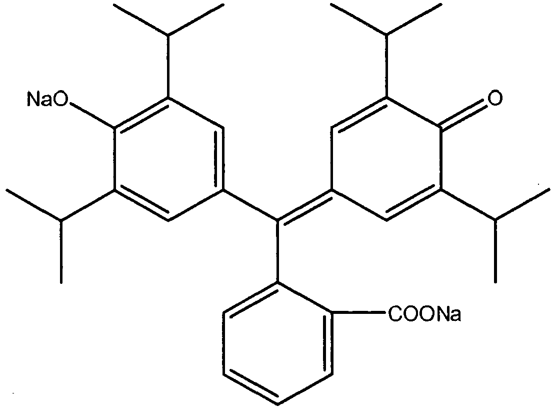

### EXAMPLE 15

### Synthesis of disodium salt of 3,3-bis-(4-hydroxy-3,5-dimethoxyphenyl)-1-(3H)-isobenzofuranone

A mixture of 3,3-bis-(4-hydroxy-3,5-dimethoxyphenyl)-1-(3H)-isobenzofuranone (0.01 mol) in ethanol (50mL, 85%) was stirred followed by addition of sodium hydroxide (0.02mol) in ethanol (50mL, 85%). The reaction mixture was stirred and refluxed for 2 hours, cooled to room temperature. The solvent was evaporated on rotary evaporator, isolated the crude product and dried. Recrystallization from ethanol furnished pure disodium salt in 90% yield. ¹H-NMR (DMSO-d₆): δ 3.61 (s, 12H, 40CH₃), 6.45-6.52 (m, 4H, aromatic), 7.04-7.78 (m, 4H, aromatic) ppm. Mass spectra: m/z 482 (M⁺).

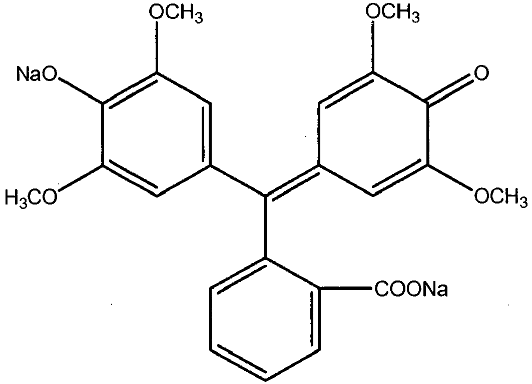

### EXAMPLE 16

### Synthesis of disodium salt of 3,3-bis-(4-hydroxy-3,5-dimethylphenyl)-1-(3H)-isobenzofuranone

A mixture of 3,3-bis-(4-hydroxy-3,5-dimethylphenyl)-1-(3H)-isobenzofuranone (0.01 mol) in ethanol (50mL, 85%) was stirred followed by addition of sodium hydroxide (0.02mol) in ethanol (50mL, 85%). The reaction mixture was stirred and refluxed for 2 hours, cooled to room temperature. The solvent was evaporated on rotary evaporator, isolated the crude product and dried. Recrystallization from ethanol furnished pure disodium salt in 95% yield. ¹H-NMR (DMSO-d₆): δ 2.11 (s, 12H, 4CH₃), 6.81-6.87 (m, 4H, aromatic), 7.23-7.84 (m, 4H, aromatic) ppm. Mass spectra: m/z 418 (M⁺).

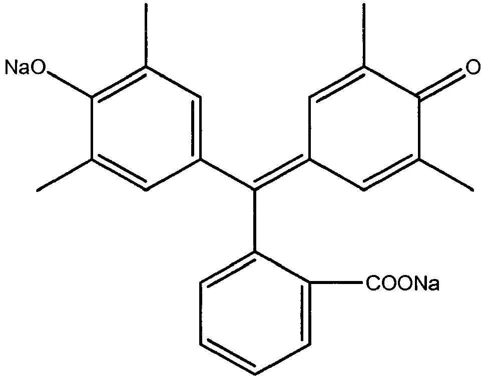

### EXAMPLE 17

### Synthesis of disodium salt of 3,3-bis-(4-hydroxy-3,6-diimethylphenyl)-1-(3H)-isobenzofuranone

A mixture of 3,3-bis-(4-hydroxy-3,6-diimethylphenyl)-1-(3H)-isobenzofuranone (0.01mol) in ethanol (50mL, 85%) was stirred followed by addition of sodium hydroxide (0.02mol) in ethanol (50mL, 85%). The reaction mixture was stirred and refluxed for 2 hours, cooled to room temperature. The solvent was evaporated on rotary evaporator, isolated the crude product and dried. Recrystallization from ethanol furnished pure disodium salt in 88% yield. ¹H-NMR (DMSO-d₆): δ 2.01 (s, 12H, 4CH₃), 6.04-6.82 (m, 4H, aromatic), 7.10-7.72 (m, 4H, aromatic) ppm. Mass spectra: m/z 418 (M⁺).

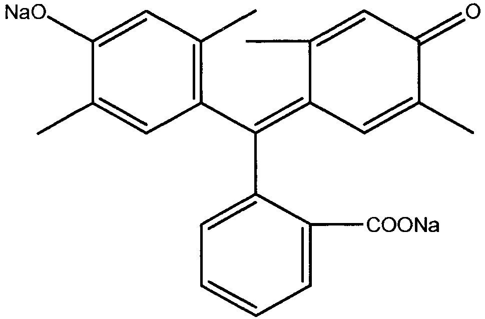

### EXAMPLE 18

### Synthesis of disodium salt of 3,3-bis-(4-hydroxy-3-ethylphenyl)-1-(3H)-isobenzofuranone

A mixture of 3,3-bis-(4-hydroxy-3-ethylphenyl)-1-(3H)-isobenzofuranone (0.01mol) in ethanol (50mL, 85%) was stirred followed by addition of sodium hydroxide (0.02mol) in ethanol (50mL, 85%). The reaction mixture was stirred and refluxed for 2 hours, cooled to room temperature. The solvent was evaporated on rotary evaporator, isolated the crude product and dried. Recrystallization from ethanol furnished pure disodium salt in 86% yield. ¹H-NMR (DMSO-d₆): δ 2.30-2.51 (q, 4H, 2CH₂), 1.00-1.10 (t, 6H, 2CH₃), 6.20-6.75 (m, 6H, aromatic), 7.12-7.84 (m, 4H, aromatic) ppm. Mass spectra: m/z 418 (M⁺).

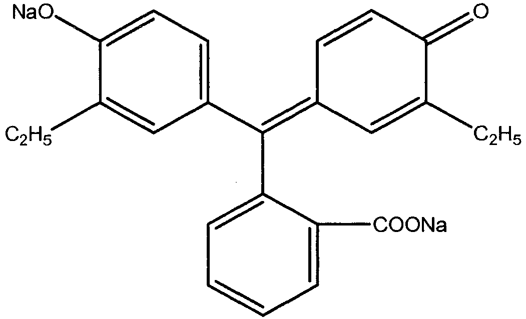

### EXAMPLE 19

### Synthesis of disodium salt of 3,3-bis-(4-hydroxy-3-isopropylphenyl)-1-(3H)-isobenzofuranone

A mixture of 3,3-bis-(4-hydroxy-3-isopropylphenyl)-1-(3H)-isobenzofuranone (0.01mol) in ethanol (50mL, 85%) was stirred followed by addition of sodium hydroxide (0.02mol) in ethanol (50mL, 85%). The reaction mixture was stirred and refluxed for 2 hours, cooled to room temperature. The solvent was evaporated on rotary evaporator, isolated the crude product and dried. Recrystallization from ethanol furnished pure disodium salt in 81% yield. ¹H-NMR (DMSO-d₆): δ 1.02-1.14 (dd, 12H, 4CH₃), 3.12-3.45 (heptate, 2H, 2CH), 6.32-6.76 (m, 6H, aromatic), 7.30-7.83 (m, 4H, aromatic) ppm. Mass spectra: m/z 446 (M⁺).

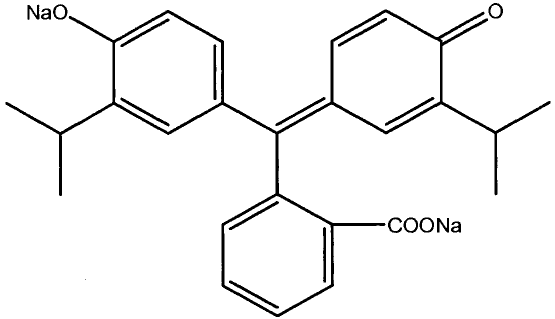

### EXAMPLE 20

### Synthesis of disodium salt of 3,3-bis-(4-hydroxy-3-methoxyphenyl)-1-(3H)-isobenzofuranone

A mixture of 3,3-bis-(4-hydroxy-3-methoxyphenyl)-1-(3H)-isobenzofuranone (0.01mol) in ethanol (50mL, 85%) was stirred followed by addition of sodium hydroxide (0.02mol) in ethanol (50mL, 85%). The reaction mixture was stirred and refluxed for 2 hours, cooled to room temperature. The solvent was evaporated on rotary evaporator, isolated the crude product and dried. Recrystallization from ethanol furnished pure disodium salt in 88% yield.

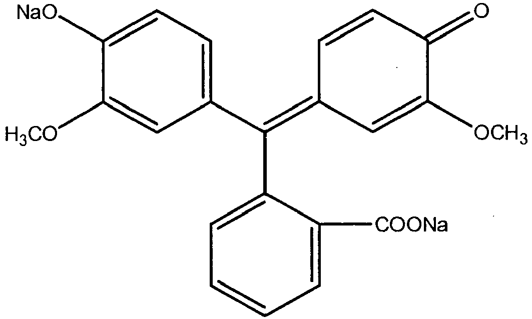

### EXAMPLE 21

### Synthesis of disodium salt of 3,3-bis-(4-hydroxy-2,3,5-trimethylphenyl)-1-(3H)-isobenzofuranone

A mixture of 3,3-bis-(4-hydroxy-2,3,5-trimethylphenyl)-1-(3H)-isobenzofuranone (0.01mol) in ethanol (50mL, 85%) was stirred followed by addition of sodium hydroxide (0.02mol) in ethanol (50mL, 85%). The reaction mixture was stirred and refluxed for 2 hours, cooled to room temperature. The solvent was evaporated on rotary evaporator, isolated the crude product and dried. Recrystallization from ethanol furnished pure disodium salt in 80% yield.

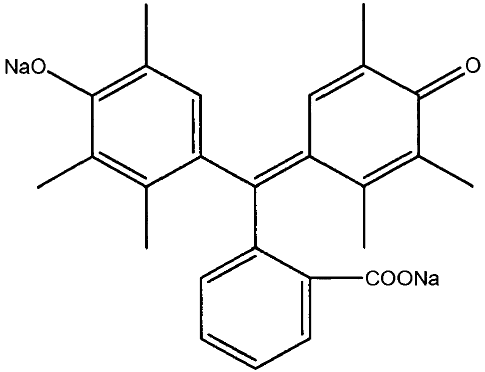

### EXAMPLE 22

### Synthesis of disodium salt of 3,3-bis-(4-hydroxy-3-sec-butylphenyl)-1-(3H)-isobenzofuranone

A mixture of 3,3-bis-(4-hydroxy-3-sec-butylphenyl)-1-(3H)-isobenzofuranone (0.01mol) in ethanol (50mL, 85%) was stirred followed by addition of sodium hydroxide (0.02mol) in ethanol (50mL, 85%). The reaction mixture was stirred and refluxed for 2 hours, cooled to room temperature. The solvent was evaporated on rotary evaporator, isolated the crude product and dried. Recrystallization from ethanol furnished pure disodium salt in 82% yield. ¹H-NMR (DMSO-d₆): δ 0.80 (t, 6H, 2CH₃), 1.29-1.37 (p, 4H, 2CH₂), 1.20 (d, 6H, 2CH₃), 2.88-2.96 (sextate, 2H, 2CH), 6.08-6.75 (m, 6H, aromatic), 7.37-7.81 (m, 4H, aromatic) ppm.

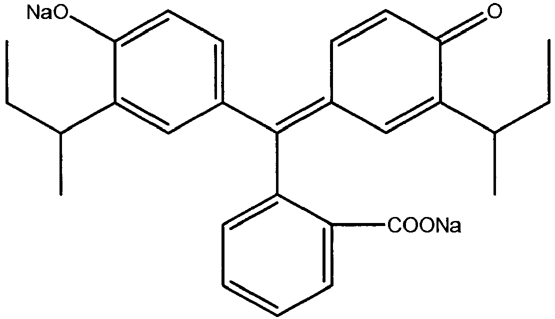

### EXAMPLE 23

### Synthesis of disodium salt of 3,3-bis-(4-hydroxy-3-nitrophenyl)-1-(3H)-isobenzofuranone

A mixture of 3,3-bis-(4-hydroxy-3-nitrophenyl)-1-(3H)-isobenzofuranone (0.01mol) in ethanol (50mL, 85%) was stirred followed by addition of sodium hydroxide (0.02mol) in ethanol (50mL, 85%). The reaction mixture was stirred and refluxed for 2 hours, cooled to room temperature. The solvent was evaporated on rotary evaporator, isolated the crude product and dried. Recrystallization from ethanol furnished pure disodium salt in 92% yield.

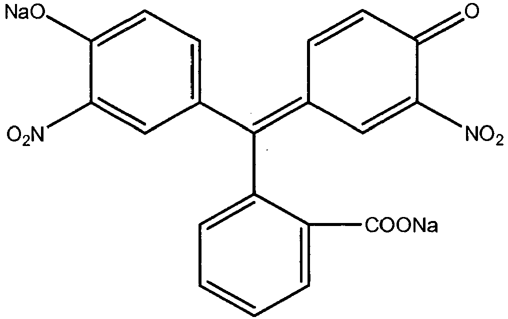

### Synthesis of Phenol and Hydrazine Acid-Base Indicators

### EXAMPLE 1

### Synthesis of sodium salt of 5-methyl-2-nitrophenol

A mixture of 5-methyl-2-nitrophenol (0.1M) in ethanol (25mL, 85%) was stirred followed by addition of sodium hydroxide (0.1M) in ethanol (25mL, 85%).

The reaction mixture was stirred at room temperature for 2 hours. The separated golden yellow solid was filtered, washed with ethanol and dried. Recrystallization from ethanol furnished pure sodium salt in 96% yield.

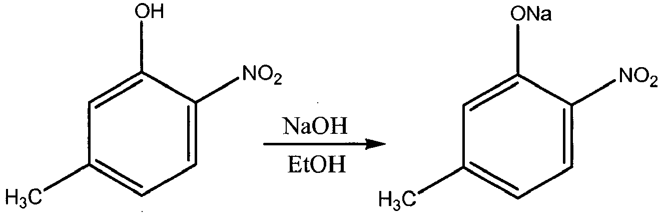

### EXAMPLE 2

### Synthesis of p-nitrobenzhydrazide

A mixture of ethyl p-nitrobenzoate (0.1M), hydrazine hydrate (0.11M) in ethanol (100mL) was stirred at room temperature for 2 hours. The separated pale yellow solid was filtered, washed with ethanol and dried. Recrystallization from ethanol furnished pure hydrazide in 88% yield.

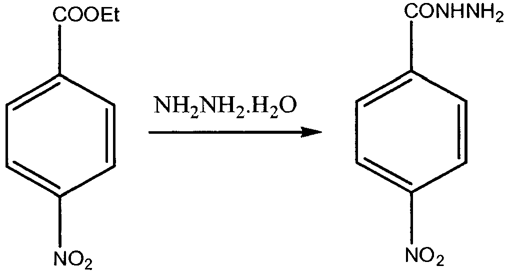

### EXAMPLE 3

### Synthesis of hydrazide

A mixture of ethyl salicylate (0.1 M), 2,4-dinitrophenylhydrazine (0.1 M) in ethanol (150mL) was stirred at room temperature for 2 hours. The separated orange solid was filtered, washed with ethanol and dried. Recrystallization from ethanol furnished pure hydrazide in 94% yield.

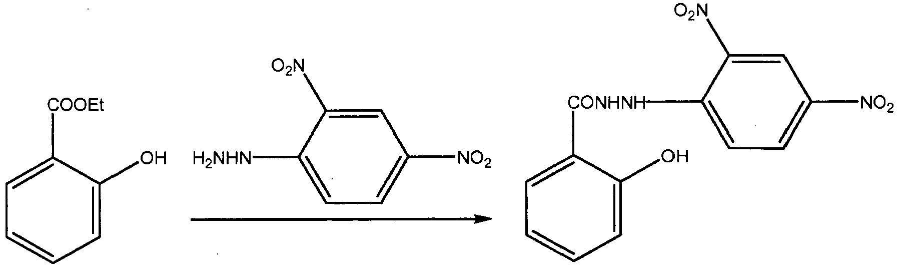

### EXAMPLE 4

### Synthesis of sodium salt of hydrazide

A mixture of hydrazide (0.1M) in ethanol (25mL) was stirred followed by addition of sodium ethoxide (0.1M) in ethanol (25mL). The reaction mixture was stirred and refluxed at for 4 hours, cooled to room temperature. The solvent was evaporated on rotary evaporator, isolated the crude yellow product and dried. Recrystallization from ethanol furnished pure sodium salt in 92% yield.

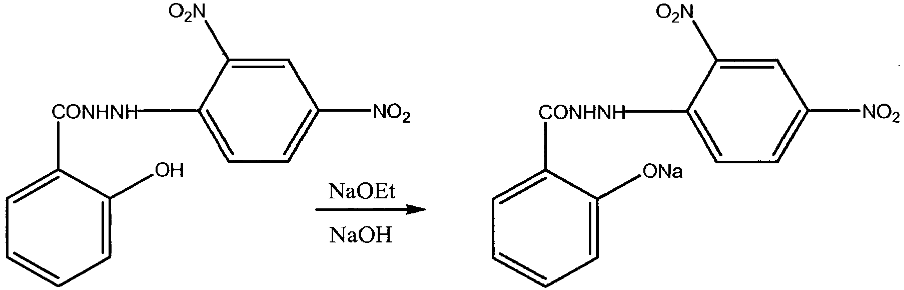

### EXAMPLE 5

### Synthesis of hydrazide

A mixture of ethyl salicylate (0.1M), 4-nitrophenylhydrazine (0.1M) in ethanol (150mL) was stirred at room temperature for 2 hours. The separated yellow solid was filtered, washed with ethanol and dried. Recrystallization from ethanol furnished pure hydrazide in 89% yield.

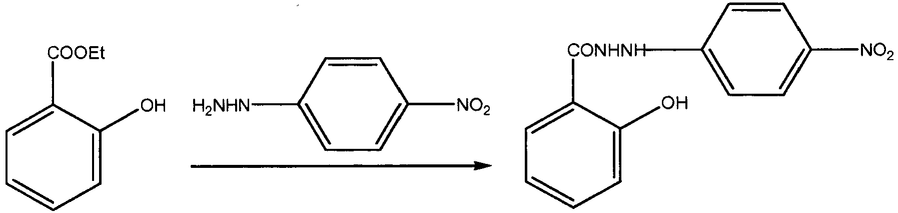

### EXAMPLE 6

### Synthesis of sodium salt of hydrazide

A mixture of hydrazide (0.1M) in ethanol (25mL) was stirred followed by addition of sodium ethoxide (0.1M) in ethanol (25mL). The reaction mixture was stirred and refluxed at for 4 hours, cooled to room temperature. The solvent was evaporated on rotary evaporator, isolated the crude yellow product and dried. Recrystallization from ethanol furnished pure sodium salt in 84% yield.

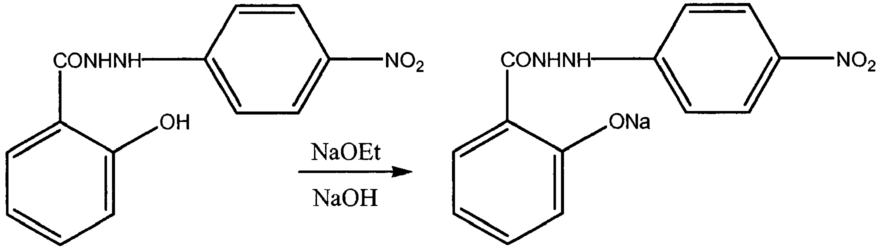

### 1.0 Color Disappearing/Color Changing Bubbles and Compositions

### 1.1 Color Disappearing Bubbles with Acid-Base Indicator and Base

### EXAMPLE 1

| **Chemical Component** | **Weight in grams** |
|---|---|
| Glycerin | 10 |
| Phenolphthalein (Na salt) | 2 |
| Methocel A4M (2% solution in water) | 6 |
| Colonial SLS | 14 |
| Deionized water | 67 |
| Triethanolamine | 0.8 |
| Liquid Germall Plus | 0.2 |

Glycerin, phenolphthalein are stirred and heated at 50°C for 30 minutes, cooled to room temperature, followed by addition of deionized water, methocel A4M, colonial SLS, triethanolamine & liquid germall plus. The reaction mixture was further stirred for 2 hours at room temperature.

### EXAMPLE 2

| **Chemical Component** | **Weight in grams** |
|---|---|
| Glycerin | 10 |
| o-Cresolphthalein (Na salt) | 2 |
| Methocel A4M (2% solution in water) | 6 |
| Colonial SLS | 14 |
| Deionized water | 67 |
| Triethanolamine | 0.8 |
| Liquid Germall Plus | 0.2 |

Glycerin, o-cresolphthalein are stirred and heated at 50°C for 30 minutes, cooled to room temperature, followed by addition of deionized water, Methocel A4M, Colonial SLS, triethanolamine & Liquid Germall Plus. The reaction mixture was further stirred for 2 hours at room temperature.

### EXAMPLE 3

| **Chemical Component** | **Weight in grams** |
|---|---|
| Glycerin | 10 |
| Thymolphthalein (Na salt) | 2 |
| Methocel A4M (2% solution in water) | 6 |
| Colonial SLS | 14 |
| Deionized water | 67 |
| Triethanolamine | 0.8 |
| Liquid Germall Plus | 0.2 |

Glycerin, thymolphthalein are stirred and heated at 50°C for 30 minutes, cooled to room temperature, followed by addition of deionized water, Methocel A4M, Colonial SLS, triethanolamine & Liquid Germall Plus. The reaction mixture was further stirred for 2 hours at room temperature.

### EXAMPLE 4

| **Chemical Component** | **Weight in grams** |
|---|---|
| Glycerin | 5 |
| Phenolphthalein (Na salt) | 2 |
| Colamid C | 4 |
| Miracare BC-27 | 7 |
| Deionized water | 81 |
| Triethanolamine | 0.8 |
| Liquid Germall Plus | 0.2 |

Glycerin, phenolphthalein, Colamid C, Miracare BC-27 are stirred and heated at 50°C for 30 minutes, cooled to room temperature, followed by addition of deionized water, triethanolamine & liquid germall plus. The reaction mixture was further stirred for 2 hours at room temperature.

### EXAMPLE 5

| **Chemical Component** | **Weight in grams** |
|---|---|
| Glycerin | 5 |
| o-Cresolphthalein (Na salt) | 2 |
| Colamid C | 4 |
| Miracare BC-27 | 7 |
| Deionized water | 81 |
| Triethanolamine | 0.8 |
| Liquid Germall Plus | 0.2 |

Glycerin, o-cresolphthalein, Colamid C, Miracare BC-27 are stirred and heated at 50°C for 30 minutes, cooled to room temperature, followed by addition of deionized water, triethanolamine & liquid germall plus. The reaction mixture was further stirred for 2 hours at room temperature.

### EXAMPLE 6

| **Chemical Component** | **Weight in grams** |
|---|---|
| Glycerin | 5 |
| Thymolphthalein (Na salt) | 2 |
| Colamid C | 4 |
| Miracare BC-27 | 7 |
| Deionized water | 81 |
| Triethanolamine | 0.8 |
| Liquid Germall Plus | 0.2 |

Glycerin, thymolphthalein, Colamid C, Miracare BC-27 are stirred and heated at 50°C for 30 minutes, cooled to room temperature, followed by addition of deionized water, triethanolamine & liquid germall plus. The reaction mixture was further stirred for 2 hours at room temperature.

Table 1 shows the color change observed in colored bubble solution in various Examples 1 through 6 as given below:

**Table 1: Color change in colored bubble solution**

| **Example #** | **Color change observed** |
|---|---|
| Example 1 | Pink to Colorless |
| Example 2 | Red to Colorless |
| Example 3 | Blue to Colorless |
| Example 4 | Pink to Colorless |
| Example 5 | Red to Colorless |
| Example 6 | Blue to Colorless |

### 1.2 Film Formed Color Disappearing Bubbles with Acid-Base Indicator and Base

### EXAMPLE 1

| **Chemical Component** | **Weight in grams** |
|---|---|
| Triethanolamine | 10 |
| Phenolphthalein (Na salt) | 5 |
| Methocel A4M (4% solution in water) | 2.5 |
| Colonial SLS | 2.5 |
| Celvol 508 (20% Solution) | 60 |
| Deionized water | 19.8 |
| Liquid Germall Plus | 0.2 |

Triethanolamine, phenolphthalein are stirred and heated at 50°C for 30 minutes, cooled to room temperature, followed by addition of deionized water, methocel A4M, colonial SLS, celvol 508 & liquid germall plus. The reaction mixture was further stirred for 2 hours at room temperature.

### EXAMPLE 2

| **Chemical Component** | **Weight in grams** |
|---|---|
| Triethanolamine | 10 |
| o-Cresolphthalein (Na salt) | 5 |
| Methocel A4M (4% solution in water) | 2.5 |
| Colonial SLS | 2.5 |
| Celvol 508 (20% Solution) | 60 |
| Deionized water | 19.8 |
| Liquid Germall Plus | 0.2 |

Triethanolamine, o-cresolphthalein are stirred and heated at 50°C for 30 minutes, cooled to room temperature, followed by addition of deionized water, methocel A4M, colonial SLS, celvol 508 & liquid germall plus. The reaction mixture was further stirred for 2 hours at room temperature.

### EXAMPLE 3

| **Chemical Component** | **Weight in grams** |
|---|---|
| Triethanolamine | 10 |
| Thymolphthalein (Na salt) | 5 |
| Methocel A4M (4% solution in water) | 2.5 |
| Colonial SLS | 2.5 |
| Celvol 508 (20% Solution) | 60 |
| Deionized water | 19.8 |
| Liquid Germall Plus | 0.2 |

Triethanolamine, thymolphthalein are stirred and heated at 50°C for 30 minutes, cooled to room temperature, followed by addition of deionized water, methocel A4M, colonial SLS, celvol 508 & liquid germall plus. The reaction mixture was further stirred for 2 hours at room temperature.

### EXAMPLE 4

| **Chemical Component** | **Weight in grams** |
|---|---|
| Triethanolamine | 10 |
| Phenolphthalein (Na salt) | 5 |
| Colamid C | 1.5 |
| Miracare BC-27 | 3.5 |
| Celvol 508 (20% Solution) | 60 |
| Deionized water | 19.8 |
| Liquid Germall Plus | 0.2 |

Triethanolamine, phenolphthalein, Colamid C, Miracare BC-27 are stirred and heated at 50°C for 30 minutes, cooled to room temperature, followed by addition of deionized water, celvol 508 & liquid germall plus. The reaction mixture was further stirred for 2 hours at room temperature.

### EXAMPLE 5

| **Chemical Component** | **Weight in grams** |
|---|---|
| Triethanolamine | 10 |
| o-Cresolphthalein (Na salt) | 5 |
| Colamid C | 1.5 |
| Miracare BC-27 | 3.5 |
| Celvol 508 (20% Solution) | 60 |
| Deionized water | 19.8 |
| Liquid Germall Plus | 0.2 |

Triethanolamine, o-cresolphthalein, Colamid C, Miracare BC-27 are stirred and heated at 50°C for 30 minutes, cooled to room temperature, followed by addition of deionized water, celvol 508 & liquid germall plus. The reaction mixture was further stirred for 2 hours at room temperature.

### EXAMPLE 6

| **Chemical Component** | **Weight in grams** |
|---|---|
| Triethanolamine | 10 |
| Thymolphthalein (Na salt) | 5 |
| Colamid C | 1.5 |
| Miracare BC-27 | 3.5 |
| Celvol 508 (20% Solution) | 60 |
| Deionized water | 19.8 |
| Liquid Germall Plus | 0.2 |

Triethanolamine, thymolphthalein, Colamid C, Miracare BC-27 are stirred and heated at 50°C for 30 minutes, cooled to room temperature, followed by addition of deionized water, celvol 508 & liquid germall plus. The reaction mixture was further stirred for 2 hours at room temperature.

Table 1 shows the color change observed in colored bubble solution in various Examples 1 through 6 as given below:

**Table 1: Color change in colored bubble solution**

| **Example #** | **Color change observed** |
|---|---|
| Example 1 | Pink to Colorless |
| Example 2 | Red to Colorless |
| Example 3 | Blue to Colorless |
| Example 4 | Pink to Colorless |
| Example 5 | Red to Colorless |
| Example 6 | Blue to Colorless |

## Claims

1. A substantially uniformly colored bubble composition comprising:
a surfactant;
water; and
an acid-base indicator comprising: 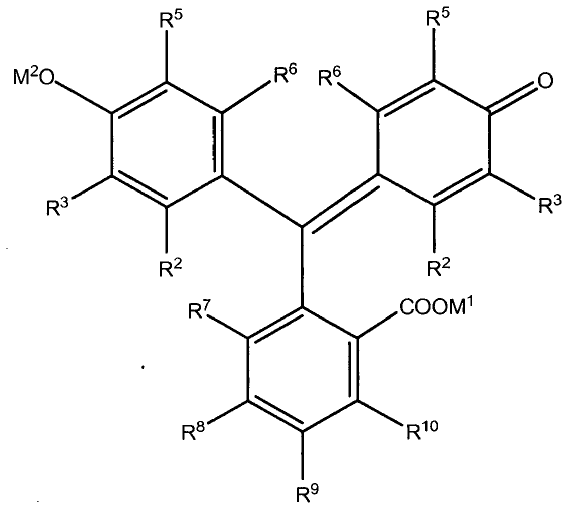 wherein
R² is selected from the group consisting of hydrogen, nitro, amino and alkyl;
R³ is selected from the group consisting of hydrogen, aryl, alkyl, nitro, acetamido and alkoxy;
R⁵ is selected from the group consisting of hydrogen, halo, alkoxy and alkyl;
R⁶ is selected from the group consisting of hydrogen and alkyl;
R⁷, R⁸, R⁹ and R¹⁰ are all hydrogen;
optionally, one of the carbons connected to R², R³, R⁵ or R⁶ can be substituted with a nitrogen atom; and
M¹ and M² are each independently a hydrogen atom, a metal ion or an ammonium ion, provided that at least one of M¹ or M² is a metal ion or an ammonium ion.

2. The substantially uniformly colored bubble composition of claim 1, wherein R² is selected from the group consisting of hydrogen and methyl; R³ is selected from the group consisting of hydrogen, phenyl, isopropyl, methyl, ethyl, sec-butyl, nitro and methoxy; R⁵ is selected from the group consisting of hydrogen, bromo, methoxy, isopropyl and methyl; and R⁶ is selected from the group consisting of hydrogen and methyl.

3. The substantially uniformly colored bubble composition of claim 1, wherein R², R³, R⁵, R⁶, R⁷, R⁸, R⁹ and R¹⁰ are all hydrogen atoms, or R² is hydrogen, R³ is Me, and R⁵, R⁶, R⁷, R⁸, R⁹ and R¹⁰ are hydrogen atoms, or R² is Me, R³ is a hydrogen atom, R⁵ is an iso-propyl group, R⁶, R⁷, R⁸, R⁹ and R¹⁰ are hydrogen atoms or R² is H, R³ is Me, R⁵ is Br and R ⁶ R⁷, R⁸, R⁹ and R¹⁰ are hydrogen atoms or R² is Me, R³ is Br, R⁵ is an isopropyl and R⁶, R⁷, R⁸, R⁹ and R¹⁰ are hydrogen atoms.

4. The substantially uniformly colored bubble composition of claim 1, wherein R² is H, R³ is phenyl and R⁵, R⁶, R⁷, R⁸, R⁹ and R¹⁰ are all hydrogen atoms, or R² is H, R³ and R⁵ are isopropyl and R⁶, R⁷, R⁸, R⁹ and R¹⁰ are all hydrogen atoms, or R² is H, R³ is methyl, R⁵ is H, R⁶ is methyl, R⁷, R⁸, R⁹ and R¹⁰ are all hydrogen atoms, or R² is H, R³ and R⁵ are methoxy and R⁶, R⁷, R⁸, R⁹ and R¹⁰ are all hydrogen atoms, or R² is H, R³ and R⁵ are methyl and R⁶, R⁷, R⁸, ^{R9} and R¹⁰ are all hydrogen atoms, or R² is H, R³ is ethyl and R⁵, R⁶, R⁷, R8, R⁹ and R¹⁰ are all hydrogen atoms, or R² is H, R³ is isopropyl and R⁵, R⁶, R⁷, R⁸ R⁹ and R'° are all hydrogen atoms, or R² is H, R³ is methoxide and R⁵, R⁶, R⁷, R⁸, R⁹ and R¹⁰ are all hydrogen atoms or R², R³ and R⁵ are all methyl and R⁶, R⁷, R⁸, R⁹ and R¹⁰ are all hydrogen atoms, or R², R⁵, R⁶, R⁷, R⁸, R⁹, R¹⁰ are all hydrogen atoms and R³ is sec-butyl, or R², R⁵, R⁶, R⁷, R⁸, R⁹, R¹⁰ are all hydrogen atoms and R³ is nitro.

5. The substantially uniformly colored bubble composition of any one of claims 1 to 4, further comprising a base.

6. The substantially uniformly colored bubble composition of claim 5, wherein the base is a metal hydroxide.

7. The substantially uniformly colored bubble composition of claim 1, wherein the surfactant is a cellulosic ether.

8. The substantially uniformly colored bubble composition of claim 1, wherein the surfactant is a combination of lauryl sulphate, C10-C16 alkyl alcohols, sodium salts and C10-C16 alcohols.

## Patentansprüche

1. Im Wesentlichen gleichmäßig gefärbte Blasenzusammensetzung, die Folgendes umfasst:
einen oberflächenaktiven Stoff;
Wasser; und
einen Säure-Basen-Indikator, umfassend: 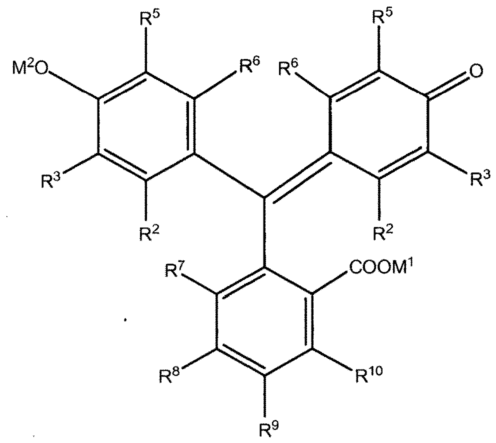 wobei
R² ausgewählt ist aus der Gruppe bestehend aus Wasserstoff, Nitro, Amino und Alkyl;
R³ ausgewählt ist aus der Gruppe bestehend aus Wasserstoff, Aryl, Alkyl, Nitro, Acetamino und Alkoxy;
R⁵ ausgewählt ist aus der Gruppe bestehend aus Wasserstoff, Halo, Alkoxy und Alkyl;
R⁶ ausgewählt ist aus der Gruppe bestehend aus Wasserstoff und Alkyl;
R⁷, R⁸⁷, R⁹ und R¹⁰ alle Wasserstoff sind;
optional einer der Kohlenstoffatome, die mit R², R³, R⁵ oder R⁶ zusammenhängen, mit einem Stickstoffatom substituiert werden können; und
M¹ und M² jeweils unabhängig ein Stickstoffatom, ein Metallion oder ein Ammoniumion sind, wenn wenigstens eines aus M¹ oder M² ein Metallion oder ein Ammoniumion ist.

2. Im Wesentlichen gleichförmig gefärbte Blasenzusammensetzung nach Anspruch 1, wobei R² ausgewählt ist aus der Gruppe bestehend aus Wasserstoff und Methyl; R³ ausgewählt ist aus der Gruppe bestehend aus Wasserstoff, Phenyl, Isopropyl, Methyl, Ethyl, sec-Butyl, Nitro und Methoxy; R⁵ ausgewählt ist aus der Gruppe bestehend aus Wasserstoff, Bromo, Methoxy, Isopropyl und Methyl; und R⁶ ausgewählt ist aus der Gruppe bestehend aus Wasserstoff und Methyl.

3. Im Wesentlichen gleichförmig gefärbte Blasenzusammensetzung nach Anspruch 1, wobei R², R³, R⁵, R⁶, R⁷, R⁸, R⁹ und R¹⁰ alle Wasserstoffatome sind, oder R² Wasserstoff ist, R³ Me ist, und R⁵, R⁶, R⁷, R⁸, R⁹ und R¹⁰ Wasserstoffatome sind, oder R² Me ist, R³ ein Wasserstoffatom ist, R⁵ eine Isopropylgruppe ist, R⁶, R⁷, R⁸, R⁹ und R¹⁰ alle Wasserstoffatome sind, oder R² H ist, R³ Me ist, R⁵ Br ist, und R⁶, R⁷, R⁸, R⁹ und R¹⁰ Wasserstoffatome sind, oder R² Me ist, R³ Br ist, R⁵ ein Isopropyl ist, und R⁶, R⁷, R⁸, R⁹ und R¹⁰ Wasserstoffatome sind.

4. Im Wesentlichen gleichförmig gefärbte Blasenzusammensetzung nach Anspruch 1, wobei R² H ist, R³ Phenyl ist, und R⁵, R⁶, R⁷, R⁸, R⁹ und R¹⁰ alle Wasserstoffatome sind, oder R² H ist, R³ und R⁵ Isopropyl sind, und R⁶, R⁷, R⁸, R⁹ und R¹⁰ alle Wasserstoffatome sind, oder R² H ist, R³ Methyl ist, R⁵ H ist, R⁶ Methyl ist, R⁷, R⁸, R⁹ und R¹⁰ alle Wasserstoffatome sind, oder R² H ist, R³ und R⁵ Methoxy sind, und R⁶, R⁷, R⁸, R⁹ und R¹⁰ alle Wasserstoffatome sind, oder R² H ist, R³ und R⁵ Methyl sind, und R⁶, R⁷, R⁸, R⁹ und R¹⁰ alle Wasserstoffatome sind, oder R² H ist, R³ Ethyl ist, und R⁵, R⁶, R⁷, R⁸, R⁹ und R¹⁰ alle Wasserstoffatome sind, oder R² H ist, R³ Isopropyl ist, und R⁵, R⁶, R⁷, R⁸, R⁹ und R¹⁰ alle Wasserstoffatome sind, oder R² H ist, R³ Methoxid ist, und R⁵, R⁶, R⁷, R⁸, R⁹ und R¹⁰ alle Wasserstoffatome sind, oder R², R³ und R⁵ alle Methyl sind, und R⁶, R⁷, R⁸, R⁹, R¹⁰ alle Wasserstoffatome sind, oder R², R⁵, R⁶, R⁷, R⁸, R⁹, R1° alle Wasserstoffatome sind, und R³ Sec-Butyl ist, oder R², R⁵, R⁶, R⁷ R⁸, R⁹, R¹⁰ alle Wasserstoffatome sind und R³ Nitro ist.

5. Im Wesentlichen gleichmäßig gefärbte Blasenzusammensetzung nach einem der Ansprüche 1 bis 4, ferner umfassend eine Base.

6. Im Wesentlichen gleichmäßig gefärbte Blasenzusammensetzung nach Anspruch 5, wobei die Base ein Metallhydroxid ist.

7. Im Wesentlichen gleichförmig gefärbte Blasenzusammensetzung nach Anspruch 1, wobei der oberflächenaktive Stoff ein Celluloseether ist.

8. Im Wesentlichen gleichförmig gefärbte Blasenzusammensetzung nach Anspruch 1, wobei der oberflächenaktive Stoff eine Kombination aus Laurylsulfat, C10-C16-Akylalkoholen, Natriumsalzen und C10-C16 Alkoholen ist.

## Revendications

1. Composition de bulles sensiblement uniformément colorées, comprenant :
un agent tensioactif ;
de l'eau ; et
un indicateur acide-base comprenant la substance de formule : 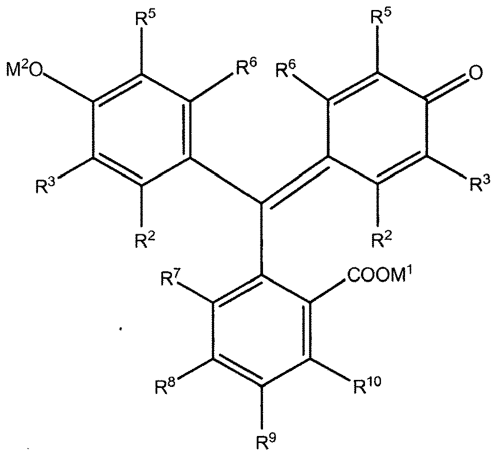 dans laquelle formule :
R² est choisi dans le groupe constitué de l'hydrogène et des groupements nitro, amino et alkyle ;
R³ est choisi dans le groupe constitué de l'hydrogène et des groupements aryle, alkyle, nitro, acétamido et alcoxy ;
R⁵ est choisi dans le groupe constitué de l'hydrogène et des groupements halo, alcoxy et alkyle ;
R⁶ est choisi dans le groupe constitué de l'hydrogène et du groupement alkyle ;
**R⁷,** R⁸, R⁹ et R¹⁰ sont tous de l'hydrogène ;
éventuellement, l'un des atomes de carbone liés à R², R³, R⁵ ou R⁶ peut être substitué par un atome d'azote ; et
M¹ et M² sont chacun indépendamment un atome d'hydrogène, un ion de métal ou un ion d'ammonium, pourvu qu'au moins l'un de M¹ ou M² soit un ion de métal ou un ion d'ammonium.

2. Composition de bulles sensiblement uniformément colorées selon la revendication 1, dans laquelle R² est choisi dans le groupe constitué de l'hydrogène et d'un groupement méthyle ; R³ est choisi dans le groupe constitué de l'hydrogène et des groupements phényle, isopropyle, méthyle, éthyle, sec-butyle, nitro et méthoxy ; R⁵ est choisi dans le groupe constitué de l'hydrogène et des groupements bromo, méthoxy, isopropyle et méthyle ; et R⁶ est choisi dans le groupe constitué de l'hydrogène et du groupement méthyle.

3. Composition de bulles sensiblement uniformément colorées selon la revendication 1, dans laquelle R², R³, R⁵, R⁶, R⁷, R⁸, R⁹ et R¹⁰ sont tous des atomes d'hydrogène, ou R² est de l'hydrogène, R³ est Me et R⁵, R⁶, R⁷, R⁸, R⁹ et R¹⁰ sont des atomes d'hydrogène, ou R² est Me, R³ est un atome d'hydrogène, R⁵ est un groupement isopropyle, R⁶, R⁷, R⁸, R⁹ et R¹⁰ sont des atomes d' hydrogène ou R² est H, R³ est Me, R⁵ est Br et R⁶, R⁷, R⁸, R⁹ et R¹⁰ sont des atomes d' hydrogène ou R² est Me, R³ est Br, R⁵ est un groupement isopropyle et R⁶, R⁷, R⁸, R⁹ et R¹⁰ sont des atomes d'hydrogène.

4. Composition de bulles sensiblement uniformément colorées selon la revendication 1, dans laquelle R² est H, R³ est un groupement phényle et R⁵, R⁶, R⁷, R⁸, R⁹ et R¹⁰ sont tous des atomes d' hydrogène, ou R² est H, R³ et R⁵ sont des groupements isopropyle et R⁶, R⁷, R⁸, R⁹ et R¹⁰ sont tous des atomes d'hydrogène, ou R² est H, R³ est un groupement méthyle, R⁵ est H, R⁶ est un groupement méthyle, R⁷, R⁸, R⁹ et R¹⁰ sont tous des atomes d'hydrogène, ou R² est H, R³ et R⁵ sont des groupements méthoxy et R⁶, R⁷, R⁸, R⁹ et R¹⁰ sont tous des atomes d'hydrogène, ou R² est H, R³ et R⁵ sont des groupements méthyle et R⁶, R⁷, R⁸, R⁹ et R¹⁰ sont tous des atomes d' hydrogène, ou R² est H, R³ est un groupement éthyle et R⁵, R⁶, R⁷, R⁸, R⁹ et R¹⁰ sont tous des atomes d' hydrogène, ou R² est H, R³ est un groupement isopropyle et R⁵, R⁶, R⁷, R⁸, R⁹ et R¹⁰ sont tous des atomes d' hydrogène, ou R² est H, R³ est un groupement méthoxyde et R⁵, R⁶, R⁷, R⁸, R⁹ et R¹⁰ sont tous des atomes d'hydrogène ou R² R³ et R⁵ sont tous des groupements méthyle et R⁶, R⁷, R⁸, R⁹ et R¹⁰ sont tous des atomes d' hydrogène, ou R², R⁵, R⁶, R⁷, R⁸, R⁹ et R¹⁰ sont tous des atomes d'hydrogène et R³ est un groupement sec-butyle, ou R², R⁵, R⁶, R⁷, R⁸, R⁹ et R¹⁰ sont tous des atomes d'hydrogène et R³ est un groupement nitro.

5. Composition de bulles sensiblement uniformément colorées selon l'une quelconque des revendications 1 à 4, comprenant en outre une base.

6. Composition de bulles sensiblement uniformément colorées selon la revendication 5, dans laquelle la base est un hydroxyde de métal.

7. Composition de bulles sensiblement uniformément colorées selon la revendication 1, dans laquelle l'agent tensioactif est un éther cellulosique.

8. Composition de bulles sensiblement uniformément colorées selon la revendication 1, dans laquelle l'agent tensioactif est une combinaison de sulfate de lauryle, d'alcools alkyliques en C₁₀-C₁₆, de sels de sodium et d'alcools en C₁₀-C₁₆.
